# EUROPÄISCHE PATENTSCHRIFT

(11) **EP 3 180 424 B1**
(45) Veröffentlichungstag und Bekanntmachung des Hinweises auf die Patenterteilung: **18.03.2020**
(21) Anmeldenummer: 15750704.7
(22) Anmeldetag: 12.08.2015
(51) Int. Cl.: C12N 9/04, C12P 33/02

(54) **3ALPHA-HYDROXYSTEROID DEHYDROGENASE-MUTANTEN UND VERFAHREN ZUR HERSTELLUNG VON URSODESOXYCHOLSÄURE**
3ALPHA-HYDROXYSTERIOD DEHYDROGENASE MUTANTS AND METHOD FOR PRODUCING URSODEOXYCHOLIC ACID
3ALPHA-HYDROXYSTÉROÏDES DÉSHYDROGÉNASES MUTANTS ET PROCÉDÉ DE FABRICATION D'ACIDE URSODÉSOXYCHOLIQUE

(30) Priorität: 12.08.2014 EP 14180614
(43) Veröffentlichungstag der Anmeldung: 21.06.2017
(73) Patentinhaber: PharmaZell GmbH, 83064 Raubling (DE)
(72) Erfinder: HUMMEL, Werner, 52445 Titz (DE); BAKONYI, Daniel, 50969 Köln (DE)
(74) Vertreter: Reitstötter Kinzebach
(86) Internationale Anmeldenummer: PCT/EP2015/068514
(87) Internationale Veröffentlichungsnummer: WO 2016/023933

(56) Entgegenhaltungen:
- WO-A1-2011/064404
- WO-A1-2012/080504
- DATABASE UniProt [Online] 18. April 2012 (2012-04-18), "SubName: Full=Short-chain alcohol dehydrogenase like protein {ECO:0000313|EMBL:EHR50841.1};", XP002744436, gefunden im EBI accession no. UNIPROT:H5X0P5 Database accession no. H5X0P5
- DATABASE UniProt [Online] 11. Juni 2014 (2014-06-11), "SubName: Full=3-alpha-hydroxysteroid dehydrogenase {ECO:0000313|EMBL:EYS85454.1};", XP002744437, gefunden im EBI accession no. UNIPROT:A0A022FPP9 Database accession no. A0A022FPP9
- DATABASE UniProt [Online] 13. November 2013 (2013-11-13), "SubName: Full=Putative oxidoreductase {ECO:0000313|EMBL:BAN54529.1};", XP002744438, gefunden im EBI accession no. UNIPROT:T2H6F4 Database accession no. T2H6F4
- EDMUND MASER ET AL: "Functional Expression, Purification, and Characterization of 3[alpha]-Hydroxysteroid Dehydrogenase/Carbonyl Reductase from Comamonas testosteroni", BIOCHEMICAL AND BIOPHYSICAL RESEARCH COMMUNICATIONS, vol. 272, no. 2, 1 June 2000 (2000-06-01), pages 622-628, XP055491607, AMSTERDAM, NL ISSN: 0006-291X, DOI: 10.1006/bbrc.2000.2813

### SEQUENCE LISTING

<110> PharmaZell GmbH
<120> Neuartige 3alpha-Hydroxysteroid Dehydrogenase-Mutanten und Verfahren zur Herstellung von Ursodesoxycholsäure
<130> M/55001-PCT
<160> 49
<170> PatentIn version 3.5
<210> 1
   <211> 792
   <212> DNA
   <213> Colinsella aerofaciems
<220>
   <221> CDS
   <222> (1)..(792)
<400> 1
   atg aac ctg agg gag aag tac ggt gag tgg ggc ctg atc ctg ggc gcg       48
   Met Asn Leu Arg Glu Lys Tyr Gly Glu Trp Gly Leu Ile Leu Gly Ala
   1               5                   10                  15
   acc gag ggc gtc ggc aag gcg ttc tgc gag aag atc gcc gcc ggc ggc       96
   Thr Glu Gly Val Gly Lys Ala Phe Cys Glu Lys Ile Ala Ala Gly Gly
               20                  25                  30
   atg aac gtc gtc atg gtc ggc cgt cgc gag gag aag ctg aac gtg ctc      144
   Met Asn Val Val Met Val Gly Arg Arg Glu Glu Lys Leu Asn Val Leu
           35                  40                  45
   gca ggc gag atc cgc gag acc tac ggc gtg gag acc aag gtc gtg cgc      192
   Ala Gly Glu Ile Arg Glu Thr Tyr Gly Val Glu Thr Lys Val Val Arg
       50                  55                  60
   gcc gac ttt agc cag ccc ggc gct gcc gag acc gtc ttc gcc gcg acc      240
   Ala Asp Phe Ser Gln Pro Gly Ala Ala Glu Thr Val Phe Ala Ala Thr
   65                  70                  75                  80
   gag ggc ctg gac atg ggc ttc atg agc tac gtg gcc tgc ctg cac agc      288
   Glu Gly Leu Asp Met Gly Phe Met Ser Tyr Val Ala Cys Leu His Ser
                   85                  90                  95
   ttc ggt aag atc cag gac acc ccc tgg gag aag cac gag gcc atg atc      336
   Phe Gly Lys Ile Gln Asp Thr Pro Trp Glu Lys His Glu Ala Met Ile
               100                 105                 110
   aac gtc aac gtc gtg acc ttc ctc aag tgc ttc cac cac tac atg cgg      384
   Asn Val Asn Val Val Thr Phe Leu Lys Cys Phe His His Tyr Met Arg
           115                 120                 125
   atc ttt gcc gcc cag gac cgc ggc gcc gtg atc aac gtc tcg tcg atg      432
   Ile Phe Ala Ala Gln Asp Arg Gly Ala Val Ile Asn Val Ser Ser Met
       130                 135                 140
   acc ggc atc agc tcc agc ccc tgg aac ggc cag tac ggc gcg ggc aag      480
   Thr Gly Ile Ser Ser Ser Pro Trp Asn Gly Gln Tyr Gly Ala Gly Lys
   145                 150                 155                 160
   gcc ttc atc ctc aag atg acc gag gcc gtg gcc tgc gag tgc gag ggc       528
   Ala Phe Ile Leu Lys Met Thr Glu Ala Val Ala Cys Glu Cys Glu Gly
                   165                 170                 175
   acc ggc gtc gac gtc gag gtc atc acc ctc ggc acc acc cta acc ccc       576
   Thr Gly Val Asp Val Glu Val Ile Thr Leu Gly Thr Thr Leu Thr Pro
               180                 185                 190
   agc ctg ctg tcc aac ctc ccc ggc ggc ccg cag ggc gag gcc gtc atg       624
   Ser Leu Leu Ser Asn Leu Pro Gly Gly Pro Gln Gly Glu Ala Val Met
           195                 200                 205
   aag atc gcc ctc acc ccc gag gag tgc gtt gac gag gcc ttt gag aag       672
   Lys Ile Ala Leu Thr Pro Glu Glu Cys Val Asp Glu Ala Phe Glu Lys
       210                 215                 220
   ctg ggt aag gag ctc tcc gtc atc gcc ggc cag cgc aac aag gac tcc       720
   Leu Gly Lys Glu Leu Ser Val Ile Ala Gly Gln Arg Asn Lys Asp Ser
   225                 230                 235                     240
   gtc cac gac tgg aag gca aac cac acc gag gac gag tac atc cgc tac       768
   Val His Asp Trp Lys Ala Asn His Thr Glu Asp Glu Tyr Ile Arg Tyr
                   245                 250                 255
   atg ggg tcg ttc tac cgc gac tag                                       792
   Met Gly Ser Phe Tyr Arg Asp
               260
<210> 2
   <211> 263
   <212> PRT
   <213> Colinsella aerofaciems
<400> 2
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Gly Arg Arg Glu Glu Lys Leu Asn Val Leu
           35                  40                  45
   Ala Gly Glu Ile Arg Glu Thr Tyr Gly Val Glu Thr Lys Val Val Arg
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
       130                 135                 140
   Thr Gly Ile Ser Ser Ser Pro Trp Asn Gly Gln Tyr Gly Ala Gly Lys
   145                 150                 155                 160
   Ala Phe Ile Leu Lys Met Thr Glu Ala Val Ala Cys Glu Cys Glu Gly
                   165                 170                 175
   Thr Gly Val Asp Val Glu Val Ile Thr Leu Gly Thr Thr Leu Thr Pro
               180                 185                 190
   Ser Leu Leu Ser Asn Leu Pro Gly Gly Pro Gln Gly Glu Ala Val Met
           195                 200                 205
   Lys Ile Ala Leu Thr Pro Glu Glu Cys Val Asp Glu Ala Phe Glu Lys
       210                 215                 220
   Leu Gly Lys Glu Leu Ser Val Ile Ala Gly Gln Arg Asn Lys Asp Ser
   225                 230                 235                 240
   Val His Asp Trp Lys Ala Asn His Thr Glu Asp Glu Tyr Ile Arg Tyr
                   245                 250                 255
   Met Gly Ser Phe Tyr Arg Asp
               260
<210> 3
   <211> 774
   <212> DNA
   <213> Comamonas testosteroni
<220>
   <221> CDS
   <222> (1)..(774)
<400> 3
   atg tcc atc atc gtg ata agc ggc tgc gcc acc ggc att ggt gcc gct         48
   acg cgc aag gtc ctg gag gcg gcc ggt cac cag atc gta ggc atc gat         96
   ata cgc gat gcg gaa gtg att gcc gat ctc tcg acg gcc gaa ggt cga        144
   aag cag gcg att gcc gat gta ctg gcg aag tgc agc aag ggc atg gac        192
   ggc ctg gtg ctg tgc gcc ggc ctg gga ccg cag acc aag gtg ctt ggc        240
   aat gtg gtt tcg gtc aat tat ttt ggc gcg acc gag ctg atg gat gcc        288
   ttt ttg cca gcg ctg aaa aaa ggc cat cag ccc gca gcc gtc gtc atc        336
   tcg tcc gtg gct tcc gcg cat ctg gct ttt gac aag aac cca ctg gcg        384
   ctg gca ctg gaa gcc ggc gag gaa gcc aag gcc cgc gcc att gtc gaa        432
   cat gcg gga gag cag ggc gga aat ctg gcc tat gcg ggc agc aag aat        480
   gct ttg acg gtg gct gtg cgc aaa cgc gcc gcc gcc tgg ggc gag gct        528
   ggc gtg cgc ctg aac acc atc gcc ccc ggt gca acc gag act ccc ttg        576
   ctg cag gcg ggc ctg cag gac ccg cgc tat ggc gaa tcc att gcc aag        624
   ttc gtt cct ccc atg ggc cgc cgt gcc gag ccg tcc gag atg gcg tcg        672
   gtc atc gcc ttt ttg atg agc ccg gcc gca agc tat gtg cat ggc gcg        720
   cag atc gtc att gat ggc ggc att gat gcg gtg atg cgc ccg aca cag        768
   ttc tga                                                                774
<210> 4
   <211> 257
   <212> PRT
   <213> Comamonas testosteroni
<400> 4
<210> 5
   <211> 265
   <212> PRT
   <213> Artificial Sequence
<220>
   <223> 3alpha HSDH WT + HIS tag
<400> 5
   Met Ser Ile Ile Val Ile Ser Gly Cys Ala Thr Gly Ile Gly Ala Ala
   1               5                   10                  15
   Thr Arg Lys Val Leu Glu Ala Ala Gly His Gln Ile Val Gly Ile Asp
               20                  25                  30
   Ile Arg Asp Ala Glu Val Ile Ala Asp Leu Ser Thr Ala Glu Gly Arg
           35                  40                  45
   Lys Gln Ala Ile Ala Asp Val Leu Ala Lys Cys Ser Lys Gly Met Asp
       50                  55                  60
   Gly Leu Val Leu Cys Ala Gly Leu Gly Pro Gln Thr Lys Val Leu Gly
   65                  70                  75                  80
   Asn Val Val Ser Val Asn Tyr Phe Gly Ala Thr Glu Leu Met Asp Ala
                   85                  90                  95
   Phe Leu Pro Ala Leu Lys Lys Gly His Gln Pro Ala Ala Val Val Ile
               100                 105                 110
   Ser Ser Val Ala Ser Ala His Leu Ala Phe Asp Lys Asn Pro Leu Ala
           115                 120                 125
   Leu Ala Leu Glu Ala Gly Glu Glu Ala Lys Ala Arg Ala Ile Val Glu
       130                 135                 140
   His Ala Gly Glu Gln Gly Gly Asn Leu Ala Tyr Ala Gly Ser Lys Asn
   145                 150                 155                 160
   Ala Leu Thr Val Ala Val Arg Lys Arg Ala Ala Ala Trp Gly Glu Ala
                   165                 170                 175
   Gly Val Arg Leu Asn Thr Ile Ala Pro Gly Ala Thr Glu Thr Pro Leu
                   180                 185                 190
   Leu Gln Ala Gly Leu Gln Asp Pro Arg Tyr Gly Glu Ser Ile Ala Lys
           195                 200                 205
   Phe Val Pro Pro Met Gly Arg Arg Ala Glu Pro Ser Glu Met Ala Ser
       210                 215                 220
   Val Ile Ala Phe Leu Met Ser Pro Ala Ala Ser Tyr Val His Gly Ala
   225                 230                 235                 240
   Gln Ile Val Ile Asp Gly Gly Ile Asp Ala Val Met Arg Pro Thr Gln
                   245                 250                 255
   Phe Leu Glu His His His His His His
               260                 265
<210> 6
   <211> 265
   <212> PRT
   <213> Artificial Sequence
<220>
   <223> 3alpha-HSDH [R34N];
<400> 6
   Met Ser Ile Ile Val Ile Ser Gly Cys Ala Thr Gly Ile Gly Ala Ala
   1               5                   10                  15
   Thr Arg Lys Val Leu Glu Ala Ala Gly His Gln Ile Val Gly Ile Asp
               20                  25                  30
   Ile Asn Asp Ala Glu Val Ile Ala Asp Leu Ser Thr Ala Glu Gly Arg
           35                  40                  45
   Lys Gln Ala Ile Ala Asp Val Leu Ala Lys Cys Ser Lys Gly Met Asp
       50                  55                  60
   Gly Leu Val Leu Cys Ala Gly Leu Gly Pro Gln Thr Lys Val Leu Gly
   65                  70                  75                  80
   Asn Val Val Ser Val Asn Tyr Phe Gly Ala Thr Glu Leu Met Asp Ala
                   85                  90                  95
   Phe Leu Pro Ala Leu Lys Lys Gly His Gln Pro Ala Ala Val Val Ile
               100                 105                 110
   Ser Ser Val Ala Ser Ala His Leu Ala Phe Asp Lys Asn Pro Leu Ala
           115                 120                 125
   Leu Ala Leu Glu Ala Gly Glu Glu Ala Lys Ala Arg Ala Ile Val Glu
       130                 135                 140
   His Ala Gly Glu Gln Gly Gly Asn Leu Ala Tyr Ala Gly Ser Lys Asn
   145                 150                 155                 160
   Ala Leu Thr Val Ala Val Arg Lys Arg Ala Ala Ala Trp Gly Glu Ala
                   165                 170                 175
   Gly Val Arg Leu Asn Thr Ile Ala Pro Gly Ala Thr Glu Thr Pro Leu
               180                 185                 190
   Leu Gln Ala Gly Leu Gln Asp Pro Arg Tyr Gly Glu Ser Ile Ala Lys
           195                 200                 205
   Phe Val Pro Pro Met Gly Arg Arg Ala Glu Pro Ser Glu Met Ala Ser
       210                 215                 220
   Val Ile Ala Phe Leu Met Ser Pro Ala Ala Ser Tyr Val His Gly Ala
   225                 230                 235                 240
   Gln Ile Val Ile Asp Gly Gly Ile Asp Ala Val Met Arg Pro Thr Gln
                   245                 250                 255
   Phe Leu Glu His His His His His His
               260                 265
<210> 7
   <211> 265
   <212> PRT
   <213> Artificial Sequence
<220>
   <223> 3alpha-HSDH [L68A];
<400> 7
   Met Ser Ile Ile Val Ile Ser Gly Cys Ala Thr Gly Ile Gly Ala Ala
   1               5                   10                  15
   Thr Arg Lys Val Leu Glu Ala Ala Gly His Gln Ile Val Gly Ile Asp
               20                  25                  30
   Ile Arg Asp Ala Glu Val Ile Ala Asp Leu Ser Thr Ala Glu Gly Arg
           35                  40                  45
   Lys Gln Ala Ile Ala Asp Val Leu Ala Lys Cys Ser Lys Gly Met Asp
       50                  55                  60
   Gly Leu Val Cys Cys Ala Gly Leu Gly Pro Gln Thr Lys Val Leu Gly
   65                  70                  75                  80
   Asn Val Val Ser Val Asn Tyr Phe Gly Ala Thr Glu Leu Met Asp Ala
                   85                  90                  95
   Phe Leu Pro Ala Leu Lys Lys Gly His Gln Pro Ala Ala Val Val Ile
               100                 105                 110
   Ser Ser Val Ala Ser Ala His Leu Ala Phe Asp Lys Asn Pro Leu Ala
           115                 120                 125
   Leu Ala Leu Glu Ala Gly Glu Glu Ala Lys Ala Arg Ala Ile Val Glu
       130                 135                 140
   His Ala Gly Glu Gln Gly Gly Asn Leu Ala Tyr Ala Gly Ser Lys Asn
   145                 150                 155                 160
   Ala Leu Thr Val Ala Val Arg Lys Arg Ala Ala Ala Trp Gly Glu Ala
                   165                 170                 175
   Gly Val Arg Leu Asn Thr Ile Ala Pro Gly Ala Thr Glu Thr Pro Leu
               180                 185                 190
   Leu Gln Ala Gly Leu Gln Asp Pro Arg Tyr Gly Glu Ser Ile Ala Lys
           195                 200                 205
   Phe Val Pro Pro Met Gly Arg Arg Ala Glu Pro Ser Glu Met Ala Ser
       210                 215                 220
   Val Ile Ala Phe Leu Met Ser Pro Ala Ala Ser Tyr Val His Gly Ala
   225                 230                 235                 240
   Gln Ile Val Ile Asp Gly Gly Ile Asp Ala Val Met Arg Pro Thr Gln
                   245                 250                 255
   Phe Leu Glu His His His His His His
               260                 265
<210> 8
   <211> 265
   <212> PRT
   <213> Artificial Sequence
<220>
   <223> 3alpha-HSDH [R34N/L68A];
<400> 8
   Met Ser Ile Ile Val Ile Ser Gly Cys Ala Thr Gly Ile Gly Ala Ala
   1               5                   10                  15
   Thr Arg Lys Val Leu Glu Ala Ala Gly His Gln Ile Val Gly Ile Asp
               20                  25                  30
   Ile Asn Asp Ala Glu Val Ile Ala Asp Leu Ser Thr Ala Glu Gly Arg
           35                  40                  45
   Lys Gln Ala Ile Ala Asp Val Leu Ala Lys Cys Ser Lys Gly Met Asp
       50                  55                  60
   Gly Leu Val Ala Cys Ala Gly Leu Gly Pro Gln Thr Lys Val Leu Gly
   65                  70                  75                  80
   Asn Val Val Ser Val Asn Tyr Phe Gly Ala Thr Glu Leu Met Asp Ala
                   85                  90                  95
   Phe Leu Pro Ala Leu Lys Lys Gly His Gln Pro Ala Ala Val Val Ile
               100                 105                 110
   Ser Ser Val Ala Ser Ala His Leu Ala Phe Asp Lys Asn Pro Leu Ala
           115                 120                 125
   Leu Ala Leu Glu Ala Gly Glu Glu Ala Lys Ala Arg Ala Ile Val Glu
       130                 135                 140
   His Ala Gly Glu Gln Gly Gly Asn Leu Ala Tyr Ala Gly Ser Lys Asn
   145                 150                 155                 160
   Ala Leu Thr Val Ala Val Arg Lys Arg Ala Ala Ala Trp Gly Glu Ala
                   165                 170                 175
   Gly Val Arg Leu Asn Thr Ile Ala Pro Gly Ala Thr Glu Thr Pro Leu
               180                 185                 190
   Leu Gln Ala Gly Leu Gln Asp Pro Arg Tyr Gly Glu Ser Ile Ala Lys
           195                 200                 205
   Phe Val Pro Pro Met Gly Arg Arg Ala Glu Pro Ser Glu Met Ala Ser
       210                 215                 220
   Val Ile Ala Phe Leu Met Ser Pro Ala Ala Ser Tyr Val His Gly Ala
   225                 230                 235                 240
   Gln Ile Val Ile Asp Gly Gly Ile Asp Ala Val Met Arg Pro Thr Gln
                   245                 250                 255
   Phe Leu Glu His His His His His His
   260 265
<210> 9
   <211> 401
   <212> PRT
   <213> Mycobacterium vaccae
<400> 9
   Met Ala Lys Val Leu Cys Val Leu Tyr Asp Asp Pro Val Asp Gly Tyr
   1 5 10 15
   Pro Lys Thr Tyr Ala Arg Asp Asp Leu Pro Lys Ile Asp His Tyr Pro
   20 25 30
   Gly Gly Gln Ile Leu Pro Thr Pro Lys Ala Ile Asp Phe Thr Pro Gly
   35 40 45
   Gln Leu Leu Gly Ser Val Ser Gly Glu Leu Gly Leu Arg Glu Tyr Leu
   50 55 60
   Glu Ser Asn Gly His Thr Leu Val Val Thr Ser Asp Lys Asp Gly Pro
   65 70 75 80
   Asp Ser Val Phe Glu Arg Glu Leu Val Asp Ala Asp Val Val Ile Ser
   85 90 95
   Gln Pro Phe Trp Pro Ala Tyr Leu Thr Pro Glu Arg Ile Ala Lys Ala
   100 105 110
   Lys Asn Leu Lys Leu Ala Leu Thr Ala Gly Ile Gly Ser Asp His Val
   115 120 125
   Asp Leu Gln Ser Ala Ile Asp Arg Asn Val Thr Val Ala Glu Val Thr
   130 135 140
   Tyr Cys Asn Ser Ile Ser Val Ala Glu His Val Val Met Met Ile Leu
   145 150 155 160
   Ser Leu Val Arg Asn Tyr Leu Pro Ser His Glu Trp Ala Arg Lys Gly
   165 170 175
   Gly Trp Asn Ile Ala Asp Cys Val Ser His Ala Tyr Asp Leu Glu Ala
   180 185 190
   Met His Val Gly Thr Val Ala Ala Gly Arg Ile Gly Leu Ala Val Leu
   195 200 205
   Arg Arg Leu Ala Pro Phe Asp Val His Leu His Tyr Thr Asp Arg His
       210                 215                 220
   Arg Leu Pro Glu Ser Val Glu Lys Glu Leu Asn Leu Thr Trp His Ala
   225                 230                 235                 240
   Thr Arg Glu Asp Met Tyr Pro Val Cys Asp Val Val Thr Leu Asn Cys
                   245                 250                 255
   Pro Leu His Pro Glu Thr Glu His Met Ile Asn Asp Glu Thr Leu Lys
               260                 265                 270
   Leu Phe Lys Arg Gly Ala Tyr Ile Val Asn Thr Ala Arg Gly Lys Leu
           275                 280                 285
   Cys Asp Arg Asp Ala Val Ala Arg Ala Leu Glu Ser Gly Arg Leu Ala
       290                 295                 300
   Gly Tyr Ala Gly Asp Val Trp Phe Pro Gln Pro Ala Pro Lys Asp His
   305                 310                 315                 320
   Pro Trp Arg Thr Met Pro Tyr Asn Gly Met Thr Pro His Ile Ser Gly
                   325                 330                 335
   Thr Thr Leu Thr Ala Gln Ala Arg Tyr Ala Ala Gly Thr Arg Glu Ile
               340                 345                 350
   Leu Glu Cys Phe Phe Glu Gly Arg Pro Ile Arg Asp Glu Tyr Leu Ile
           355                 360                 365
   Val Gln Gly Gly Ala Leu Ala Gly Thr Gly Ala His Ser Tyr Ser Lys
       370                 375                 380
   Gly Asn Ala Thr Gly Gly Ser Glu Glu Ala Ala Lys Phe Lys Lys Ala
   385                 390                 395                 400
   Val
<210> 10
   <211> 786
   <212> DNA
   <213> Bacillus subtilis
<220>
   <221> CDS
   <222> (1)..(786)
<400> 10
   atg tat ccg gat tta aaa gga aaa gtc gtc gct att aca gga gct gct      48
   Met Tyr Pro Asp Leu Lys Gly Lys Val Val Ala Ile Thr Gly Ala Ala
   1               5                   10                  15
   tca ggg ctc gga aag gcg atg gcc att cgc ttc ggc aag gag cag gca      96
   Ser Gly Leu Gly Lys Ala Met Ala Ile Arg Phe Gly Lys Glu Gln Ala
               20                  25                  30
   aaa gtg gtt atc aac tat tat agt aat aaa caa gat ccg aac gag gta     144
   Lys Val Val Ile Asn Tyr Tyr Ser Asn Lys Gln Asp Pro Asn Glu Val
           35                  40                  45
   aaa gaa gag gtc atc aag gcg ggc ggt gaa gct gtt gtc gtc caa gga     192
   Lys Glu Glu Val Ile Lys Ala Gly Gly Glu Ala Val Val Val Gln Gly
       50                  55                  60
   gat gtc acg aaa gag gaa gat gta aaa aat atc gtg caa acg gca att     240
   Asp Val Thr Lys Glu Glu Asp Val Lys Asn Ile Val Gln Thr Ala Ile
   65                  70                  75                  80
   aag gag ttc ggc aca ctc gat att atg att aat aat gcc ggt ctt gaa     288
   Lys Glu Phe Gly Thr Leu Asp Ile Met Ile Asn Asn Ala Gly Leu Glu
                   85                  90                  95
   aat cct gtg cca tct cac gaa atg ccg ctc aag gat tgg gat aaa gtc     336
   Asn Pro Val Pro Ser His Glu Met Pro Leu Lys Asp Trp Asp Lys Val
               100                 105                 110
   atc ggc acg aac tta acg ggt gcc ttt tta gga agc cgt gaa gcg att     384
   Ile Gly Thr Asn Leu Thr Gly Ala Phe Leu Gly Ser Arg Glu Ala Ile
           115                 120                 125
   aaa tat ttc gta gaa aac gat atc aag gga aat gtc att aac atg tcc     432
   Lys Tyr Phe Val Glu Asn Asp Ile Lys Gly Asn Val Ile Asn Met Ser
       130                 135                 140
   agt gtg cac gaa gtg att cct tgg ccg tta ttt gtc cac tat gcg gca     480
   Ser Val His Glu Val Ile Pro Trp Pro Leu Phe Val His Tyr Ala Ala
   145                 150                 155                 160
   agt aaa ggc ggg ata aag ctg atg aca gaa aca tta gcg ttg gaa tac     528
   Ser Lys Gly Gly Ile Lys Leu Met Thr Glu Thr Leu Ala Leu Glu Tyr
                   165                 170                 175
   gcg ccg aag ggc att cgc gtc aat aat att ggg cca ggt gcg atc aac     576
   Ala Pro Lys Gly Ile Arg Val Asn Asn Ile Gly Pro Gly Ala Ile Asn
               180                 185                 190
   acg cca atc aat gct gaa aaa ttc gct gac cct aaa cag aaa gct gat     624
   Thr Pro Ile Asn Ala Glu Lys Phe Ala Asp Pro Lys Gln Lys Ala Asp
           195                 200                 205
   gta gaa agc atg att cca atg gga tat atc ggc gaa ccg gag gag atc     672
   Val Glu Ser Met Ile Pro Met Gly Tyr Ile Gly Glu Pro Glu Glu Ile
       210                 215                 220
   gcc gca gta gca gcc tgg ctt gct tcg aag gaa gcc agc tac gtc aca     720
   Ala Ala Val Ala Ala Trp Leu Ala Ser Lys Glu Ala Ser Tyr Val Thr
   225                 230                 235                 240
   ggc atc acg tta ttc gcg gac ggc ggt atg aca caa tat cct tca ttc     768
   Gly Ile Thr Leu Phe Ala Asp Gly Gly Met Thr Gln Tyr Pro Ser Phe
                   245                 250                 255
   cag gca ggc cgc ggt taa                                                        786
   Gln Ala Gly Arg Gly
               260
<210> 11
   <211> 261
   <212> PRT
   <213> Bacillus subtilis
<400> 11
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Glu
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
           115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Glu Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
               195                 200                 205
   Val Glu Ser Met Ile Pro Met Gly Tyr Ile Gly Glu Pro Glu Glu Ile
       210                 215                 220
   Ala Ala Val Ala Ala Trp Leu Ala Ser Lys Glu Ala Ser Tyr Val Thr
   225                 230                 235                 240
   Gly Ile Thr Leu Phe Ala Asp Gly Gly Met Thr Gln Tyr Pro Ser Phe
                   245                 250                 255
   Gln Ala Gly Arg Gly
               260
<210> 12
   <211> 27
   <212> DNA
   <213> Artificial Sequence
<220>
   <223> Primer
<400> 12
   ggcatcgata taaacgatgc ggaagtg 27
<210> 13
   <211> 27
   <212> DNA
   <213> Artificial Sequence
<220>
   <223> Primer
<400> 13
   cacttccgca tcgtttatat cgatgcc 27
<210> 14
   <211> 34
   <212> DNA
   <213> Artificial Sequence
<220>
   <223> Primer
<400> 14
   tcgtaggcat cgatatactc gatgcggaag tgat 34
<210> 15
   <211> 34
   <212> DNA
   <213> Artificial Sequence
<220>
   <223> primer
<400> 15
   atcacttccg catcgagtat atcgatgcct acga 34
<210> 16
   <211> 36
   <212> DNA
   <213> Artificial Sequence
<220>
   <223> Primer
<400> 16
   accagatcgt aggcatcgat ataagcgatg cggaag 36
<210> 17
   <211> 36
   <212> DNA
   <213> Artificial Sequence
<220>
   <223> Primer
<400> 17
   cttccgcatc gcttatatcg atgcctacga tctggt 36
<210> 18
   <211> 36
   <212> DNA
   <213> Artificial Sequence
<220>
   <223> Primer
<400> 18
   accagatcgt aggcatcgat atatgcgatg cggaag 36
<210> 19
   <211> 36
   <212> DNA
   <213> Artificial Sequence
<220>
   <223> Primer
<400> 19
   cttccgcatc gcatatatcg atgcctacga tctggt 36
<210> 20
   <211> 28
   <212> DNA
   <213> Artificial Sequence
<220>
   <223> Primer
<400> 20
   ggacggcctg gtgtcgtgcg ccggcctg 28
<210> 21
   <211> 28
   <212> DNA
   <213> Artificial Sequence
<220>
   <223> Primer
<400> 21
   caggccggcg cacgacacca ggccgtcc 28
<210> 22
   <211> 29
   <212> DNA
   <213> Artificial Sequence
<220>
   <223> Primer
<400> 22
   ggacggcctg gtgtgctgcg ccggcctgg 29
<210> 23
   <211> 29
   <212> DNA
   <213> Artificial Sequence
<220>
   <223> Primer
<400> 23
   ccaggccggc gcagcacacc aggccgtcc 29
<210> 24
   <211> 28
   <212> DNA
   <213> Artificial Sequence
<220>
   <223> Primer
<400> 24
   ggacggcctg gtgaagtgcg ccggcctg 28
<210> 25
   <211> 28
   <212> DNA
   <213> Artificial Sequence
<220>
   <223> Primer
<400> 25
   caggccggcg cacttcacca ggccgtcc 28
<210> 26
   <211> 23
   <212> DNA
   <213> Artificial Sequence
<220>
   <223> Primer
<400> 26
   acggcctggt ggtgtgcgcc ggc 23
<210> 27
   <211> 23
   <212> DNA
   <213> Artificial Sequence
<220>
   <223> Primer
<400> 27
   gccggcgcac accaccaggc cgt 23
<210> 28
   <211> 26
   <212> DNA
   <213> Artificial Sequence
<220>
   <223> Primer
<400> 28
   gacggcctgg tggcgtgcgc cggcct 26
<210> 29
   <211> 26
   <212> DNA
   <213> Artificial Sequence
<220>
   <223> Primer
<400> 29
   aggccggcgc acgccaccag gccgtc 26
<210> 30
   <211> 25
   <212> DNA
   <213> Artificial Sequence
<220>
   <223> PCR Primer
<400> 30
   aacaccatcg ccgccggtgc aaccg 25
<210> 31
   <211> 25
   <212> DNA
   <213> Artificial Sequence
<220>
   <223> PCR Primer
<400> 31
   cggttgcacc ggcggcgatg gtgtt 25
<210> 32
   <211> 28
   <212> DNA
   <213> Artificial Sequence
<220>
   <223> PCR Primer
<400> 32
   gaacaccatc gccggcggtg caaccgag 28
<210> 33
   <211> 28
   <212> DNA
   <213> Artificial Sequence
<220>
   <223> PCR Primer
<400> 33
   ctcggttgca ccgccggcga tggtgttc 28
<210> 34
   <211> 27
   <212> DNA
   <213> Artificial Sequence
<220>
   <223> PCR Primer
<400> 34
   gaacaccatc gccaccggtg caaccga 27
<210> 35
   <211> 27
   <212> DNA
   <213> Artificial Sequence
<220>
   <223> PCR Primer
<400> 35
   tcggttgcac cggtggcgat ggtgttc 27
<210> 36
   <211> 32
   <212> DNA
   <213> Artificial Sequence
<220>
   <223> PCR Primer
<400> 36
   ctgaacacca tcgccgtcgg tgcaaccgag ac 32
<210> 37
   <211> 32
   <212> DNA
   <213> Artificial Sequence
<220>
   <223> PCR Primer
<400> 37
   gtctcggttg caccgacggc gatggtgttc ag 32
<210> 38
   <211> 32
   <212> DNA
   <213> Artificial Sequence
<220>
   <223> PCR Primer
<400> 38
   ctgaacacca tcgccagcgg tgcaaccgag ac 32
<210> 39
   <211> 32
   <212> DNA
   <213> Artificial Sequence
<220>
   <223> PCR Primer
<400> 39
   gtctcggttg caccgctggc gatggtgttc ag 32
<210> 40
   <211> 24
   <212> DNA
   <213> Artificial Sequence
<220>
   <223> PCR primer
<400> 40
   cgcccccggt gcagccgaga ctcc 24
<210> 41
   <211> 24
   <212> DNA
   <213> Artificial Sequence
<220>
   <223> PCR Primer
<400> 41
   ggagtctcgg ctgcaccggg ggcg 24
<210> 42
   <211> 23
   <212> DNA
   <213> Artificial Sequence
<220>
   <223> PCR Primer
<400> 42
   cccccggtgc aagcgagact ccc 23
<210> 43
   <211> 23
   <212> DNA
   <213> Artificial Sequence
<220>
   <223> PCR Primer
<400> 43
   gggagtctcg cttgcaccgg ggg 23
<210> 44
   <211> 30
   <212> DNA
   <213> Artificial Sequence
<220>
   <223> PCR Primer
<400> 44
   atcgcccccg gtgcagtcga gactcccttg 30
<210> 45
   <211> 30
   <212> DNA
   <213> Artificial Sequence
<220>
   <223> PCR Primer
<400> 45
   caagggagtc tcgactgcac cgggggcgat 30
<210> 46
   <211> 28
   <212> DNA
   <213> Artificial Sequence
<220>
   <223> PCR Primer
<400> 46
   tcgcccccgg tgcaggcgag actccctt 28
<210> 47
   <211> 28
   <212> DNA
   <213> Artificial Sequence
<220>
   <223> PCR Primer
<400> 47
   aagggagtct cgcctgcacc gggggcga 28
<210> 48
   <211> 33
   <212> DNA
   <213> Artificial Sequence
<220>
   <223> PCR Primer
<400> 48
   atcgcccccg gtgcatggga gactcccttg ctg 33
<210> 49
   <211> 33
   <212> DNA
   <213> Artificial Sequence
<220>
   <223> PCR Primer
<400> 49
   cagcaaggga gtctcccatg caccgggggc gat 33

## Beschreibung

Die Erfindung betrifft neuartige 3α-Hydroxysteroid Dehydrogenase (3α-HSDH)-Mutanten, insbesondere der 3α-HSDH von *Comamonas testosteroni (frühere Bezeichnung Pseudomonas testosteroni)* die für diese Enzym-Mutanten kodierenden Sequenzen; Gegenstand der Erfindung sind auch neuartige Verfahren zur Synthese von 3α-Hydroxysteroiden und insbesondere Ursodesoxycholsäure (UDCS) unter Verwendung der Enzym-Mutanten; sowie die Herstellung von UDCS unter Verwendung rekombinanter, mehrfach modifizierter Mikroorganismen.

### Hintergrund der Erfindung:

Gallensäuren sind Biomoleküle, die für die Verdauung und Absorption von Fetten, Fettsäuren und hydrophoben Vitaminen erforderlich sind. Eine Gallensäure, die beim Menschen nur in geringen Anteilen vorkommt, ist Ursodesoxycholsäure (als UDCA oder UDCS bezeichnet). Sie hat mittlerweile große therapeutische Bedeutung bei der Auflösung von Cholesterin-haltigen Gallensteinen erlangt. Industriell wird diese Verbindung durch chemische oder enzymatische Schritte im Tonnenmaßstab hergestellt. Eine wichtige Vorstufe für die Synthese von UDCA ist 12-Ketoursodesoxycholsäure, die durch eine Wolff-Kishner-Reduktion in UDCA umgewandelt werden kann. Eine literaturbeschriebene Route zur Synthese von 12-Ketoursodesoxycholsäure startet von Cholsäure (3α,7α,12α-Trihydroxy-5β-cholansäure), die durch zwei oxidative Schritte, die durch 7α- und 12α-HSDHs katalysiert werden, und einen reduktiven Schritt, katalysiert durch eine 7β-HSDH, hergestellt werden kann (Bovara R et al. (1996) A new enzymatic route to the synthesis of 12-ketoursodeoxycholic acid. Biotechnol. Lett. 18:305-308; Monti D et al. (2009) One-pot multienzymatic synthesis of 12-ketoursodeoxycholic acid: Subtle cofactor specificities rule the reaction equilibria of five biocatalysts working in a row. Adv. Synth. Catal. 351:1303-1311). Eine weitere Route startet von 7-Ketolithocholsäure, die durch stereoselektive Reduktion der 7-Keto-Gruppe zu UDCA umgesetzt werden kann, auch dieser Schritt wird vorteilhafterweise enzymatisch katalysiert durchgeführt, katalysiert durch eine 7β-HSDH (Higashi S et al. (1979) Conversion of 7-ketolithocholic acid to ursodeoxycholic acid by human intestinal anaerobic microorganisms: Interchangeability of chenodeoxycholic acid and ursodeoxycholic acid. Gastroenterologia Japonica 14:417-424; Liu L et al. (2011) Identification, cloning, heterologous expression, and characterization of a NADPH-dependent 7 beta-hydroxysteroid dehydrogenase from Collinsella aerofaciens. Appl. Microbiol. Biotechnol. 90:127-135.). Eine weitere günstige Syntheseroute geht von Dehydrocholsäure (DHCA) aus, die durch zwei reduktive Schritte zu 12-Ketoursodesoxycholsäure umgesetzt werden kann, diese beiden Schritte können durch zwei stereoselektive HSDHs (3α- and 7β-HSDHs) katalysiert werden (Carrea G et al. (1992) Enzymatic synthesis of 12-ketoursodeoxycholic acid from dehydrocholic acid in a membrane reactor. Biotechnol. Lett. 14:1131-1135; Liu L et al. (2013) Onestep synthesis of 12-ketoursodeoxycholic acid from dehydrocholic acid using a multienzymatic system. Appl. Microbiol. Biotechnol. 97:633-639).

Als 7β-HSDH hat sich das Enzym aus *C*. *aerofaciens* als gut geeignet erwiesen. Mittlerweile ist die Gensequenz dieses Enzyms aus *C*. *aerofaciens* bekannt, so dass zum einen das Enzym nach Klonierung rekombinant verfügbar gemacht werden kann, zum anderen besteht die Möglichkeit, mit Methoden des Proteinengineering Mutanten dieses Enzyms zu erzeugen und damit gfls. vorteilhaftere Enzymvarianten aufzufinden. Derartige Enzymvarianten sind beispielsweise in WO 2012/080504 und WO 2011/064404 beschrieben.

Als 3α-HSDH hat sich das Enzym aus *C*. *testosteroni* als gut geeignet erwiesen. Mittlerweile ist die Gensequenz dieses Enzyms bekannt, so dass zum einen das Enzym nach Klonierung rekombinant verfügbar gemacht werden kann, zum anderen besteht die Möglichkeit, mit Methoden des Proteinengineering Mutanten dieses Enzyms zu erzeugen und damit ggf. vorteilhaftere Enzymvarianten aufzufinden.

Zur medikamentösen Behandlung von Gallensteinleiden werden seit vielen Jahren u. a. die Wirkstoffe Ursodesoxycholsäure (UDCS oder UDCA) und das zugehörige Diastereomer Chenodesoxycholsäure (CDCS oder CDCA) eingesetzt. Beide Verbindungen unterscheiden sich lediglich durch die Konfiguration der Hydroxygruppe am C-Atom 7 (UDCS: β - Konfiguration, CDCS: α-Konfiguration). Zur Herstellung von UDCS sind im Stand der Technik verschieden Verfahren beschreiben, welche rein chemisch durchgeführt werden oder aus einer Kombination chemischer und enzymatischer Verfahrensschritte bestehen. Ausgangspunkt ist jeweils Cholsäure (CS oder CA) oder die aus Cholsäure hergestellte CDCS.

So kann die klassisch chemische Methode zur UDCS Herstellung wie folgt schematisch dargestellt werden:

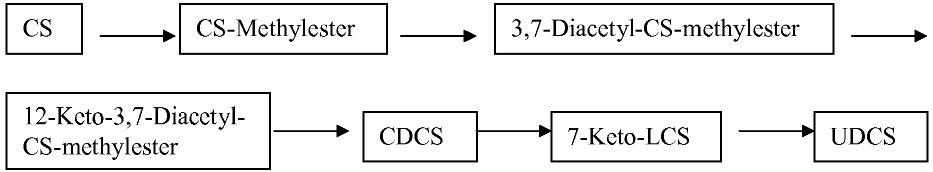

Ein gravierender Nachteil ist unter anderem folgender: da die chemische Oxidation nicht selektiv ist, müssen die Carboxy-Gruppe und die 3α und 7α-Hydroxy-Gruppe durch Veresterung geschützt werden.

Ein alternatives chemisch/enzymatische Verfahren basierend auf der Verwendung des Enzyms 12α-Hydroxysteroid Dehydrogenase (12α-HSDH) kann wie folgt dargestellt werden und ist z.B. beschrieben in der PCT/EP2009/002190 der vorliegenden Anmelderin.

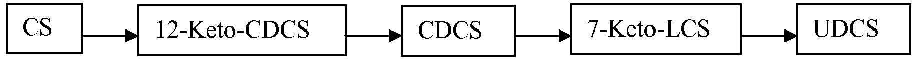

Die 12α-HSDH oxidiert dabei CS selektiv zu 12-Keto-CDCS. Die zwei nach der klassisch chemischen Methode erforderlichen Schützschritte entfallen dabei.

Weiterhin wird von Monti, D., et al., (One-Pot Multienzymatic Synthesis of 12-Ketoursodeoxycholic Acid: Subtle Cofactor Specificities Rule the Reaction Equilibria of Five Biocatalysts Working in a Row. Advanced Synthesis & Catalysis, 2009) ein alternatives enzymatisch-chemisches Verfahren beschrieben, das wie folgt schematisch darstellbar ist:

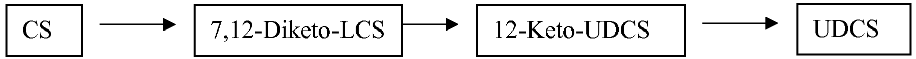

Die CS wird zuerst von 7α-HSDH aus *Bacteroides fragilis* ATCC 25285 (Zhu, D., et al., Enzymatic enantioselective reduction of -ketoesters by a thermostable 7 -hydroxysteroid dehydrogenase from Bacteroides fragilis. Tetrahedron, 2006. 62(18): p. 4535-4539) und 12α-HSDH zu 7,12-Diketo-LCS oxidiert. Diese beiden Enzyme sind jeweils NADH abhängig. Nach der Reduktion durch 7β-HSDH (NADPH abhängig) aus *Clostridium absonum* ATCC 27555 (DSM 599) (MacDonald, I.A. and P.D. Roach, Bile induction of 7 alpha- and 7 beta-hydroxysteroid dehydrogenases in Clostridium absonum. Biochim Biophys Acta, 1981. 665(2): p. 262-9) entsteht 12-Keto-UDCS. Durch Wolff-Kishner-Reduktion wird das Endprodukt erhalten. Nachteilig bei diesem Verfahren ist, dass wegen der Gleichgewichtslage der katalysieren Reaktion eine komplette Umsetzung nicht möglich ist, und dass für die erste Stufe der Umsetzung zwei unterschiedliche Enzyme eingesetzt werden müssen, was das Verfahren verteuert. Zur Kofaktor-Regenerierung werden Lactat Dehydrogenase (LDH; zur Regenerierung von NAD⁺) und Glucose Dehydrogenase (GlcDH oder GDH, zur Regenerierung von NADPH) eingesetzt. Nachteilig bei der dort verwendeten Kofaktor-Regenerierung ist, dass das entstehende Ko-Produkt nur sehr schwer aus dem Reaktionsgemisch zu entfernen ist, so dass das Reaktionsgleichgewicht nicht positiv beeinflusst werden kann, was eine unvollständige Umsetzung des Edukts bedingt.

Eine 7β-HSDH aus Stamm *Collinsella aerofaciens* ATCC 25986 (DSM 3979; ehemalige *Eubacterium aerofaciens*) wurde im Jahr 1982 von Hirano und Masuda beschrieben (Hirano, S. and N. Masuda, Characterization of NADP-dependent 7 beta-hydroxysteroid dehydrogenases from Peptostreptococcus productus and Eubacterium aerofaciens. Appl Environ Microbiol, 1982. 43(5): p. 1057-63). Sequenzinformationen wurden zu diesem Enzym nicht offenbart. Das durch Gelfiltration bestimmte Molekulargewicht betrug 45,000 Da (vgl. Hirano, Seite 1059, linke Spalte). Weiterhin konnte für das dortige Enzym die Reduktion der 7-Oxo-Gruppe zur 7β-Hydroxygruppe nicht beobachtet werden (vgl. Hirano, Seite 1061, Diskussion 1. Absatz). der Fachmann erkennt somit, dass das von Hirano et al beschriebene Enzym nicht zur Katalyse der Reduktion von Dehydrocholsäure (hierin als DHCS oder DHCA bezeichnet) in 7-Position zu 3,12-Diketo-7β-CS geeignet ist.

In der älteren internationalen Patentanmeldung PCT/EP2010/068576 der Anmelderin wird eine neuartige 7β-HSDH aus *Collinsella aerofaciens* ATCC 25986 beschrieben, welche unter anderem ein Molekulargewicht (bei SDS-Gelelektrophorese) von etwa 28-32 kDa, ein Molekulargewicht (bei Gelfiltration, unter nicht denaturierenden Bedingungen, wie insbesondere ohne SDS): von etwa 53 bis 60 kDa, und die Befähigung zur stereoselektiven Reduktion der 7-Carbonyl-Gruppe von 7-Keto-LCS in eine 7β-Hydroxy-Gruppe aufweist.

Außerdem wird in der PCT/EP2010/068576 ein Verfahren zur UDCS Herstellung bereitgestellt, welches schematisch wie folgt darstellbar ist:

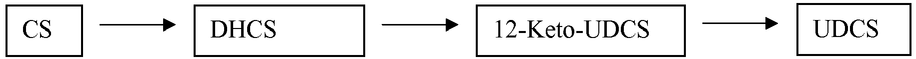

Dabei erfolgt die Oxidation von CS auf klassisch chemischem Weg in einfacher Weise. Die DHCS wird von Enzympaar 7β-HSDH und 3α-HSDH einzeln nacheinander oder in einem Topf zu 12-Keto-UDCS reduziert. Kombiniert mit der Wolff-Kishner-Reduktion kann UDCS somit in nur drei Schritte aus CS synthetisiert werden. Während die 7β-HSDH vom Kofaktor NADPH abhängig ist, benötigt die 3α-HSDH den Kofaktor NADH. Die Verfügbarkeit von Enzympaaren mit Abhängigkeit vom gleichen Kofaktor oder erweiterter Anhängigkeit, (z.B. von den Kofaktoren NADH und NADPH) wäre von Vorteil, weil damit die Kofaktor-Regenerierung vereinfacht werden könnte.

Die WO 2012/080504 beschreibt neuartige Mutanten der 7β-HSDH aus *C*. *aerofaciens* im Sequenzbereich der Aminosäurereste 36 bis 42 der *C*. *aerofaciens* Sequenz, sowie biokatalytische Verfahren zur Herstellung von UDCS, und insbesondere auch neuartige Ganzzell-Verfahren, wobei 7β-HSDH und 3α-HSDH zusammen auf das Substrat einwirken..

Die WO 2011/147957 beschreibt neuartige knock-out Stämme, die zur Herstellung von UDCS besonders geeignet sind, da die unerwünschte 7α-HSDH Enzymaktivität gezielt ausgeschalten werden konnte.

Hwang et al. erwähnen in PLOS ONE (2013) 8, 5, e63594 speziellen Einfachmutanten (P185A, G oder W; T188A, S oder W) und Doppelmutanten (W173F/P185W und W173F/T188W) der 3α-HSDH aus *C*. *testosteronii.* Diese Mutanten, welche N-His-Tags besitzen, werden zur NAD-abhängigen Oxidation des Substrats Androsteron vorgeschlagen. Andere Substrate werden nicht diskutiert.

Aufgabe der Erfindung ist die Bereitstellung weiter verbesserter 3α-HSDHs. Insbesondere sollten Enzym-Mutanten bereitgestellt werden, welche noch vorteilhafter zur enzymatischen oder mikrobiellen Herstellung von UDCS über die stereospezifische Reduktion von DHCS 3-Position eingesetzt werden können, und insbesondere eine verbesserte Aktivität für Substrat und/oder Kofaktor, und/oder eine geringere Substratinhibition und/oder veränderte Kofaktor-Nutzung (erhöhte, veränderte Spezifität oder erweiterte Abhängigkeit) aufweisen.

### Kurzfassung der Erfindung:

Obige Aufgaben konnten überraschenderweise gelöst werden durch die Erzeugung und Charakterisierung von verbesserten Mutanten der 3α-HSDH aus aeroben Bakterien der Gattung *Comamonas,* insbesondere des Stammes *Comamonas testosteroni* und deren Einsatz bei der Umsetzung von Cholsäureverbindungen, insbesondere bei der Herstellung von UDCS.

Auf Grund von Struktur- und Homologiebetrachtungen wurde erfindungsgemäß versucht, Sequenzbereiche zu definieren, die für die Coenzymbindung oder auch die Substraterkennung verantwortlich sein könnten. Damit ergeben sich Möglichkeiten, in diesen Bereichen durch Mutagenese gezielt Aminosäuren zu verändern, um durch diese StrukturVeränderungen Enzymeigenschaften zu verändern. So liegt bei der 3α-HSDH von *Comamonas testosteroni* m Bereich der Aminosäuren um ca. 8 bis 68 der sog. "Rossmann-Fold", der für die Coenzymbindung zuständig ist. Erfindungsgemäß wurde nun insbesondere versucht, Aminosäuren in diesem Bereich der Coenzymbindung derart zu verändern, dass das Enzym statt NADH das NADPH akzeptiert. Beim Versuch, die Aminosäure Leucin in Position 68 durch Alanin zu ersetzen, wurde überraschenderweise gefunden, dass diese 3α-HSDH-Mutante eine deutlich höhere Aktivität aufweist. Das hat sich in der Folge auch bestätigt, als mehrere weitere Mutanten, die alle in Position 68 Mutationen aufwiesen, höhere Aktivitäten als das Wildtyp-Enzym zeigten. Auch Enzym-Mutanten, die bis zur Homogenität aufgereinigt und mit dem entsprechend gereinigten Wildtyp-Enzym verglichen wurden, zeigten deutlich höhere spezifische Enzymaktivitäten.

Eindeutig ist die Verbesserung der Aktivität an der Erhöhung der spezifischen Aktivität, also der auf die Proteinmenge bezogene Aktivitätswert, bei den Mutanten 3α-HSDH [R34N], 3α-HSDH [R34C], 3α-HSDH [L68A], 3α-HSDH [L68C], 3α-HSDH [L68K], 3α-HSDH [L68S] und der Doppelmutante 3α-HSDH [R34N/L68A]. Ein Austausch einer Aminosäure wird durch die Schreibweise kenntlich gemacht, beispielsweise bedeutet R34N, dass das Arginin (R) in Position 34 durch Asparagin (N) ersetzt wurde. Als Wildtyp-Enzym wird die 3α-HSDH, die aus *C*. *testosteroni* gewonnen werden kann oder dieses Enzym mit einem C-terminalen Hexahistidin (His-) Tag versehen, bezeichnet.

Weiterhin wurden überraschend deutlich verbesserte spezifische Aktivitäten bei Einfachmutanten, wie z.B. der 3α-HSDH [T188A], 3α-HSDH [T188S], und Doppelmutanten wie z.B. 3α-HSDH [L68A/T188A] oder 3α-HSDH [L68A/T188S] hinsichtlich der Substrate CDCA und /oder NADH gefunden.

Weiterhin wurde obige Aufgabe gelöst durch Bereitstellung eines biokatalytischen (mikrobiellen bzw. enzymatischen) Prozesses, umfassend die enzymatische Umsetzung von DHCS über zwei durch die hierin beschriebenen 3α-HSDH Mutanten bzw. 7β-HSDH-katalysierte reduktive Teilschritte, die gleichzeitig oder zeitlich versetzt in beliebiger Reihenfolge ablaufen können, zu 12-Keto-UDCS und Kofaktorregenerierung durch Verwendung von Dehydrogenasen, welche den verbrauchen Kofaktor aus beiden reduktiven Teilschritten regenerieren.

### Figurenbeschreibung:

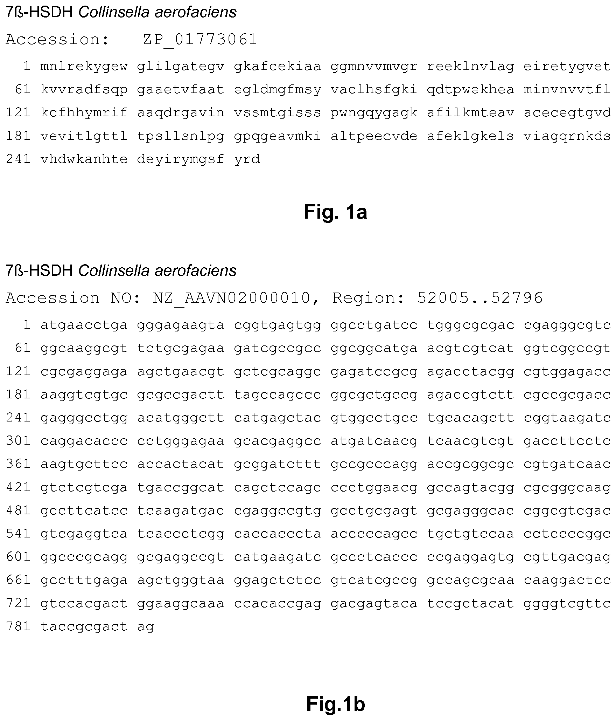

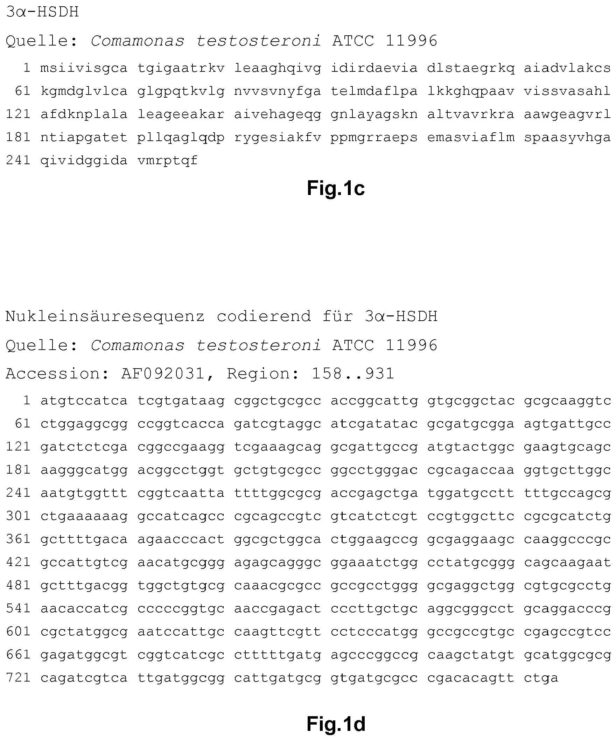

Figur 1a zeigt die Aminosäuresequenz der 7β-HSDH aus Collinsella aerofaciens (SEQ ID NO:2) und Figur 1b die kodierende Nukleinsäuresequenz (SEQ ID NO:1) zur Aminosäuresequenz von Figur 1a; Figur 1c zeigt die Aminosäuresequenz der 3α-HSDH aus *Comamonas testosteroni* (SEQ ID NO:4) und Figur 1d die kodierende Nukleinsäuresequenz (SEQ ID NO: 3) zur Aminosäuresequenz von Figur 1c;

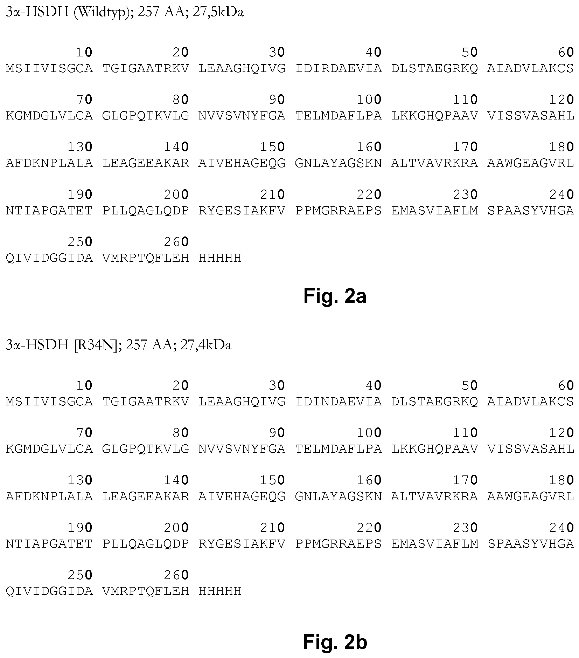

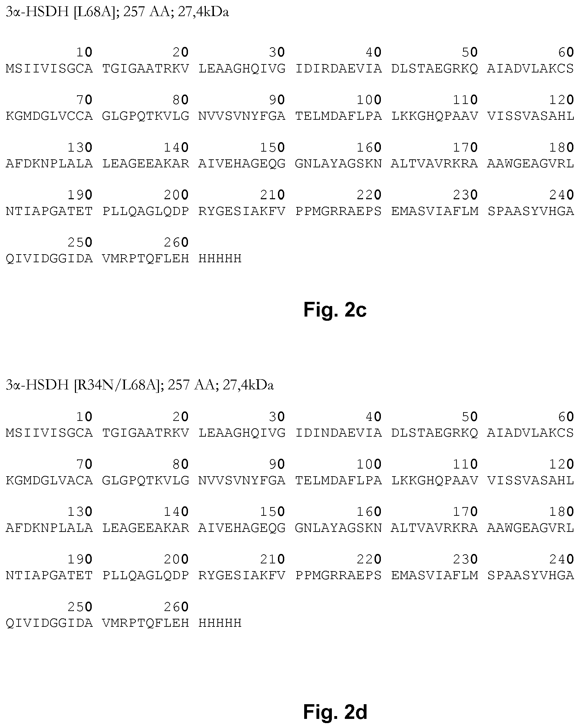

Figur 2a zeigt die Aminosäuresequenz der 3α -HSDH aus *Comamonas testosteroni* C-terminal verlängert um die His-Tag Sequenz LEHHHHHH; Figur 2b die davon abgeleitete Mutante 3α -HSDH [R34N]; Figur 2c die davon abgeleitete Mutante3α -HSDH [L68A]; Figur 2d die davon abgeleitete Mutante3α -HSDH [R34N/L68A];

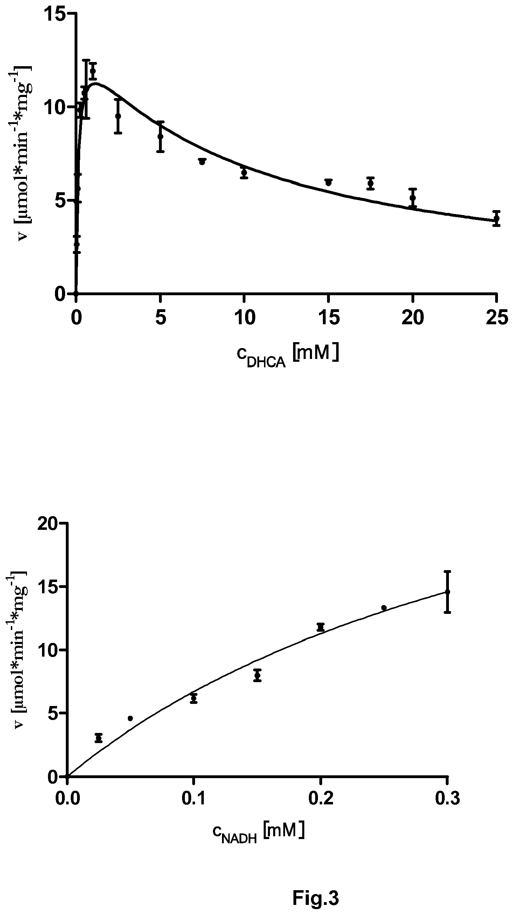

Figur 3 zeigt die Michaelis-Menten-Kinetik der aufgereinigten 3α-HSDH mit His-tag für das Substrat DHCA (oberes Bild) bzw. für den Cofaktor NADH (unteres Bild).

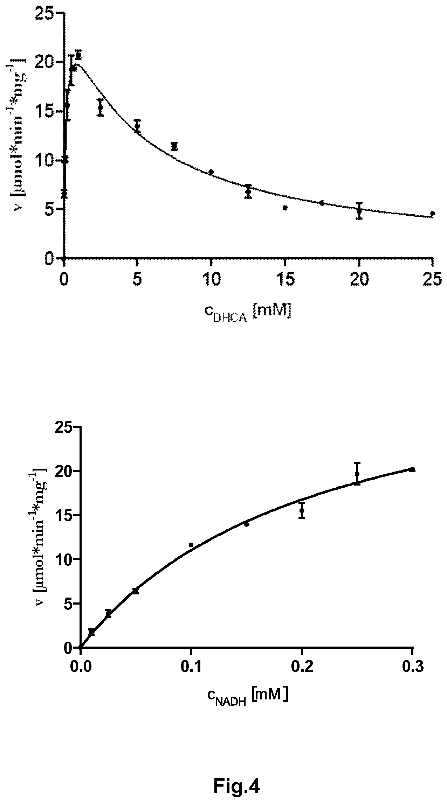

Figur 4 zeigt die Michaelis-Menten-Kinetik der Alanin-Mutante [L68A] für das Substrat DHCA (oberes Bild) und für das Coenzym NADH (unteres Bild)

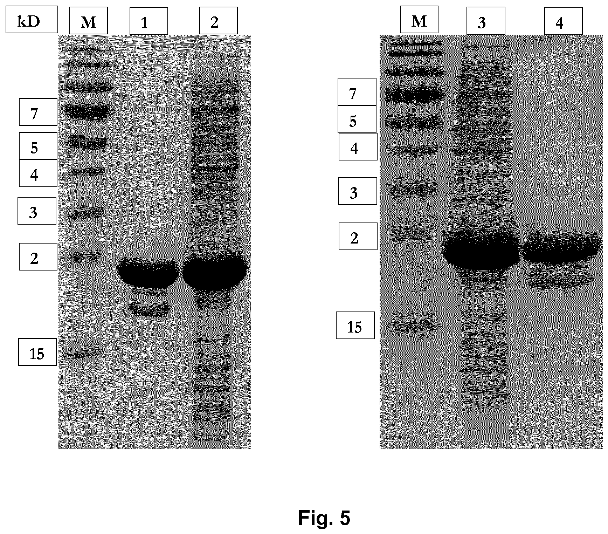

Figur 5 zeigt das SDS-Gel der 3α-HSDH [R34N/L68A] (linkes Bild) sowie 3α-HSDH [L68A] (rechtes Bild) und nach Expression in Schüttelkolben-Fermentation in LB. Als lösliche Fraktion ist der zellfreie Rohextrakt aufgetragen. Die 3α-HSDHs haben eine Größe von ca. 27,4 kDa. Aufgetragen wurden ca. 10 µg Protein. Legende: 1 = aufgereinigte 3α-HSDH [R34N/L68A]; 2 = Rohextrakt 3α-HSDH [R34N/L68A]; 3 = aufgereinigte 3α-HSDH [L68A]; 4 = Rohextrakt 3α-HSDH [L68A]

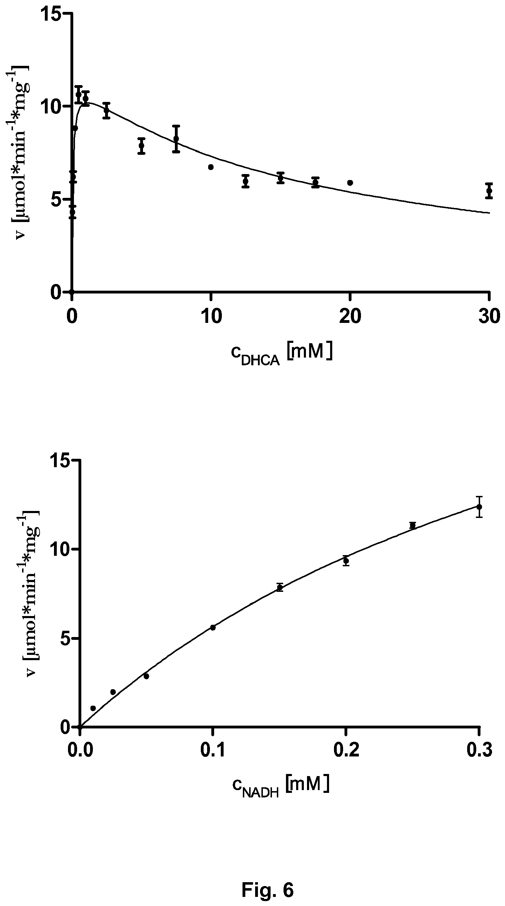

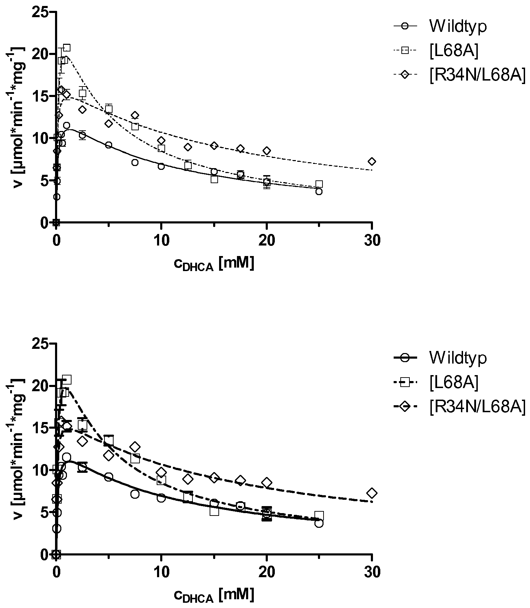

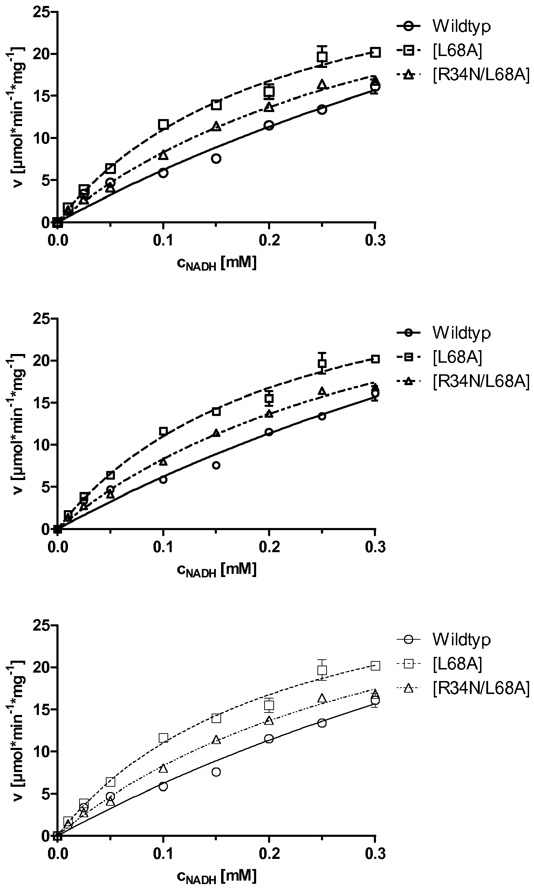

Figur 6 zeigt die Michaelis-Menten-Kinetik der Doppelmutante [R34N/L68A] für das Substrat DHCA (oberes Bild) und für das Coenzym NADH (unteres Bild)

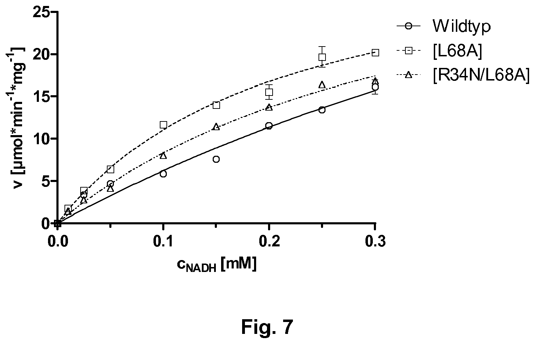

Figur 7 zeigt den Vergleich der kinetischen Konstanten von Wildtyp-Enzym, der Mutante L68A und der Doppelmutante R34N/L68A. (Substrat DHCA: oberes Bild und Coenzym NADH: unteres Bild)

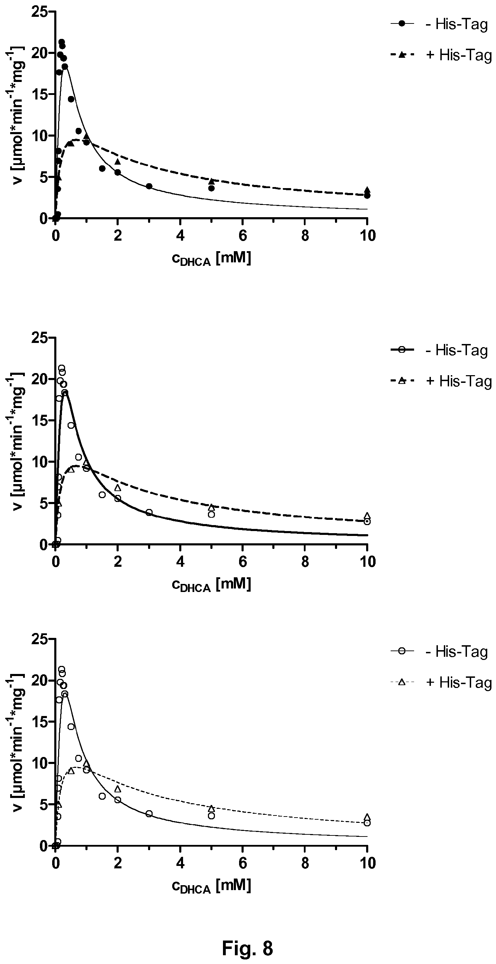

Figur 8 zeigt die Michaelis-Menten-Kinetik der Wildtyp 3α-HSDH für das Substrat DHCA, aufgenommen jeweils mit Rohextrakt. Die Kurve mit Punkten zeigt die Abhängigkeit der Reaktionsgeschwindigkeit von der Substratkonzentration für das Enzym ohne His-Tag, die Kurve mit Dreiecken für das Enzym mit C-terminalem His-Tag.

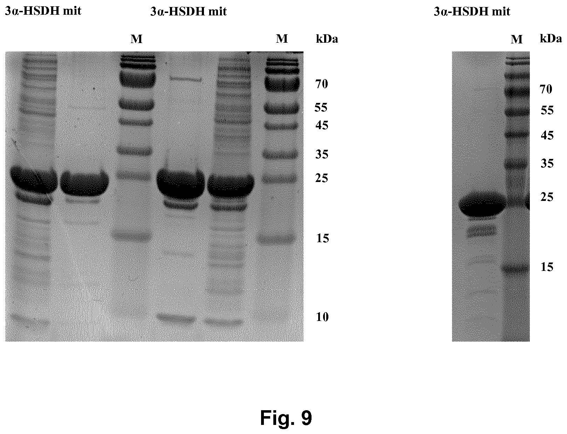

Figur 9 zeigt das SDS-Gel der 3α-HSDH mit 3xHis- und 9xHis-Tag (linkes Bild) sowie dem Standard 6xHis-Tag (rechtes Bild). Die Expression wurde in Schüttelkolben-Fermentation in LB-Medium durchgeführt. Als lösliche Fraktion ist der zellfreie Rohextrakt sowie das aufgereinigte Protein aufgetragen. Die 3α-HSDH 3xHis hat eine Größe von ca. 27,1 kDa, die 6xHis eine von ca. 27,5 kDa und die 9xHis eine Größe von ca. 27,9 kDa. Aufgetragen wurden ca. 10 µg Protein.

### Spezielle Ausführungsformen der Erfindung

Die Erfindung betrifft insbesondere folgende spezielle Ausführungsformen:

1. 3α-Hydroxysteroiddehydrogenase (3α-HSDH), welche wenigstens die stereospezifische enzymatische Reduktion eines 3-Ketosteroids zum korrespondierenden 3-Hydroxysteroid (wie z.B. von 3,12-Diketo-7β-CS oder DHCS zu 12-Keto-UDCS oder 7,12-Diketo-3α-CS (7,12-Diketo-LCS), insbesondere unter stöchiometrischem Verbrauch von NADH und/oder NADPH, insbesondere NADH) katalysiert, wobei das Enzym eine Mutation in Position 34 und/oder 68 von SEQ ID NO:4 oder in den korrespondierenden Sequenzpositionen einer davon abgeleiteten Aminosäuresequenz mit wenigstens 80% Sequenzidentität, wie z.B. wenigstens 85, 90, 91, 92, 93, 94, 95, 96, 97, 98, 99 oder 99,5% Sequenzidentität, zu SEQ ID NO:4 umfasst und gegebenenfalls zusätzlich C-terminal um einen Rest umfassend 1 bis 10 Histidin-Reste verlängert ist, wobei die C-terminale Kettenverlängerung bis zu 25, insbesondere bis zu 15, 14. 13, 12, 11 oder 10 gleiche oder verschiedene (proteinogene) Aminosäurereste umfassen kann und beispielsweise eine Peptidkette ist, die 3xHis, 6xHis oder 9xHis umfasst, wobei die Histidinreste direkt an den C-Terminus oder über eine 1 bis 10, insbesondere 1 bis 3 beliebigen Aminosäurereste umfassende Linkergruppe angebunden sein können. Beispielhaft sind zu nennen: LEHHH, LEHHHHHH und LEHHHHHHHHH. Weiterhin sind Gegenstand solche 3α-HSDHs, die zusätzlich zu einer oder mehreren der obigen Mutationen N-terminal um einen Rest umfassend 1 bis 10 Histidin-Reste Verlängert ist, wobei die N-terminale Kettenverlängerung bis zu 25, 24, 23, 22, 21, 20, 19, 18, 17, 16, 15, 14,13, 12, 11 oder 10 gleiche oder verschiedene (proteinogene) Aminosäurereste umfassen kann und beispielsweise eine Peptidkette ist, die 3xHis, 6xHis oder 9xHis umfasst, wobei die Histidinreste direkt an den N-Terminus oder über eine 1 bis 10 beliebigen Aminosäurereste umfassende Linkergruppe angebunden sein können. Beispielhaft ist zu nennen: MGSSHHHHHHSSGLVPRGSH-.

Beispielsweise weisen bevorzugte 3α-HSDHs eine Mutation in Position 34 und/oder 68 auf und sind ggf. N-terminal oder C-terminal zusätzlich verlängert; oder sie sind nur N-terminal oder C-terminal verlängert; jeweils wie hierin näher definiert.

Weiterhin umfasst die Erfindung eine 3α-HSDH, die wenigstens 80%, wie z.B. wenigstens 85, 90, 91, 92, 93, 94, 95, 96, 97, 98, 99 oder 99,5 100% Sequenzidentität, zu SEQ ID NO:4 besitzt und beispielsweise ein natives, nichtmutiertes Enzym gemäß SEQ ID NO:4 ist und welche C-terminal um einen Rest umfassen 9 konsekutive Histidin-Reste verlängert ist.

Bei den Mutationen in Position 34 und/oder 68 von SEQ ID NO:4 der erfindungsgemäßen 3α-HSDHs handelt es sich um die Aminosäuresubstitutionen R34X₁, L68X₂ und R34X₁/ L68X₂, wobei X₁ für N, C, S oder L steht und X₂ für A, C, K, S oder V steht.

2. 3α-HSDH die wenigstens die stereospezifische enzymatische Reduktion eines 3-Ketosteroids zum korrespondierenden 3-Hydroxysteroid katalysiert, und
eine durch Aminosäuremutation in SEQ ID NO:4 veränderte Aminosäure-Sequenz aufweist, wobei die Aminosäuresequenz-Mutation ausgewählt ist unter:
a) den Einfachmutanten
   R34X₁ und
   L68X₂ und den
b) den Doppelmutanten
   R34X₁/L68X₂
   worin
   X₁ für N, C, S oder L steht;
   X₂ für A, C, K, S oder V steht;
   wobei besonders bevorzugte Kombinationen von X₁, X₂ X₁ = N und /oder X₂ = A umfassen,
c) C-terminal um eine 9 konsekutive Histidin Reste umfassende Peptidkette verlängerte Additionsmutanten; wobei z.B. die Histidinreste direkt an den C-Terminus oder über eine 1 bis 10, insbesondere1 bis 3 Aminosäurereste umfassende Linkergruppe angebunden sein können;
d) Mutanten gemäß a) und b), jeweils zusätzlich C-terminal um einen Rest umfassend 1 bis 10 Histidin-Reste verlängert und/oder N-terminal um eine 1 bis 10 Histidin Reste umfassende Peptidkette verlängerte Additionsmutanten; wobei z.B. die Histidinreste direkt an den N-Terminus oder über eine 1 bis 10 Aminosäurereste umfassende Linkergruppe angebunden sein können;
   wobei die mutierte, d.h. veränderte Aminosäuresequenz eine Sequenzidentität zu SEQ ID NO:4 von 80% bis weniger als 100%, wie z.B. 85, 90, 91, 92, 93, 94, 95, 96, 97, 98, 99 oder 99,5% Sequenzidentität, aufweist.

Nichtlimitierende Beispiele geeigneter Mutanten sind
Einfachmutanten umfassend: [R34N], [R34C], [R34S], [R34L], [L68A], [I68C], [L68K], [L68S] und [L68V]
Doppelmutanten umfassend: [R34N/L68A]
jeweils sowohl ohne His-Tag als auch mit C-terminalen bzw. N-terminalen His-Tag.

Die erfindungsgemäßen 3α-HSDHs zeigen im Vergleich zur 3α-HSDH mit SEQ ID NO:4 folgendes Eigenschaftsprofil:
i) eine erhöhte spezifische Aktivität (vₘₐₓ [U/mg]) für Dehydrocholsäure (DHCA) bei der enzymatischen Reduktion von DHCA mit NADH als Cofaktor,
ii) eine verringerte Substratinhibition durch DHCA,
iii) eine erhöhte spezifische Aktivität (vₘₐₓ [U/mg]) für NADH bei der enzymatischen Reduktion von DHCA mit NADH als Cofaktor,
   wobei diese Eigenschaften i) bis iii) einzeln oder in beliebiger Kombination vorliegen können.

3. 3α -HSDH nach einem der vorhergehenden Ausführungsformen, die im Vergleich zur nativen, nicht-mutierten 3α-HSDH mit SEQ ID NO:4 folgendes Eigenschaftsprofil zeigt:
a. eine erhöhte spezifische Aktivität (Vₘₐₓ [U/mg]) für Dehydrocholsäure (DHCA) bei der enzymatischen Reduktion von DHCA mit NADH als Kofaktor; wobei z.B. die spezifische Aktivität (U/mg) in Gegenwart des Kofaktors NAD(P)H im Vergleich zum nicht-mutierten Enzym um wenigsten 1, 5, 10, 50 oder 100 % , insbesondere aber wenigstens um das 1-fache, insbesondere um das 2- bis 100-fache oder 3- bis 20-fache oder 5- bis 10-fache erhöht ist,
b. eine verringerte Substratinhibition durch DHCA, wie z.B. mit Kᵢ-Werten im Bereich von >10 mM, wie z.B. bei 11 bis 200 mM, 12 bis 150 mM, 15 bis 100 mM,
c. wobei diese Eigenschaften a) und b) einzeln oder in beliebiger Kombination vorliegen können, d.h. nur a), nur b), nur c), a) und b), a) und c) oder b) und c).

Ferner ist hierin eine 3α-Hydroxysteroiddehydrogenase (3α-HSDH) beschrieben, welche wenigstens die stereospezifische enzymatische Reduktion eines 3-Ketosteroids zum korrespondierenden 3-Hydroxysteroid, (wie z.B. von 3,12-Diketo-7β-CS oder DHCS zu 12-Keto-UDCS oder 7,12-Diketo-3α-CS (7,12-Diketo-LCS), insbesondere unter stöchiometrischem Verbrauch von NADH und/oder NADPH, vor allem unter stöchiometrischem Verbrauch von NADH, katalysiert, wobei das Enzym eine Mutation in Position 188 und/oder Position 185 und gegebenenfalls Position 68 von SEQ ID NO:4 oder in den korrespondierenden Sequenzpositionen einer Aminosäuresequenz mit wenigstens 80%, wie z. B. wenigstens wie z.B. 85, 90, 91, 92, 93, 94, 95, 96, 97, 98, 99 oder 99,5%,Sequenzidentität zu SEQ ID NO:4 umfasst; und / oder C-terminal eine Kettenverlängerung um bis zu 15, 14, 13, 12, 11 oder 10 gleiche oder verschiedene (proteinogene) Aminosäurereste, insbesondere um eine Peptidkette, umfassend 1 bis 10, wie z.B. 3 bis 9 basische Aminosäurereste, wie Lysin, Arginin oder insbesondere Histidin (wie 3xHis, 6xHis oder 9xHis), aufweist; wobei die Histidinreste direkt an den C-Terminus oder über eine 1 bis 3 beliebigen Aminosäurereste umfassende Linkergruppe angebunden sein können. Beispielhaft sind zu nennen: LEHHH, LEHHHHHH und LEHHHHHHHHH. Weiterhin sind solche 3α-HSDHs beschrieben, die zusätzlich zu einer oder mehreren der obigen Mutationen oder alternativ dazu, N-terminal eine Kettenverlängerung um bis zu 25 gleiche oder verschiedene Aminosäurereste aufweisen. Diese sind um bis zu 25, 24, 23, 22, 21, 20, 19, 18, 17, 16, 15, 14.,13, 12, 11 oder 10 gleiche oder verschiedene (proteinogene) Aminosäurereste, insbesondere um eine Peptidkette, umfassend 1 bis 10, wie z.B. 3 bis 9 basische Aminosäurereste, wie Lysin, Arginin oder insbesondere Histidin (wie 3xHis, 6xHis oder 9xHis), verlängert; wobei die Histidinreste direkt an den N-Terminus oder über eine 1 bis 10 beliebigen Aminosäurereste umfassende Linkergruppe angebunden sein können. Beispielhaft ist zu nennen: MGSSHHHHHHSSGLVPRGSH-.

Beispielsweise weisen derartige 3α-HSDHs je eine Mutation in Position 188 und/oder 185 und ggf. in Position 68 auf und sind ggf. N-terminal oder C-terminal zusätzlich verlängert; oder sie sind nur N-terminal oder C-terminal verlängert; jeweils wie hierin näher definiert.

Ferner ist hierin ein 3α-HSDH beschrieben, die wenigstens die stereospezifische enzymatische Reduktion eines 3-Ketosteroids zum korrespondierenden 3-Hydroxysteroid katalysiert, und
eine durch Aminosäuremutation in SEQ ID NO:4 veränderte Aminosäure-Sequenz aufweist, wobei die Aminosäuresequenz-Mutation ausgewählt ist unter Ein- oder Mehrfachmutationen umfassend:
a) P185X₁ und/oder
b) T188X₃
   und gegebenenfalls zusätzlich
c) L68X₂
   wobei X₁ für einen von Prolin (P) verschiedenen (proteinogenen) Aminosäurerest steht;
   X₂ für einen von Leucin (L) verschiedenen (proteinogenen) Aminosäurerest steht;
   X₃ für einen von Threonin (T) verschiedenen (proteinogenen) Aminosäurerest steht;
d) gegebenenfalls zusätzlich mit C-terminaler Verlängerung umfassend 1 bis 10 Histidin (H) Reste; und/oder gegebenenfalls zusätzlich mit N-terminaler Verlängerung umfassend 1 bis 10 Histidin (H) Reste.
wobei die mutierte, d.h. veränderte Aminosäuresequenz eine Sequenzidentität zu SEQ ID NO:4 von 80% bis weniger als 100%, wie z.B. 85, 90, 91, 92, 93, 94, 95, 96, 97, 98, 99 oder 99,5% Sequenzidentität, aufweist.

Die oben beschriebenen 3α-HSDHs mit Mutation(en) in Position 188 und/oder Position 185 und gegebenenfalls Position 68 können beispielsweise ausgewählt sein unter
a) den Einfachmutanten
   P185X₁
   T188X₃
b) den Doppelmutanten
   P185X₁/T188X₃;
   L68X₂/T188X₃;
   L68X₂/T185X₁ ;
   worin
   X₁ für A, T, S, G oder V, insbesondere A steht;
   X₂ für A, G, C, K, S oder V, insbesondere A, steht; und
   X₃ für A, G, W, S oder V, insbesondere A oder S steht;
c) gegebenenfalls zusätzlich mit C-terminaler Verlängerung umfassend 1 bis 10 Histidin-Reste, wobei z.B. die Histidinreste direkt an den C-Terminus oder über eine 1 bis 10 Aminosäurereste umfassende Linkergruppe angebunden sein können. und; und/oder
d) gegebenenfalls zusätzlich mit N-terminaler Verlängerung umfassend 1 bis 10 Histidin-Reste; wobei z.B. die Histidinreste direkt an den N-Terminus oder über eine 1 bis 10 Aminosäurereste umfassende Linkergruppe angebunden sein können. und
e) Mutanten gemäß a) und b), jeweils zusätzlich enthaltend die Additionsmutation gemäß c) und /oder d), insbesondere gemäß c) oder d).

Insbesondere sind zu nennen die Doppelmutanten P185X₁/T188X₃; L68X₂/T188X₃; L68X₂/T185X₁; ohne bzw. mit N- oder C-terminaler Verlängerung wie oben definiert.

Nichtlimitierende Beispiele geeigneter Mutanten sind:
Einfachmutanten umfassend: [T188A], [T188S], [T188G], [T188V], [T188W], [P185A], [P185T], [P185S], [P185G], [P185V], [P185A],
Doppelmutanten umfassend: [L68A/T188A] and [L68A/T188S]
jeweils sowohl ohne His-Tag als auch mit C-terminalen bzw. N-Terminalen His-Tag.

Die oben beschriebenen 3α-HSDHs mit Mutation(en) in Position 188 und/oder Position 185 und gegebenenfalls Position 68 können im Vergleich zur nativen, nicht-mutierten 3α-HSDH mit SEQ ID NO:4 beispielsweise folgendes Eigenschaftsprofil zeigen:
a. eine erhöhte spezifische Aktivität (vₘₐₓ [U/mg]) für Dehydrocholsäure (DHCA) bei der enzymatischen Reduktion von DHCA mit NADH als Cofaktor; wobei z.B. die spezifische Aktivität (U/mg) in Gegenwart des Kofaktors NADH im Vergleich zum nichtmutierten Enzym um wenigsten 1, 5, 10, 50 oder 100 % , insbesondere aber wenigstens um das 1-fache, insbesondere um das 2- bis 100-fache oder 3- bis 20-fache oder 5-bis 10-fache erhöht ist,
b. eine verringerte Substratinhibition durch DHCA, wie z.B. mit Ki-Werten im Bereich von >10 mM, wie z.B. bei 11 bis 200 mM, 12 bis 150 mM, 15 bis 100 mM,
c. eine erhöhte spezifische Aktivität (vₘₐₓ [U/mg]) für NADH bei der enzymatischen Reduktion von DHCA mit NADH als Cofaktor. wobei z.B. die spezifische Aktivität (U/mg) in Gegenwart des von DHCA im Vergleich zum nichtmutierten Enzym um wenigsten 1, 5 , 10 , 50 oder 100 % , insbesondere aber wenigstens um das 1-fache, insbesondere um das 2- bis 100-fache oder 3- bis 20-fache oder 5- bis 10-fache erhöht ist, wobei diese Eigenschaften a) bis c) einzeln oder in beliebiger Kombination vorliegen können, d.h. nur a), nur b), nur c), a) und b), a) und c) oder b) und c).

4. Nukleotidsequenz kodierend für eine 3α-HSDH nach einem der vorhergehenden Ausführungsformen 1 bis 3.
Beispielhaft zu nennen sind Nukleinsäuresequenzen, ausgewählt unter Nukleinsäuresequenzen
a) gleichzeitig kodierend für eine GDH und eine 3α-HSDH Mutante nach einer der Ausführungsformen 1 bis 8 und gegebenenfalls eine 7β-HSDH;
b) kodierend für ein Fusionsprotein umfassend eine GDH und eine 3α-HSDH Mutante nach einer der Ausführungsformen 1 bis 3 und gegebenenfalls eine 7β-HSDH;
wobei die kodierenden Sequenzen unabhängig voneinander ein- oder mehrfach in dem Konstrukt, wie z.B. in 2, 3, 4, 5, oder 6 bis 10 Kopien, enthalten sein können. Durch Wahl der geeigneten Kopienzahl können so eventuell gegebene Aktivitätsunterschiede in den einzelnen Expressionsprodukten kompensiert werden.

5. Expressionskassette, umfassend unter der Kontrolle wenigstens einer regulativer Sequenz wenigstens eine Nukleotidsequenz nach Ausführungsform 4 sowie ggf. kodierende Sequenzen für wenigstens ein (wie z.B. 1, 2 oder 3) weiteres Enzym, ausgewählt unter Hydroxysteroiddehydrogenasen, insbesondere 7β-HSDH, und zur Kofaktorregenerierung geeignete Dehydrogenasen, wie z.B. FDH, GDH, ADH, G-6-PDH, PDH. Insbesondere können die in einer Expressions-Kassette enthaltenen Enzyme verschiedene, bevorzugt aber gleiche Kofaktorenpaare nutzen, wie z.B. das Kofaktorenpaar NAD⁺/NADH oder NADP⁺/NADPH.

6. Expressionsvektor, umfassend wenigstens eine Expressionskassette nach Ausführungsform 5.

7. Rekombinanter Mikroorganismus, der wenigstens eine Nukleotidsequenz gemäß Ausführungsform 4 oder wenigstens eine Expressionskassette nach Ausführungsform 5 oder wenigstens einen Expressionsvektor nach Ausführungsform 6 trägt.

8. Rekombinanter Mikroorganismus nach Ausführungsform 7, der zusätzlich gegebenenfalls die kodierende Sequenz für wenigstens ein weiteres Enzym, ausgewählt unter Hydroxysteroiddehydrogenasen (HSDH) und zur Kofaktorregenerierung geeignete Dehydrogenasen, trägt.

9. Rekombinanter Mikroorganismus nach Ausführungsform 8, wobei die weitere HSHD ausgewählt ist unter 7β-HSDHs; und
die Dehydrogenase ausgewählt ist unter NADPH-regenerierenden Enzymen, wie NADPH Dehydrogenasen, Alkoholdehydrogenasen (ADH), und NADPH regenerierenden Formiatdehydrogenasen (FDH), sowie Glucosedehydrogenase (GDH), Glucose-6-Phosphat-Dehydrogenase (G-6-PDH), oder Phosphit-Dehydrogenasen (PtDH), oder NADH-regenerierenden Enzymen, wie NADH Dehydrogenasen, NADH regenerierenden Formiatdehydrogenasen (FDH), NADH regenerierenden Alkoholdehydrogenasen (ADH), NADH regenerierenden Glucose-6-Phosphat-Dehydrogenasen (G6PDH), NADH regenerierenden Phosphitdehydrogenasen (PtDH) sowie NADH regenerierenden Glucosedehydrogenasen (GDH).

Beispielsweise ist zu nennen ein rekombinanter Mikroorganismus, welcher zur gleichzeitigen Expression einer erfindungsgemäßen 3α-HSDH Mutante, einer hierin beschriebenen GDH und ggf. einer hierin beschriebenen 7β-HSDH befähigt ist.

Rekombinanter Mikroorganismus welche die kodierenden Sequenzen für 3α-HSDH Mutante, GDH bzw. Mutanten davon und 7β-HSDH auf einem oder mehreren (verschiedenen) Expressionskonstrukten trägt. Gegenstand der Erfindung sind somit rekombinante Mikroorganismen, welche mit einem Einplasmidsystem modifiziert (wie z.B. transformiert) sind, das die kodierenden Sequenzen für 3α -HSDH Mutante, GDH bzw. Mutanten davon und 7β-HSDH bzw. Mutanten davon in einer oder mehreren Kopien, wie z.B. in 2, 3, 4, 5, oder 6 bis 10 Kopien, tragen. Gegenstand der Erfindung sind somit auch rekombinante Mikroorganismen, welche mit einem Einplasmidsystem modifiziert (wie z.B. transformiert) sind, das die kodierenden Sequenzen für 7β-HSDH bzw. Mutanten davon, GDH bzw. Mutanten davon und 3α-HSDH bzw. Mutanten davon in einer oder mehreren Kopien, wie z.B. in 2, 3, 4, 5 oder 6 bis 10 Kopien tragen. Die Enzyme (7β-HSDH, GDH und 3α-HSDH bzw. deren Mutanten) können aber auch auf 2 oder 3 getrennten, miteinander kompatiblen Plasmiden in einer oder mehreren Kopien enthalten sein. Geeignete Basisvektoren zur Herstellung von Einplasmidsystemen und Multicopy-Plasmiden sind dem Fachmann bekannt. Als Beispiele können genannt werden für Einplasmidsystem, z.B. pET21aund für Multicopyplasmide z.B. die von der Firma Novagen vertriebenen Duet-Vektoren, wie pACYCDuet-1, pETDuet-1, pCDFDuet-1, pRSFDuet-1 und pCOLADuet-1. Derartige Vektoren, deren Kompatibilität mit anderen Vektoren und mikrobiellen Wirtsstämmen sind z.B. dem "User Protocol TB340 Rev. E0305 der Firma Novagen zu entnehmen.

Die optimale Kombination von Enzymen für die Generierung von Plasmidsystemen kann der Fachmann unter Berücksichtigung der Lehre der vorliegenden Erfindung ohne unzumutbaren Aufwand vornehmen. So kann der Fachmann beispielsweise in Abhängigkeit von der Kofaktorspezifität des jeweils verwendeten HSDH-Enzyms das zur Kofaktorregenerierung am besten geeignete Enzym, ausgewählt unter den oben genannten Dehydrogenasen, insbesondere GDH und den jeweiligen Mutanten davon, auswählen.

Weiterhin besteht die Möglichkeit, die zur Umsetzung gewählten Enzyme auf zwei oder mehrere Plasmide zu verteilen und mit den so hergestellten Plasmiden zwei oder mehrere verschiedene rekombinante Mikroorganismen herzustellen, die dann gemeinsam für die erfindungsgemäße biokatalytische Umsetzung eingesetzt werden. Die jeweilige zur Herstellung des Plasmids verwendete Enzymkombination kann dabei insbesondere auch unter der Vorgabe einer vergleichbaren Kofaktor-Nutzung erfolgen. So kann beispielsweise ein erster Mikroorganismus mit einem Plasmid modifiziert werden, welches die kodierende Sequenz für eine 7β-HSDH oder Mutante davon und eine GDH tragen. Ein zweiter Mikroorganismus kann dagegen mit einem Plasmid modifiziert sein, das die kodierende Sequenz für eine 3α-HSDH oder Mutante davon und die kodierende Sequenz für eine GDH trägt. Beide Enzympaare können so gewählt sein dass sie gleiche Kofaktoren-Paare regenerieren können. Beide Mikroorganismen können dann gleichzeitig zur erfindungsgemäßen biokatalytischen Umsetzung eingesetzt werden.

Die Verwendung von zwei getrennten Biokatalysatoren (rekombinanter Mikroorganismen) kann gegenüber der Verwendung nur eines Biokatalysators, in dem alle Syntheseenzyme exprimiert werden, zwei wesentliche Vorteile bieten:
a) Beide Biokatalysatoren können separat voneinander gentechnisch modifiziert und optimiert werden. Insbesondere ist der Einsatz von verschiedenen Kofaktorregenerierungsenzymen möglich, die entweder auf NADH- oder auf NADPH-Regenerierung optimiert sind.
b) Für die Biokatalyse können die Biokatalysatoren in unterschiedlichen Anteilen eingesetzt werden. Dies ermöglicht ein Eingreifen in die einzelnen Reaktionsgeschwindigkeiten des Multienzymprozesses während der Biokatalyse, selbst nachdem sämtliche Biokatalysatoren bereits hergestellt sind.

10. Rekombinanter Mikroorganismus nach einer der Ausführungsformen 7 bis 9 welcher ein 7α-HSDH knock-out-Stamm ist, wie z.B. beschrieben in der WO2011/147957.

11. Verfahren zur enzymatischen oder mikrobiellen Synthese von 3α-Hydroxysteroiden, wobei man das korrespondierende 3-Ketosteroid in Gegenwart einer 3α-HSDH gemäß der Definition in einer der Ausführungsformen 1 bis 3 oder in Gegenwart eines diese 3α-HSDH exprimierenden rekombinanten Mikroorganismus nach einer der Ausführungsformen 7 bis 10 reduziert, und gegebenenfalls wenigstens ein gebildetes Reduktionsprodukt aus dem Reaktionsansatz isoliert.

12. Verfahren nach Ausführungsform 11, wobei das 3-Ketosteroid ausgewählt ist unter Dehydrocholsäure (DHCA),
den Derivaten davon, wie insbesondere einem Salz, Amid oder Alkylester der Säure.

13. Verfahren nach Ausführungsform 11 oder 12, wobei die Reduktion in Gegenwart und insbesondere unter Verbrauch von NADPH und/oder NADH erfolgt.

14. Verfahren nach Ausführungsform 13, wobei verbrauchtes NADPH durch Kopplung mit einem NADPH-regenerierenden Enzym regeneriert wird, wobei dieses insbesondere ausgewählt ist unter NADPH Dehydrogenasen, Alkoholdehydrogenasen (ADH), und NADPH regenerierenden Formiatdehydrogenasen (FDH) und einer NADPH-regenerierenden Glucosedehydrogenase (GDH), wobei das NADPH-regenerierende Enzym gegebenenfalls von einem rekombinanten Mikroorganismus exprimiert wird; und/oder wobei verbrauchtes NADH durch Kopplung mit einem NADH-regenerierenden Enzym regeneriert wird, wobei dieses insbesondere ausgewählt ist unter NADH-Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH), wobei das NADH-regenerierende Enzym ggf. in einem rekombinanten Mikroorganismus exprimiert wird

15. Verfahren nach Ausführungsform 14, wobei das NADPH-regenerierende Enzym ausgewählt ist unter
a. FDHs, einschließlich Mutanten einer NAD⁺-abhängigen FDH, welche wenigstens die enzymatische Oxidation von Ameisensäure zu CO₂ katalysiert, wobei die Mutante im Vergleich zum nicht-mutierten Enzym zusätzlich NADP⁺ als Kofaktor akzeptiert; und
b. GDHs.

16. Verfahren zur Herstellung von Ursodesoxycholsäure (UDCS) der Formel (1) 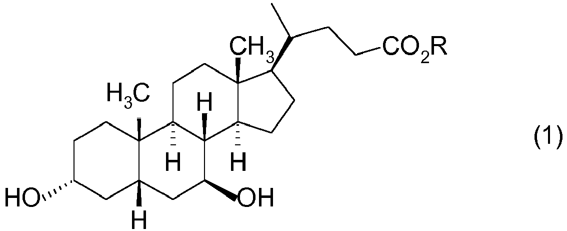 worin
R für Alkyl, H, ein Alkalimetallion oder N(R³)₄⁺steht, worin die Reste R³ gleich oder verschieden sind und für H oder Alkyl stehen, oder die Gruppe -CO₂R durch die Säureamid-Gruppe -CONR¹R², ersetzt ist, worin R¹ und R² unabhängig voneinander für einen Alkylrest stehen;
wobei man
a) gegebenenfalls eine Cholsäure (CS) der Formel (2) 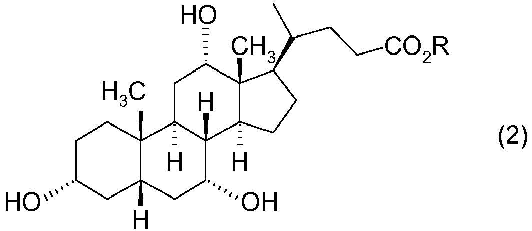 worin R die oben angegebenen Bedeutungen besitzt oder die Gruppe -CO₂R durch die Säureamid-Gruppe -CONR¹R², wie oben definiert ersetzt ist,
   zur Dehydrocholsäure (DHCS) der Formel (3) 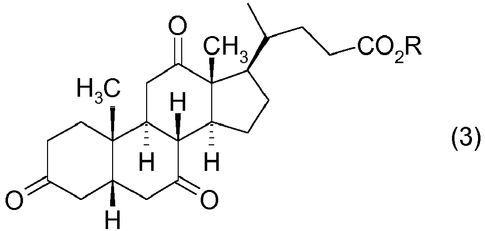 worin R die oben angegebenen Bedeutungen besitzt, oder die Gruppe -CO₂R durch die Säureamid-Gruppe -CONR¹R², wie oben definiert ersetzt ist, chemisch oxidiert;
b) DHCS in Gegenwart wenigstens einer 3α-HSDH-Mutante gemäß der Definition in einer der Ausführungsformen 1 bis 3 (vorliegend als isoliertes Enzym oder exprimiert von einer entsprechenden rekombinanten Mikroorganismus) und in Gegenwart wenigstens einer 7β-HSDH (vorliegend als isoliertes Enzym oder exprimiert von einer entsprechenden rekombinanten Mikroorganismus) zur korrespondierenden 12-Keto-ursodesoxychiolsäure (12-Keto UDCS) der Formel (5) 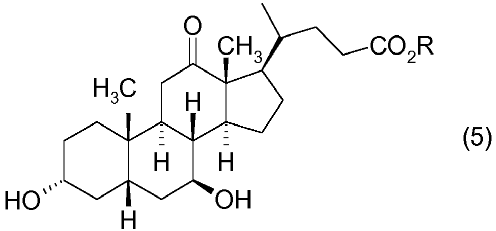 worin R die oben angegebenen Bedeutungen besitzt, oder die Gruppe -CO₂R durch die Säureamid-Gruppe -CONR¹R², wie oben definiert ersetzt ist, insbesondere in Gegenwart und unter Verbrauch von NADH und /oder NADPH reduziert und anschließend
c) 12-Keto-UDCS der Formel (5) chemisch zu UDCS reduziert; und
d) das Reaktionsprodukt gegebenenfalls weiter aufreinigt.

17. Verfahren nach Ausführungsform 16, wobei zumindest Schritt b) in Gegenwart eines rekombinanten Mikroorganismus nach einem der Ausführungsformen 7 bis 10 durchgeführt werden.

18. Verfahren nach Ausführungsform 16 oder 17, wobei Schritt b) mit gleichen oder verschiedenen Kofaktorregenerierungssystemen gekoppelt ist.

19. Verfahren zur Herstellung von UDCS der Formel (1) 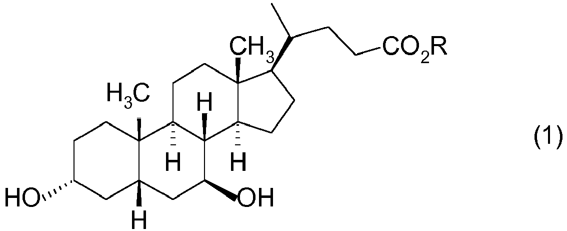 worin
R für Alkyl, H, ein Alkalimetallion oder N(R³)₄⁺steht, worin die Reste R³ gleich oder verschieden sind und für H oder Alkyl stehen oder die Gruppe -CO₂R durch die Säureamid-Gruppe -CONR¹R², wie oben definiert ersetzt ist
wobei man
a) gegebenenfalls eine CS der Formel (2) 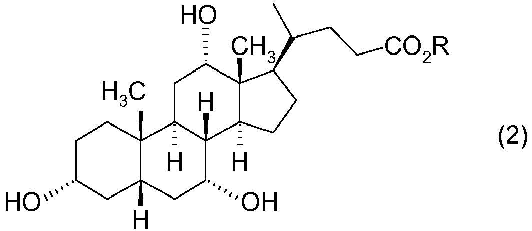 worin R die oben angegebenen Bedeutungen besitzt, oder die Gruppe -CO₂R durch die Säureamid-Gruppe -CONR¹R², wie oben definiert ersetzt ist, zur DHCS der Formel (3) 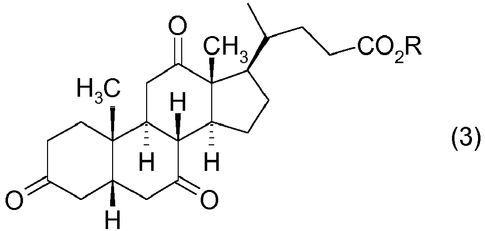 worin R die oben angegebenen Bedeutungen besitzt, oder die Gruppe -CO₂R durch die Säureamid-Gruppe -CONR¹R² wie oben definiert ersetzt ist chemisch oxidiert;
b) DHCS in Gegenwart wenigstens einer 7β-HSDH und in Gegenwart wenigstens einer 3α-HSDH-Mutante gemäß einer der Ausführungsformen 1 bis 3 (exprimiert von einer entsprechenden rekombinanten Mikroorganismus) zur korrespondierenden 12-Keto UDCS der Formel (5) 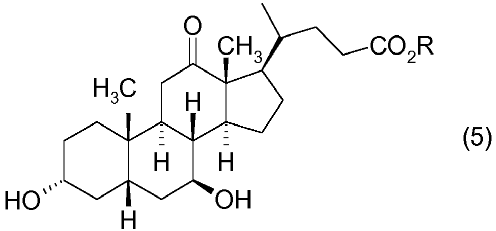 worin R die oben angegebenen Bedeutungen besitzt, oder die Gruppe -CO₂R durch die Säureamid-Gruppe -CONR¹R² wie oben definiert ersetzt ist, insbesondere in Gegenwart und unter Verbrauch von NADH und/oder NADPH, reduziert und anschließend
c) 12-Keto-UDCS der Formel (5) chemisch zu UDCS reduziert; und
d) das Reaktionsprodukt gegebenenfalls weiter aufreinigt;
   wobei die Umsetzungen des Schritts b) in Gegenwart eines rekombinanten Mikroorganismus nach einer der Ausführungsformen 7 bis 10 erfolgen, wie z.B. in Gegenwart ganzer Zellen eines oder mehrere verschiedener rekombinanter Mikroorganismen nach einer der Ausführungsformen 7 bis 10 erfolgen, wobei der/die Mikroorganismen die zur Umsetzung und Kofaktorregenerierung erforderlichen Enzyme in einer hierin näher beschriebenen Weise tragen.

Der Verfahrensschritt b) kann dabei unterschiedlich ausgestaltet sein. Entweder können beide Enzyme (7β-HSDH-Mutante und 3a-HSDH) gleichzeitig zugegen sein (z.B. Ein-Topf-Reaktion mit beiden isolierten Enzymen oder ein oder mehrere entsprechende rekombinante Mikroorganismen sind zugegen, der beide Enzyme exprimiert), oder die Teilreaktionen können in beliebiger Reihenfolge (erst die 7β-HSDH-Mutante-katalysierte Reduktion und dann die 3α-HSDH-katalysierte Reduktion; oder erst die 3α-HSDH -katalysierte Reduktion und dann die 7β-HSDH-Mutante-katalysierte Reduktion) ablaufen.

Weiterhin kann Schritt b) mit einem Kofaktorregenerierungssystem gekoppelt sein, bei welchem NADPH durch eine NADPH regenerierende GDH unter Verbrauch von Glucose regeneriert wird; oder mit einem Kofaktorregenerierungssystem gekoppelt ist, bei welchem verbrauchtes NADH durch eine NADH regenerierende GDH, ADH oder FDH regeneriert wird.

Ferner ist hierin ein Bioreaktor zur Durchführung eines Verfahrens nach einer der Ausführungsformen 11 bis 19 beschrieben, der insbesondere wenigstens eines der Enzyme 7β-HSDH, FDH, und/oder 3α-HSDH bzw. deren Mutanten; oder 7β-HSDH, GDH und/oder 3α-HSDH bzw. deren Mutanten enthält.

Die vorliegende Erfindung wird durch die Patentansprüche definiert und ist nicht auf die hierin beschriebenen konkreten Ausführungsformen beschränkt. Der Fachmann wird durch die Lehre der vorliegenden Erfindung vielmehr in die Lage versetzt, ohne unzumutbaren Aufwand weitere Ausgestaltungen der Erfindung bereitzustellen. So kann er beispielsweise auch weitere Enzymmutanten gezielt generieren und diese auf das gewünschte Eigenschaftsprofil (verbesserte Kofaktor-Abhängigkeit und /der Stabilität, verringerte Substratinhibition) screenen und optimieren; oder weitere geeignete Wildtypenzyme (7β- und 3α-HSDHs, FDHs, GDHs ADHs usw) isolieren und erfindungsgemäß verwenden. Weiterhin kann er beispielsweise je nach Eigenschaftsprofil (insbesondere Kofaktor-Abhängigkeit) der verwendeten HSDHs, wie insbesondere 7β-HSDH und 3α-HSDH oder Mutanten davon, geeignete zur Kofaktorregenerierung brauchbare Dehydrogenasen (GDH, FHD, ADH usw.) und Mutanten davon auswählen, und die ausgewählten Enzyme auf einen oder mehrere Expressionskonstrukte oder Vektoren verteilen und damit erforderlichenfalls einen oder mehrere rekombinante Mikroorganismen erzeugen, die dann ein optimiertes ganzzellbasiertes Herstellungsverfahren ermöglichen.

### Weitere Ausgestaltungen der Erfindung

### 1. Allgemeine Definitionen und verwendete Abkürzungen

Werden keine anderen Angaben gemacht, so bezeichnet der Begriff "7β-HSDH" ein Dehydrogenase-Enzym, welches wenigstens die stereospezifische und/oder regiospezifische Reduktion von DHCS oder 7,12-Diketo-3α-CS (7,12-Diketo-LCS) zu 3,12-Diketo-7β-CS oder 12-Keto-UDCS insbesondere unter stöchiometrischem Verbrauch von NADPH, und gegebenenfalls die entsprechende Umkehrreaktion katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen. Geeignete Nachweisverfahren sind z.B. im folgenden experimentellen Teil beschrieben oder aus der Literatur bekannt (z.B. Characterization of NADP-dependent 7 beta-hydroxysteroid dehydrogenases from Peptostreptococcus productus and Eubacterium aerofaciens. S Hirano and N Masuda. Appl Environ Microbiol. 1982). Enzyme dieser Aktivität sind unter der EC Nummer 1.1.1.201 klassifiziert. Geeignete Enzyme sind z.B. aus *Colinsellea aerofaciens* isolierbar.

Werden keine anderen Angaben gemacht, so bezeichnet der Begriff "3α-HSDH" ein Dehydrogenase-Enzym, welches wenigstens die stereospezifische und/oder regiospezifische Reduktion von 3,12-Diketo-7β-CS oder DHCS zu 12-Keto-UDCS oder 7,12-Diketo-3α-CS (7,12-Diketo-LCS), insbesondere unter stöchiometrischem Verbrauch von NADH und/oder NADPH, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus Comanomonas testosteroni (i.e. *Comamonas testosteroni*) (z.B. ATCC11996) erhältlich. Eine NADPH abhängige 3α-HSDH ist z.B. aus der Nagern bekannt und ebenfalls einsetzbar. (Cloning and sequencing of the cDNA for rat liver 3 alpha-hydroxysteroid/dihydrodiol dehydrogenase, J E Pawlowski, M Huizinga and T M Penning, May 15, 1991 The Journal of Biological Chemistry, 266, 8820-8825). Enzyme dieser Aktivität sind unter der EC Nummer 1.1.1.50 klassifiziert.

Werden keine anderen Angaben gemacht, so bezeichnet der Begriff "GDH" ein Dehydrogenase-Enzym, welches wenigstens die Oxidation von β-D-Glucose zu D-Glucono-1,5-Lacton unter stöchiometrischem Verbrauch von NAD⁺ und/oder NADP⁺ sowie ggf. die entsprechende Umkehrreaktion katalysiert. Geeignete Enzyme sind z.B. aus *Bacillus subtilis* oder *Bacillus megaterium* erhältlich. Enzyme dieser Aktivität sind unter der EC Nummer 1.1.1.47 klassifiziert.

Werden keine anderen Angaben gemacht, so bezeichnet der Begriff "FDH" ein Dehydrogenase-Enzym, welches wenigstens die Oxidation von Ameisensäure (oder korrespondierenden Formiat-Salzen) zu Kohlendioxid unter stöchiometrischem Verbrauch von NAD⁺ und/oder NADP⁺, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus *Candida boidinii, Pseudomonas* sp, oder *Mycobacterium vaccae* erhältlich. Enzyme dieser Aktivität sind unter der EC Nummer 1.2.1.2 klassifiziert.

Unter einer "Reinform" oder einem "reinen" oder "im wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie .z.B. der Biuret-Methode oder dem Proteinnachweis nach Lowry.et al.(vgl Beschreibung in R.K. Scopes, Protein Purification, Springer Verlag, New York, Heidelberg, Berlin (1982)).

Unter einem "Redoxäquivalent" versteht man eine als Elektronendonor bzw. Elektronenakzeptor brauchbare, niedermolekulare organische Verbindung, wie beispielsweise Nikotinamidderivate wie NAD⁺ und NADH⁺ bzw. deren reduzierte Formen NADH bzw. NADPH. "Redoxäquivalent" und "Kofaktor" werden im Kontext der vorliegenden Erfindung als Synonym verwendet. So kann ein "Kofaktor" im Sinne der Erfindung auch als "redoxfähiger Kofaktor", d.h. als Kofaktor, der in reduzierter und einer oxidierter Form vorliegen kann, umschrieben werden.

Unter einem "verbrauchten" Kofaktor versteht man diejenige reduzierte bzw. oxidierte Form des Kofaktors, die im Verlauf einer vorgegebene Reduktions- bzw. Oxidationsreaktion eines Substrats in die korrespondierende oxidierte bzw. reduzierte Form überführt wird. Durch Regenerierung wird die bei der Reaktion gebildete oxidierte bzw. reduzierte Kofaktor-Form wieder in die reduzierte bzw. oxidierte Ausgangsform zurück überführt, so dass diese für die Umsetzung des Substrats wieder zur Verfügung steht.

Unter einer "veränderten Kofaktor-Nutzung versteht man im Rahmen der vorliegenden Erfindung eine qualitative oder quantitative Veränderung im Vergleich zu einer Referenz. Insbesondere ist eine veränderte Kofaktor-Nutzung durch Vornahme von Aminosäuresequenzmutationen zu beobachten. Diese Veränderung ist dann im Vergleich zum nicht-mutierten Ausgangsenzym feststellbar. Dabei kann die Aktivität in Bezug auf einen bestimmten Kofaktor durch Vornahme einer Mutation erhöht oder erniedrigt werden oder vollständig unterbunden werden. Eine veränderte Kofaktor-Nutzung umfasst aber auch Änderungen dergestalt, dass anstelle einer Spezifität für einen einzelnen Kofaktor nunmehr wenigstens ein weiterer, vom ersten Kofaktor verschiedener, zweiter Kofaktor nutzbar ist (d.h. es liegt eine erweiterte Kofaktor-Nutzung vor) Umgekehrt kann aber auch eine ursprünglich vorhandene Befähigung zur Nutzung zweier verschiedener Kofaktoren so abgeändert werden, dass Spezifität nur für einen dieser Kofaktoren erhöht bzw. für einen dieser Kofaktoren verringert oder vollständig aufgehoben wird. So kann beispielsweise ein Enzym, das vom Kofaktor NAD (NADH) abhängig ist, aufgrund einer Veränderung der Kofaktor-Nutzung nunmehr sowohl von NAD (NADH) als auch vom Kofaktor NADP (NADPH) abhängig sein oder die ursprüngliche Abhängigkeit von NAD (NADH) kann vollständig in eine Abhängigkeit von NADP (NADPH) überführt werden und umgekehrt.

Die Begriffe "NAD⁺/NADH-Abhängigkeit" bzw. "NADP⁺/NADPH-Abhängigkeit" sind erfindungsgemäß, wenn nicht anders definiert, weit auszulegen. Diese Begriffe umfassen sowohl "spezifische" Abhängigkeiten, d.h. ausschließlich Abhängigkeit von NAD⁺/NADH bzw. NADP⁺/NADPH, als auch die Abhängigkeit der erfindungsgemäß verwendeten Enzyme von beiden Kofaktoren, d.h. Abhängigkeit von NAD⁺/NADH und NADP⁺/NADPH.

Entsprechendes gilt für die verwendeten Begriffe "NAD⁺/NADH-akzeptierend" bzw. "NADP⁺/NADPH-akzeptierend".

Die Begriffe "NAD⁺/NADH-regenerierend" bzw. "NADP⁺/NADPH-regenerierend" sind erfindungsgemäß, wenn nicht anders definiert, weit auszulegen. Diese Begriffe umfassen sowohl "spezifische" d.h. ausschließliche Befähigung verbrauchten Kofaktor NAD⁺/NADH bzw. NADP⁺/NADPH zu regenerieren, als auch die Befähigung beide Kofaktoren, d.h. NAD⁺/NADH und NADP⁺/NADPH, zu regenerieren.

"Proteinogene" Aminosäuren umfassen insbesondere (Einbuchstabencode): G, A, V, L, I, F, P, M, W, S, T, C, Y, N, Q, D, E, K, R und H.

Unter einer "Immobilisierung" versteht man erfindungsgemäß die kovalente oder nicht-kovalente Bindung eines erfindungsgemäß verwendeten Biokatalysators, wie z.B. einer 7ß-HSDH an einem festen, d.h. in dem umgebenden flüssigen Medium im Wesentlichen unlöslichen, Trägermaterial. Erfindungsgemäß können entsprechend auch ganze Zellen, wie die erfindungsgemäß verwendeten, rekombinanten Mikroorganismen, mit Hilfe derartiger Träger immobilisiert sein.

Eine "im Vergleich zum nicht-mutierten Enzym verringerte Substratinhibition" bedeutet, dass das die beim nicht-mutierten Enzym für ein bestimmtes Substrat beobachtete Substratinhibition nicht mehr zu beobachten ist, d.h. im Wesentlichen nicht mehr messbar ist, oder erst bei höherer Substratkonzentration einsetzt, d.h. der Kᵢ-Wert erhöht ist.

Erfindungsgemäße N- oder C-terminale Verlängerungen umfassen etwa 15 bis 25 proteinogene Aminosäurereste, welche, gegebenenfalls reversibel, z.B. über eine Protease -Schnittstelle, an einen terminalen Aminosäurerest angehängt sind. Bevorzugte Reste umfassen sogenannte an sich bekannte "His-Tags" mit z.B. 3, 6 oder 9 konsekutiven Histidinresten in der Verlängerungssequenz.

Unter einer "Cholsäure-Verbindung" versteht man erfindungsgemäß Verbindungen mit dem Kohenstoffgrundgerüst, insbesondere der Steroidstruktur der Cholsäure und dem Vorhandensein von Keto- und/oder Hydroxy- oder Acyloxy-Gruppen in der Ringposition 7 und gegebenenfalls den Ringpositionen 3 und/oder 12.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das NH₄⁺-Salz bzw. solche Ammoniumsalze umfasst, worin wenigstens ein Wasserstoffatom durch einen C₁-C₆-Alkylrest ersetzt sein kann. Typische Alkylreste sind insbesondere C₁-C₄-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon

"Alkylester" erfindungsgemäßer Verbindungen sind insbesondere Niedrigalkyl-Ester, wie z.B. C₁-C₆-Alkylester. Als nicht limitierende Beispiele sind zu nennen Methyl-, Ethyl-, n-oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäßer Säuren mit Ammoniak oder primären oder sekundären Monoaminen. Derartige Amine sind beispielsweise Mono- oder Di-C₁-C₆-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

Erfindungsgemäße "Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und C₁-C₆-Alkylgruppen, wie z.B. Benzoyl oder Toluoyl.

Die erfindungsgemäß eingesetzten bzw. hergestellten Hydroxysteroidverbindungen, wie z.B. Cholsäure, Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

In folgender Tabelle sind die Strukturformeln, deren chemischen Namen und die verwendeten Abkürzungen wesentlicher chemischer Verbindungen tabellarisch zusammengefasst:

| Formel | Abkürzung | Chemischer Name |
|---|---|---|
| 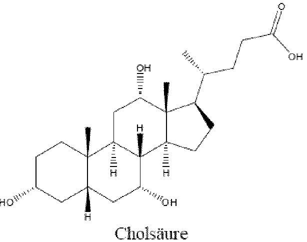 | CS | Cholsäure |
| 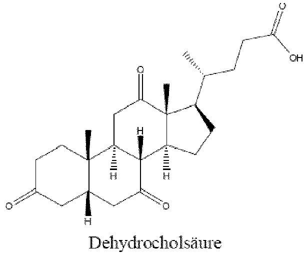 | DHCS | Dehydrocholsäure |
| 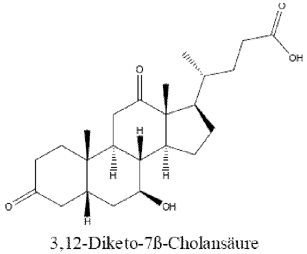 | 3,12-Diketo-7β-CS | 3,12-Diketo-7β-Cholansäure |
| 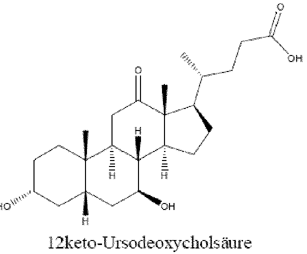 | 12Keto-UDCS | 12Keto-Ursodeoxycholsäure |
| 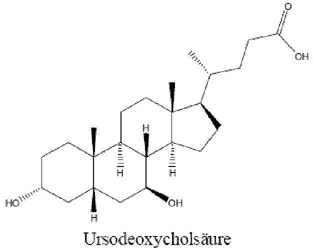 | UDCS | Ursodeoxycholsäure |
| 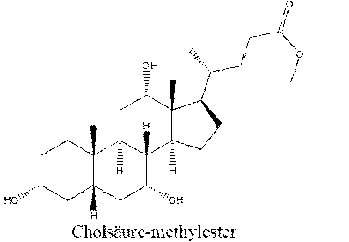 | CS-Methylester | Cholsäure-methylester |
| 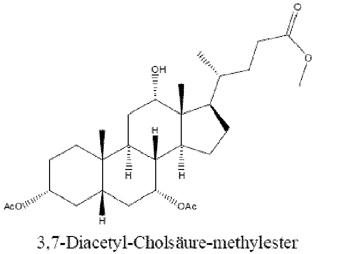 | 3,7-Diacetyl-CS-methylester | 3,7-Diacetyl-Cholsäure-methylester* |
| 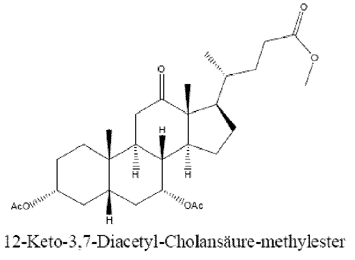 | 12-Keto-3,7-Diacetyl-CS-methylester | 12-Keto-3,7-Diacetyl-Cholansäure-methylester* |
| 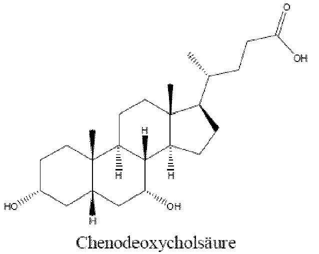 | CDCS | Chenodeoxycholsäure |
| 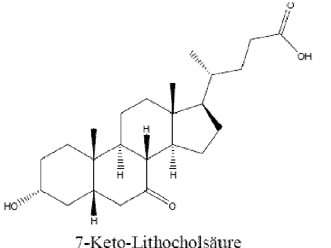 | 7-Keto-LCS | 7-Keto-Lithocholsäure |
| 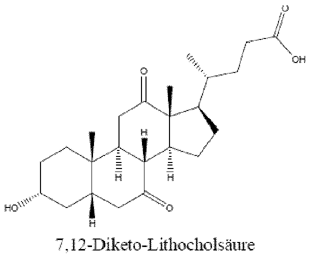 | 7,12-Diketo-LCS | 7,12-Diketo-Lithocholsäure |
| 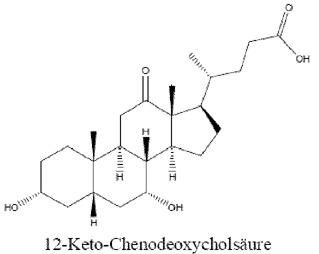 | 12-Keto-CDCS | 12-Keto-Chenodeoxycholsäure |

### 2. Proteine

Die vorliegende Offenbarung ist nicht auf die konkret offenbarten Proteine bzw. Enzyme mit 7β-HSDH-, FDH-, GDH- oder 3α-HSDH Aktivität bzw. deren Mutanten beschränkt, sondern erstreckt sich vielmehr auch auf funktionale Äquivalente davon.

"Funktionale Äquivalente" oder Analoga der konkret offenbarten Enzyme sind im Rahmen der vorliegenden Offenbarung davon verschiedene Polypeptide, welche weiterhin die gewünschte biologische Aktivität, wie z.B. 7β HSDH-Aktivität, besitzen.

So versteht man beispielsweise unter "funktionalen Äquivalenten" Enzyme, die im verwendeten Test auf 7β-HSDH-, FDH-, GDH- oder 3α-HSDH -Aktivität eine um mindestens 1%, wie z.B. mindestens 10% oder 20 %, wie z.B. mindestens 50 % oder 75% oder 90 % höhere oder niedrigere Aktivität eines Ausgangs-Enzyms, umfassend eine hierin definierte Aminosäuresequenz aufweisen.

Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C.

Die 7β-HSDH-Aktivität kann mit Hilfe verschiedener bekannter Tests nachgewiesen werden. Ohne darauf beschränkt zu sein, sei ein Test unter Verwendung eines Referenzsubstrates, wie z. B. CS oder DHCS, unter standardisierten Bedingungen wie im experimentellen Teil definiert, zu nennen.

Tests zur Bestimmung der FDH-, GDH-, 7β-HSDH- oder 3α-HSDH-Aktivität sind ebenfalls an sich bekannt.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere, wie z.B. 1, 2, 3, 4, 5, 6, 7, 8, 9, 10, 11, 12, 13, 14 oder 15, Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| **Ursprünglicher Rest** | **Beispiele der Substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxylgruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

"Funktionale Äquivalente" sind ferner Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Die Offenbarung umfasst weiterhin die Anwendung des 7β-HSDH-Wildtyps aus *Collinsella aerofaciens* ATCC 25986, wie er in der WO2011/064404der Anmelderin beschrieben ist, worauf hiermit ausdrücklich Bezug genommen wird.

Dieser aus *Collinsella aerofaciens* DSM 3979 erhältliche 7β-HSDH ist insbesondere durch wenigstens eine weitere der folgenden Eigenschaften, wie z.B. 2, 3, 4, 5, 6 oder 7 oder aller solcher Eigenschaften, gekennzeichnet:
a) Molekulargewicht (SDS-Gelelektrophorese): etwa 28-32 kDa, insbesondere etwa 29 bis 31 kDa oder etwa 30 kDa;
b) Molekulargewicht (Gelfiltration, unter nicht denaturierenden Bedingungen, wie insbesondere ohne SDS): etwa 53 bis 60 kDa, insbesondere etwa 55 bis 57 kDa , wie 56.1 kDa. Dies belegt die dimere Beschaffenheit der 7β-HSDH aus *Collinsella aerofaciens* DSM 3979;
c) Stereoselektiven Reduktion der 7-Carbonyl-Gruppe von 7-Keto-LCS in eine 7β-Hydroxy-Gruppe;
d) pH Optimum für die Oxidation von UDCS im Bereich von pH 8,5 bis 10,5, insbesondere 9 bis 10;
e) pH Optimum für die Reduktion von DHCS und 7-Keto-LCS im Bereich von pH 3,5 bis 6,5, insbesondere bei pH 4 bis 6;
f) wenigstens einen kinetischen Parameter aus folgender Tabelle für wenigstens eines der dort genannten Substrate/Kofaktoren; im Bereich von ±20%, insbesondere ±10%, ±5%, ±3% ±2% oder ±1% um den in der folgenden Tabelle jeweils konkret genannten Wert.

| | K_{M} (µM) | Vₘₐₓ (U/mg Protein)^{b)} | k_{cat} (1 µmol/(µmol×min)) |
|---|---|---|---|
| NADP⁺ | 5.32 | 30.58 | 944.95 |
| NADPH | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| NAD⁺ | -^{a)} | - | Spuren |
| NADH | - | - | Spuren |

| | | | |
|---|---|---|---|
| ^{a)} konnten aufgrund der sehr geringen Aktivität nicht bestimmt werden ^{b)} 1 U = 1 µmol/min | | | |

g) Phylogenetische Sequenzverwandtschaft der prokaryotischen 7β-HSDH aus *Collinsella aerofaciens* DSM 3979 mit der tierischen 11β-HSDH-Untergruppe, umfassend *Cavia porcellus, Homo sapiens* und *Mus musulus,* verwandt.

Beispielsweise zeigt diese 7β-HSDH folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f).

Eine derartige 7β-HSDH oder davon abgeleitetes funktionales Äquivalent ist zudem gekennzeichnet durch
a. die stereospezifische Reduktion eines 7-Ketosteroids zum korrespondierenden 7β-Hydroxysteroid, und/oder
b. die regiospezifische Hydroxylierung eines Ketosteroids umfassend eine Ketogruppe in 7-Position und wenigstens eine weitere Ketogruppe am Steroidgerüst zum korrespondierenden 7β-Hydroxysteroid, wie insbesondere von Dehydrocholsäure (DHCS) in 7-Position zur korrespondierenden 3,12-Diketo-7β-Cholansäure, katalysiert, und z.B. NADPH abhängig ist.

Eine derartige 7β-HSDH hat insbesondere eine Aminosäuresequenz gemäß SEQ ID NO:2 (Accession NO: ZP_01773061) oder eine davon abgeleiteten Sequenz mit einem Identitätsgrad von wenigstens 60%, wie z.B. wenigstens 65, 70, 75, 80, 85, oder 90, wie z.B. wenigstens 91, 92, 93, 94, 95, 96, 97, 98, 99 oder 99,5% zu dieser Sequenz; gegebenenfalls zusätzlich gekennzeichnet durch eine der folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f) gemäß obiger Definition.

### 3. Nukleinsäuren und Konstrukte

### 3.1 Nukleinsäuren

Gegenstand der Erfindung sind auch Nukleinsäuresequenzen, die für eine erfindungsgemäße 3α-HSDH kodieren. Ferner sind hierin Nukleinsäuren beschrieben, die für ein Enzym mit 7β-HSDH-, FDH-, GDH- und/oder 3α-HSDH Aktivität und deren Mutanten kodieren.

Es sind auch Nukleinsäuren mit einem bestimmten Identitätsgrad zu den hierin beschriebenen konkreten Sequenzen beschrieben.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

### Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

### Pairwise alignment parameter:

| | |
|---|---|
| FAST algorithm | on |
| K-tuplesize | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Dier hier offenbarten Nukleinsäuresequenzen umfassen einzel- und doppelsträngige DNA- und RNA-Sequenzen, wie z.B. cDNA und mRNA, kodierend für eines der obigen Polypeptide und deren funktionalen Äquivalente, und sind z.B. unter Verwendung künstlicher Nukleotidanaloga zugänglich.

Offenbart sind hierin sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Offenbarung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Offenbart sind hierin auch Derivate der konkret offenbarten oder ableitbaren Nukleinsäuresequenzen.

So können weitere erfindungsgemäße Nukleinsäuresequenzen z.B. von SEQ ID NO:1, 3 oder 10 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Offenbart sind ebenso durch konservative Nukleotidsubstitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO:1, 3 oder 10 sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, insbesondere der SEQ ID NO:1, 3 oder 10 beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO:1, 3 oder 10 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO:1, 3 oder 10 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Protein sind dem Fachmann seit langem geläufig, wie beispielsweise
- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcärel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die Ergebnisse liefern wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

### 3.2 Konstrukte

Gegenstand der Erfindung sind außerdem Expressionskonstrukte, enthaltend unter der genetischen Kontrolle regulativer Nukleinsäuresequenzen eine für wenigstens ein erfindungsgemäßes Polypeptid kodierende Nukleinsäuresequenz; sowie Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO:1, 3 oder 10 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, Ipp-, lac-, Ipp-lac-, lacl^{q-,} T7-, T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, Agt11 oder pBdCl, in Streptomyces plJ101, pIJ364, plJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHIac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 4. Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben. Einen Überblick über bakterielle Expressionssysteme für die heterologe Expression von Proteinen liefertz.B. auch Terpe, K. Appl. Microbiol. Biotechnol. (2006) 72: 211-222.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gramnegative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Comamonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit 7β-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 5. Herstellung der UDCS

### 1. Schritt: Chemische Umsetzung von CS zu DHCS

Die Hydroxygruppen von CS werden mit Chromsäure bzw. Chromaten in saurer Lösung (z.B. H₂SO₄) zu Carbonylgruppe in an sich bekannter Weise auf klassischchemischem Weg oxidiert. Dadurch entsteht DHCS.

### 2. Schritt: Enzymatische oder mikrobielle Umsetzung von DHCS zu 12-Keto-UDCS

In wässriger Lösung wird DHCS durch 3α-HSDH und 7β-HSDH bzw. Mutanten davon spezifisch zu 12-Keto-UDCS in Anwesenheit von NADPH bzw. NADH reduziert. Der Kofaktor NADPH bzw. NADH kann durch eine ADH bzw. FDH bzw. GDH bzw. Mutanten davon von Isopropanol bzw. Natium-formiat bzw. Glucose regeneriert werden. Die Reaktion läuft unter milder Bedingung. Beispielsweise kann die Reaktion bei pH = 6 bis 9, insbesondere etwa pH = 8 und bei etwa 10 bis 30, 15 bis 25 oder etwa 23°C durchgeführt werden. Im falle eines mikrobiellen Umsetzungsschrittes können rekombinante Mikroorganismen, welche die erforderliche(n) Enzymaktivität(en) exprimieren in Gegenwart des umzusetzenden Substrates (DHCS) anaerob oder aerob in geeigneten Flüssigmedien kultiviert werden. Geeignete Kultivierungsbedingungen sind dem Fachmann an sich bekannt. Sie umfassen Umsetzungen im pH bereich von beispielsweise 5 bis 10 oder 6 bis 9, bei Temperaturen im Bereich von 10 bis 60 oder 15 bis 45 oder 25 bis 40 oder 37 °C. Geeignete Medien umfassen z,B, die unten beschriebenen LB und TB Medien. Die Umsetzungsdauer kann dabei z,B, batchweise oder kontinuierlich oder in sonstigen üblichen Verfahrensvarianten erfolgen (wie oben beschrieben). Die Unsetzungsdauer kann dabei z.B. im Bereich von Minuten bis mehreren Stunden oder tagen Liegen, und z.B. 1h bis 48 h betragen. Gegebenenfalls kann, wenn Enzymaktivität nicht kontinuierlich exprimiert wird, diese durch Zugabe eines geeigneten Induktors, nach Erreichen einer Zielzelldichte , z,B, von etwa OD₆₀₀ = 0,5 bis 1,0, eingeleitet werden.

Weitere mögliche geeignete Abwandlungen des mikrobiellen Herstellungsverfahrens hinsichtlich Fahrweise der Fermentation, Zusätze zum Medium, Enzymimmobilisierung und Isolierung der Wertstoffe sind auch dem folgenden Abschnitt betreffend "Herstellung der Enzyme bzw. Mutanten" zu entnehmen

### 3. Schritt Chemische Umsetzung von 12-Keto-UDCS zu UDCS

Die 12-Carbonylgruppe von 12-Keto-UDCS wird mittels Wolff-Kishner-Reduktion in an sich bekannter Weise entfernt, dadurch entsteht UDCS aus 12-Keto-UDCS. In der Reaktion wird zuerst die Carbonylgruppe mit Hydrazin zum Hydrazon umgesetzt. Anschließend wird das Hydrazon in Gegenwart einer Base (z.B. KOH) auf 200 °C erhitzt, hierbei wird Stickstoff abgespaltet und UDCS entsteht.

### 6. Rekombinante Herstellung der Enzyme und Mutanten

Gegenstand der Erfindung sind weiterhin Verfahren zur rekombinanten Herstellung erfindungsgemäßer Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 7. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

### Experimenteller Teil:

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wie bei Sambrook et al. (1989) a.a.O. beschrieben durchgeführt werden.

### A. Allgemeine Angaben

### Materialien:

Die genomische DNA von *Collinsella aerofaciens* DSM 3979 (ATCC 25986, frühere Bezeichnung *Eubacterium aerofaciens*) wurde von der Deutschen Sammlung für Mikroorganismen und Zellkulturen (DSMZ) bezogen.

Die genomische DNA von *C*. *testosteroni* kann von der Deutschen Sammlung für Mikroorganismen und Zellkulturen (DSMZ) bezogen werden oder mit an sich bekannten Methoden aus einem *C*. *testosteroni-Stamm* isoliert werden.

UDCS und 7-Keto-LCS sind an sich bekannte und in der Literatur beschriebene Ausgangsverbindungen. Alle übrigen Chemikalien wurden von Sigma-Aldrich und Fluka (Deutschland) bezogen. Sämtliche Restriktionsendonukleasen, die T4 DNA Ligase, die *Taq* DNA Polymerase, die *Phusion* DNA Polymerase und Isopropyl-β-D-1-thiogalactopyranosid (IPTG) wurden von Fermentas (Deutschland) bezogen.

### Medien:

LB-Medium, enthaltend Trypton 10 g, Hefeextrakt 5 g, NaCl 10 g pro Liter Medium

TB-Medium, enthaltend Trypton 12 g, Hefeextrakt 24 g, 4 mL Glycerin, 10 % KPi.-Puffer (11,55 g KH₂PO₄, 62,7 g K₂HPO₄, ad 500 mL H₂O) pro Liter Medium

Minimalmedium, modifiziert nach Wilms et al., BIOTECHNOLOGY AND BIOENGI-NEERING, 2001 VOL. 73, NO. 2, 95-103

### Sequenzen:

In Figur 2 Aufgeführt sind die Aminosäure-Sequenzen des Wildtyp-Enzyms der 7β-HSDH und von Mutanten, allerdings alle jeweils mit einem C-terminalen Hexa-Histidin-Tag versehen. In dieser Art sind alle Enzyme isoliert und charakterisiert worden)

### Beispiel 1: Herstellung der

### Beispiel 1: Herstellung der rekombinanten 3α-HSDH

### A) Plasmidtransformation:

Zur Expression rekombinanter 3α-HSDH-Enzyme (Wildtyp- und Mutantenenzyme) wurde der kommerziell verfügbare Expressionsvektor pET28a(+) verwendet, in den das jeweilige 3α-HSDH-Gen über die Schnittstellen der Restriktionsenzyme Ncol/Eco31I und Xhol in üblicher Weise einkloniert wurde. Der Expressionsvektor pET28a(+) ermöglicht es, dass direkt bei der Klonierung des HSDH-Gens eine Gensequenz an das HSDH-Gen angehängt wird, die für eine Abfolge von 6 Histidin-Molekülen kodiert. Diese Sequenz (als Hexahistidin-Tag oder His-Tag bezeichnet) erscheint dann nach der Expression C-terminal am HSDH-Protein. Das Ursprungs- bzw. Wildtyp-HSDH-Gen stammt aus dem Bakterium *Comamonas testosteroni,* das Plasmid, das das jeweilige 3α-HSDH-Gen enthält, wird als pET28a(+)_3α-HSDH bezeichnet. Zur Transformation des pET28a(+)_3α-HSDH-Plasmids wurden 5µl Ligationsansatz mit 100 µl kompetenten *E*. *coli*-Zellen BL21(DE3)Δ7α-HSDH versetzt und die Transformation nach Hanahan (J. Mol. Biol. (1983), Vol. 166, pp. 557) durchgeführt, wobei dieselben Schritte mit dem Plasmid, das das Wildtyp-Gen und mit dem Plasmid, das mutierte HSDH-Gene enthält, durchgeführt wurden. Der *E. coli*-Stamm BL21(DE3)Δ7α-HSDH, der hier regelmäßig verwendet wurde, zeichnet sich dadurch aus, dass in diesem Stamm das Gen für die 7α-HSDH deletiert wurde. Die genaue Charakterisierung der für diese Arbeiten verwendeten *E*. *coli*-Stämme ist in Tabelle 1 zusammengefasst.

**Tabelle 1: Verwendete Escherichia coli Stämme**

| **Stamm** | **Genotyp** |
|---|---|
| *Escherichia coli* DH5α | F-endA1 glnV44 thi-1 recA1 relA1 gyrA96 deoRnupG Φ80dlacZΔM15 Δ(lacZYA-argF)U169, hsdR17(rK - mK+),λ- |
| *Escherichia coli* BL21 (DE3) Δ7α-HSDH (= *E. coli* mit deletierter7α-HSDH) | F-ompTgaldcmlonhsdSB(rB-mB-) λ(DE3 [lacl lacUV5-T7 gene 1 ind1 sam7 nin5]) hshA- KanR+ |

### B) Expression und Zellvermehrung:

Zur Expression wurde der das Expressionskonstrukt (pET28a(+)_3α-HSDH-Plasmid) enthaltende *E*. *coli-Stamm* in LB-Medium (Trypton 10 g, Hefeextrakt 5 g, NaCl 10 g pro Liter) für 20Std. bei 25°C vermehrt, wobei das Medium 50 µg/ml Kanamycin enthält. Die Zellen wurden durch Zentrifugation (5,000×g, 30 min, 4°C) geerntet.

### C) Rohextrakt-Gewinnung:

Das Pellet wurde im Aufschlusspuffer (10 mM Imidazol, 50 mM Natriumphosphat, 300 mM NaCl, pH 8) resuspendiert, wobei pro 1 g Zellen (Feuchtmasse) 4 ml Puffer zugesetzt wurden. Die Zellen wurden dann unter konstanter Kühlung durch einminütige Ultraschallbehandlung (30 W Leistung, 50% Arbeitsintervall und 1 min Pause) unter Verwendung eines Ultraschallgeräts Sonopuls HD2070 (Fa. Bandelin, Berlin, Deutschland) aufgeschlossen. Der Aufschluss wurde dreimal wiederholt. Die Zellsuspension wurde zentrifugiert (18,000×g, 30 min, 4°C), wobei der Überstand als zellfreier Rohextrakt bezeichnet wird.

### D) Reinigung des Enzyms:

Der His-Tag ermöglicht eine sehr einfache Reinigung des HSDH-Proteins, da diese Sequenz von speziellem Chromatographiematerial (NTA (Ni-Nitrilotriacetat)-modifiziertes Trägermaterial, beladen mit 2-wertigen Nickelionen; z.B. "His Pur Ni-NTA" (Fa. Nimagen B. V., Nijmegen,Niederlande) hoch-spezifisch gebunden wird. Dazu wird der zellfreie Rohextrakt auf dieses Material enthaltende Tropfsäule aufgetragen, die vorher mit dem Aufschlusspuffer (3 bis 5 Säulenvolumina) äquilibriert wurde. Schwach bindendes Protein wurde durch Waschen mit 3 bis 5 Säulenvolumina Waschpuffer (20 mMImidazol, 50 mM Natriumphosphat, 300 mM NaCl, pH 8) entfernt. Das His-Tag-3α-HSDH-Protein wurde mit Imidazol-haltigem Elutionspuffer (250 mM Imidazol, 50 mM Natriumphosphat, 300 mM NaCl, pH 8) eluiert. Das Verfahren wurde bei Raumtemperatur durchgeführt. Durch Umpufferung wurde das enthaltende Imidazol zu entfernen.

Die Proteinkonzentration wurde nach der von Bradford beschriebenen Methode bestimmt (100 µl Probe mit 900 µl Bradford Reagenz mischen, für mind. 15 min im Dunkeln inkubieren, dann bei 595 nm gegen eine mit Rinderserum-Albumin erstellte Eichkurve bestimmen). Für eine Analyse mittels SDS-PAGE (SDS-Polyacrylamid-Gelelektrophorese) wurde das Gel mit CoomassieBrilliant Blue angefärbt.

### E) Aktivitätsbestimmung:

Die Aktivität der 3α-HSDH und der Mutanten wurde mit einem photometrischen Assay bestimmt, wobei die Abnahme der Extinktion bei 340 nm über einen Zeitraum von 30 Sekunden gemessen wurde. Die Reaktionslösung enthielt in einem Gesamtvolumen von 1 ml: 874 µl 50 mM Kaliumphosphat (KPi) Puffer, pH 8.0; 100 µl 100 mM einer Dehydrocholsäure-(DHCA) Lösung (DHCA gelöst in 50 mMKPi, pH 8); 10 µl der Enzymlösung (gfls. verdünnt); 16 µl einer 12,5 mM NADH-Lösung (gelöst in dest. H₂O). Die Aktivität wird im Folgenden in Units (U) angegeben, wobei 1 U der Abnahme von 1 µmol NADPH / min entspricht.

### Beispiel 2: Herstellung von 3α-HSDH Mutanten in Aminosäure-Position 34 und deren Charakterisierung

### A) Primer:

Für die positionsgerichtete Mutagenese der 3α-HSDH wurden die in Tabelle 2 angegebenen Mutageneseprimer verwendet. Die Primer wurden auf Grundlage der 3α-HSDH-Gensequenz dahingehend ausgewählt, dass sie den gewünschten Aminosäurenaustausch bewirken.

Zur Erzeugung der Mutanten wurden folgende Primerpaare verwendet:

**Tabelle 2: Primer für die positionsgerichtete Mutagenese der 3α-HSDH an Position 34**

| ***Bezeichnung*** | ***Austausch*** | ***Primer*** | ***5' -*> *3' Sequenz*** |
|---|---|---|---|
| R34N_for | Asparagin | forward | GGCATCGATATAAACGATGCGGAAGTG |
| R34N_rev | | reverse | CACTTCCGCATCGTTTATATCGATGCC |
| R34L_for | Leucin | forward | TCGTAGGCATCGATATACTCGATGCGGAAGTGAT |
| R34L_rev | | reverse | ATCACTTCCGCATCGAGTATATCGATGCCTACGA |
| R34S_for | Serin | forward | ACCAGATCGTAGGCATCGATATAAGCGATGCGGAAG |
| R34S_rev | | reverse | CTTCCGCATCGCTTATATCGATGCCTACGATCTGGT |
| R34C_for | Cystein | forward | ACCAGATCGTAGGCATCGATATATGCGATGCGGAAG |
| R34C_rev | | reverse | CTTCCGCATCGCATATATCGATGCCTACGATCTGGT |

### B) QuikChange®-PCR:

Ein gezielter Austausch einer Aminosäure kann mit Hilfe der "QuikChange®-PCR"-Methode erreicht werden. Dazu wurde folgende PCR-Reaktion (Reaktionsansatz s. Tab 3) durchgeführt: Zuerst erfolgte ein 2 min langer initialer Denaturierungsschritt bei 95°C, danach wurden 20 Zyklen von Denaturierung (30 s bei 95°C), Primerhybridisierung (1 min bei 60 - 68°C) und Elongation (11 min bei 68°C) durchgeführt. Als letzter Schritt erfolgte eine finale Elongation von 10 min bei 68°C bevor die Polymerasekettenreaktion durch Kühlen auf 4°C beendet wurde. Als Templat wurde ein pET28a-Vektor mit dem Gen der 3α-HSDH (Wildtyp) verwendet.

**Tabelle 3: PCR-Ansatz für die Erzeugung der verschiedenen 3α-HSDH Varianten**

| **PCR-Reaktionsansatz** | |
|---|---|
| Puffer (10x) | 5,0 µl |
| dNTP-Mix (10 mM) | 1,5µl |
| Forward-Primer (10 mol/µl) | 2,5 µl |
| Revers-Primer (10 pmol/µl) | 2,5 µl |
| Template | 1,0 µl |
| Pfu Polymerase | 0,5 µl |
| DMSO | 2,0 µl |
| ddH₂O | 35,0µl |
| | 50,0 µl |

Um Aminosäuren in Proteinsequenzen gezielt austauschen zu können, wird die DNA-Sequenz des entsprechenden Gens positionsgerichtet mutiert. Hierzu werden zueinander komplementäre Primer genutzt, die die gewünschte Mutation in ihrer Sequenz tragen. Als Template dient N6-adeninmethylierte, doppelsträngige Plasmid-DNA, die das zu mutierende Gen tragen. N6-adeninmethylierte Plasmid-DNA werden aus einem dam+*E. coli*-Stamm wie zum Beispiel *E. coli* DH5α isoliert.

Die Polymerasekettenreaktion wird wie oben beschrieben durchgeführt. Dabei werden die Primer komplementär zum Template verlängert, wodurch Plasmide mit der gewünschten Mutation entstehen, die einen Strangbruch aufweisen. Anders als bei anderen PCR-Reaktionen steigt die DNA-Ausbeute hierbei nur linear, da neu gebildete DNA-Moleküle nicht als Template für die PCR-Reaktion dienen können.

Nach Abschluss der PCR-Reaktion wurde das PCR-Produkt mittels PCR-Purification-Kit (Analytik Jena AG, Jena, Deutschland) aufgereinigt und die parentale, N6-adeninmethylierte mit Hilfe des Restriktionsenzyms *dpn*I verdaut. Dieses Enzym hat die Besonderheit, dass es unspezifisch N6-adeninmethylierte DNA restringiert, jedoch nicht die neu gebildete, nichtmethylierte DNA. Die Restriktion erfolgte, indem 1 µl *dpn*I zum PCR-Reaktionsansatz hinzu gegeben und für mind. 2 h oder über Nacht bei 37°C inkubiert wurde. Zur Transformation von 100 µl chemisch-kompetenten DH5α-Zellen wurden 7,5 µl dieses Ansatzes eingesetzt.

### C) Aktivitätswerte der Enzymmutanten:

Die Aktivitätsmessungen (s. Beispiel 1) der Mutanten, die in Position 34 mutagenisiert worden sind, ergab die in der folgenden Tabelle 4 aufgeführten Werte.

**Tabelle 4: Aktivitäten der verschiedenen 3α-HSDH-Varianten, die an der Position 34 verändert wurden.**

| **Mutante** | **Volumetrische Aktivität [U/ml]** | **spezifische Aktivität [U/mg]** |
|---|---|---|
| 3α-HSDH (WT) | 38,0 | 2,1 |
| 3α-HSDH [R34N] | 58,0 | 3,0 |
| 3α-HSDH [R34C] | 38,5 | 2,8 |
| 3α-HSDH [R34S] | 38,7 | 2,3 |
| 3α-HSDH [R34L] | 20,1 | 1,5 |

Die beste Mutante ([R34N]) zeigt eine verbesserte Aktivität um ca. das 3-fache.

### Beispiel 3: Herstellung von 3α-HSDH Mutanten in Aminosäure-Position 68 und deren Charakterisierung

Im Folgenden sind Mutanten der 3α-HSDH beschrieben, bei denen Asparaginsäure in Position 68 gegen einige andere Aminosäuren ausgetauscht worden. Vorteilhafte Mutanten werden detailliert kinetisch charakterisiert, indem über Michaelis-Menten-Kinetiken die Parameter K_{M} und vₘₐₓ bestimmt werden. Um Mutanten mit dem Wildtyp-Enzym vergleichen zu können, wurde vorab das Wildtyp-Enzym ebenfalls gereinigt und kinetisch charakterisiert.

### A) Michaelis-Menten-Kinetik des Wildtyp-Enzyms

Um die Veränderungen der Mutante bezüglich der kinetischen Konstanten deutlich zu machen, ist im Folgenden das Wildtyp-Enzym gereinigt und charakterisiert worden.

In Figur 3 und Tabelle 5 sind die Daten für das aufgereinigte Enzym mit einem His-Tag aufgeführt.

**Tabelle 5: Kinetische Konstanten für das Wildtypenzym für das Substrat DHCA und den Cofaktor NADH. (x = keine Inhibierung)**

| ***Fraktion*** | ***Substrat*** | ***vₘₐₓ(U*/*mg)*** | ***K_{M} (µM)*** | ***K_{I} (mM)*** |
|---|---|---|---|---|
| 3α-HSDH | DHCA | 13,86 ± 0,8 | 134,3 ± 25,6 | 9,78 ± 1,4 |
| | NADH | 35,36 ± 8,1 | 427,2 ± 146,3 | x |

### A) Primer für die Konstruktion von Mutanten:

Für die positionsgerichtete Mutagenese der 3α-HSDH wurden die in Tabelle 6 aufgeführten Mutageneseprimer verwendet. Die Primer wurden auf Grundlage der 3α-HSDH-Gensequenz dahingehend ausgewählt, dass sie den gewünschten Aminosäurenaustausch bewirken.

Zur Erzeugung der Mutanten wurden folgende Primerpaare verwendet:

**Tabelle 6: Primer für die positionsgerichtete Mutagenese der 3α-HSDH an Position 68**

| ***Bezeichnung*** | ***Austausch*** | ***Primer*** | ***5' -> 3' Sequenz*** |
|---|---|---|---|
| L68S_for | Serin | forward | GGACGGCCTGGTGTCGTGCGCCGGCCTG |
| L68S_rev | | reverse | CAGGCCGGCGCACGACACCAGGCCGTCC |
| L68C_for | Cystein | forward | GGACGGCCTGGTGTGCTGCGCCGGCCTGG |
| L68C_rev | | reverse | CCAGGCCGGCGCAGCACACCAGGCCGTCC |
| L68K_for | Lysin | forward | GGACGGCCTGGTGAAGTGCGCCGGCCTG |
| L68K_rev | | reverse | CAGGCCGGCGCACTTCACCAGGCCGTCC |
| L68V_for | Valin | forward | ACGGCCTGGTGGTGTGCGCCGGC |
| L68V_rev | | reverse | GCCGGCGCACACCACCAGGCCGT |
| L68A_for | Alanin | forward | GACGGCCTGGTGGCGTGCGCCGGCCT |
| L68A_rev | | reverse | AGGCCGGCGCACGCCACCAGGCCGTC |

### B) QuikChange®-PCR:

Der gezielte Austausch von Asparaginsäure in Position 68 wurde wie in Beispiel 2B beschrieben mittels QuikChange®-PCR durchgeführt.

### C) Aktivitätswerte der Enzymmutanten:

Die photometrischen Aktivitätsmessungen (s. Beispiel 1) der Mutanten, die in Position 68 mutagenisiert worden sind, ergab die in der folgenden Tabelle 7 aufgeführten Werte.

**Tabelle 7: Aktivitäten der verschiedenen 3α-HSDH-Varianten, die an der Position 68 verändert wurden.**

| **Mutante** | **Volumetrische Aktivität [U/ml]** | **spezifische Aktivität [U/mg]** |
|---|---|---|
| 3α-HSDH (WT) | 44,9 | 2,1 |
| 3α-HSDH [L68A] | 157 | 6,4 |
| 3α-HSDH [I68C] | 57,5 | 3,4 |
| 3α-HSDH [L68K] | 40 | 3,1 |
| 3α-HSDH [L68S] | 39,6 | 3,1 |
| 3α-HSDH [L68V] | 60 | 2,3 |

### D) Michaelis-Menten-Kinetiken

Die beste Mutante (3α-HSDH [L68A]) wurde aufgereinigt (siehe 1 D) und ihre kinetischen Parameter vₘₐₓ und K_{M} bestimmt. In Figur 4 sind die Verlaufsdiagramme der Kinetiken abgebildet und in Tabelle 8 die daraus abgeleiteten kinetischen Konstanten aufgelistet.

**Tabelle 8:Kinetische Konstanten für die Mutante L68A für das Substrat DHCA und den Cofaktor NADH. (x = keine Inhibierung)**

| ***Enzym*** | ***Substrat*** | ***vₘₐₓ(U*/*mg)*** | ***K_{M} (mM)*** | ***K_{I} (mM)*** |
|---|---|---|---|---|
| L68A | DHCA | 27,3 ± 1,17 | 0,17 ± 0,02 | 4,55 ± 0,41 |
| | NADH | 34,77 ± 2,77 | 0,22 ± 0,03 | x |

Aus Tabelle 7 geht hervor, dass die maximale Geschwindigkeit gegenüber dem Wildtyp-Enzym (s. im Folgenden unter 3E) um ca. das 2-fache gesteigert werden konnte. Der K_{M}-Wert der Mutante liegt in einer ähnlichen Größenordnung wie der Wert für das Wildtyp-Enzym.

### Beispiel 4: Herstellung einer 3α-HSDH Mutante, die in mehreren Positionen Aminosäure-Austausche enthält und deren Charakterisierung

Neben den in den Beispielen 2 und 3 beschriebenen Einzelmutanten kann man Mutationen auch vorteilhaft kombinieren. Als Beispiel wurde eine Doppelmutante erzeugt, bei der gleichzeitig die Positionen 34 und 68 verändert wurden. Dabei wurde der beste Aminosäure-Austausch in Position 34 ([R34N]) kombiniert mit dem besten Austausch in Position 68 ([L68A]).

### A) Erzeugung der Doppelmutante und Aktivitätswerte

Die Methode zur Gewinnung der Doppelmutante und der Aktivitätstestrichtete sich dabei nach den Methoden, wie sie unter Beispiel 2B und 2C beschrieben sind. Die Aktivität der Doppelmutante im Rohextrakt im Vergleich zur Aktivität des Wildtyp-Enzyms ist in Tab. 9 zusammengestellt.

**Tabelle 9: Aktivität der 3α-HSDH Doppelmutante, die an der Position 34 und 68 verändert wurden.**

| **erzeugte Mutante** | **Volumetrische Aktivität [U/ml]** | **spezifische Aktivität [U/mg]** |
|---|---|---|
| 3α-HSDH (WT) | 44,9 | 2,1 |
| 3α-HSDH [R34N/L68A] | 139,7 | 4,5 |

### B) Expressionskontrolle durch SDS-Polyacrylamid-Gelelektrophoress (SDS-PAGE)

Eine SDS-PAGE wurde durchgeführt, um die Expressionsleistung der heterologen Expression bewerten zu können. In Figur 5 sind die SDS-Gele der Mutante 3α-HSDH [R34N/L68A] sowie [L68A] dargestellt. Die Größe der Mutanten beträgt ca. 27,4 kDa. Die Abbildung zeigt, dass die 3α-HSDHs im SDS-Gel nicht ihrer Größe entsprechend wandert. Sie läuft immer etwas unterhalb der 25 kDa Bande des Markers.

### C) Michaelis-Menten-Kinetiken

Die Mutante 3α-HSDH [R34N/L68A] wurde aufgereinigt und die kinetischen Parameter für das gereinigte Enzym bestimmt. In Figur 6 sind die Diagramme der Michaelis-Menten-Kinetiken dargestellt.

### D) Aktivitätswerte für die Doppelmutante

In Tab. 10 sind die Aktivitätswerte für die Doppelmutante zusamengestellt, wie sie sich aus den Michaelis-Menten-Kinetiken ergeben haben.

**Tabelle 10: Kinetische Konstanten für das aufgereinigte Enzym 3α-HSDH [R34N/L68A] für das Substrat DHCA und den Cofaktor NADH. Legende: x = keine Inhibierung**

| ***Enzym*** | ***Substrat*** | ***vₘₐₓ(U*/*mg)*** | ***K_{M} (mM)*** | ***K_{I} (mM)*** |
|---|---|---|---|---|
| R34N/L68A | DHCA | 16,45 ± 0,56 | 0,08 ± 0,01 | 17,6 ± 1,9 |
| | NADH | 44,38 ± 3,1 | 0,45 ± 0,07 | x |

Die maximale Aktivität der Doppelmutante konnte gegenüber dem Wildtyp um ca. 25% gesteigert werden. Deutlich verbessert hat sich der K_{M}-Wert: Für das Wildtyp-Enzym wurde ein Wert von 134 µM bestimmt, die Mutante zeigt einen K_{M}-Wert von 80 µM.

### E) Zusammenfassender Vergleich der Michaelis-Menten-Kinetiken von Mutanten ([L68A] und Doppelmutante [R34N/L68A]) mit dem Wildtyp

In der Figur 7 wurden die Verlaufsdiagramme der jeweiligen Mutanten mit dem Wildtyp verglichen. Für den Vergleich wurde hier das schon modifizierte Wildtyp Enzym mit His-Tag genommen. Hier zeigt sich, dass die Einzelmutante (L68A) zwar eine verbesserte Aktivität (mit DHCA) aufweist, die Substratüberschußinhibierung aber etwas ausgeprägter ist. Die Kombination der beiden Positionen hat ergeben, dass diese Mutante bei niedrigen SubstratKonzentrationen (DHCA) nicht ganz so aktiv wie der Wildtyp ist, bei höheren Substratkonzentrationen, also in einem Bereich, der für die Anwendung von Bedeutung ist, weist die Doppelmutante deutlich höhere Aktivitäten als das Wildtyp-Enzym auf.

### Beispiel 5: Einfluss des Hexahistidin (His)-Tags

Beim Vergleich der Aktivitäten des Wildtyp-Enzyms mit und ohne His-Tag fiel auf, dass sich die Aktivitäten deutlich voneinander unterschieden. Die Unterschiede wurden dann im Vergleich der Michaelis-Menten-Kinetiken deutlich (Figur 8). In Tab. 11 sind die K_{M}- und vₘₐₓ-Werte vergleichend gegenübergestellt. Der Vergleich wurde mit nicht-gereinigten Enzymen (Rohextrakte) aufgenommen, da die Reinigung des Enzyms ohne His-Tag sehr aufwändig ist. Es ist allerdings nicht zu erwarten, dass sich die relativen Kinetikverläufe und damit die Aussagen über den Einfluss des His-Tags mit gereinigten Enzymen ändern werden.

**Tabelle 11: Kinetische Konstanten der nicht getaggten als auch getaggten3α-HSDH für das Substract DHCA.Legende: * = Ablesung der kinetischen Konstanten aus der Grafik.**

| ***Fraktion*** | ***Substrat*** | ***vₘₐₓ(U*/*mg)*** | ***K_{M} (µM)*** | ***K_{I} (mM)*** |
|---|---|---|---|---|
| Rohextrakt (ohne His-Tag) | DHCA | 25* | 200* | 1* |
| Rohextrakt (mit His-Tag) | DHCA | 13,5 ± 0,72 | 183,4 ± 25,4 | 2,9 ± 0,3 |

Figur 8 und die Werte in Tab. 11 zeigen, dass das Enzym zumindest C-terminal ohne Einbuße an Aktivität mit einem His-Tag versehen werden kann. Charakteristisch für das Wildtyp-Enzym ist, dass es zwar eine hohe Affinität zum Substrat aufweist und bereits bei ca. 1 mM maximale Aktivität erreicht hat, allerdings fällt die Aktivität bei weiterer Erhöhung von DHCA drastisch ab (Figur 8, schwarze Punkte). Das nicht getaggte Enzym hat nur noch eine Restaktivität von ca. 10 - 20% ein. Die His-Tag-Variante des Enzyms zeigt dagegen ein deutlich anderes Verhalten. Die Aktivität steigt mit zunehmender DHCA-Konzentration im Bereich niedriger DHCA-Konzentrationen nicht ganz so stark an, d.h., die Affinität ist etwas erniedrigt, dafür zeigt dieses Enzym aber nicht mehr die starke SubstratüberschussInhibierung. Das getaggte Enzym hat somit gerade für die präparative Anwendung, die bei höheren DHCA-Konzentrationen stattfinden, bessere Eigenschaften als das nicht getaggte Enzym.

### Beispiel 6: Einfluss der Länge der Histidin(His)-Tags

Beim Vergleich der Aktivitäten des Wildtyp-Enzyms mit und ohne His-Tag fiel auf, dass sich die Aktivitäten deutlich voneinander unterschieden. Um den Einfluss des 6xHis-Tags zu verdeutlichen, wurde mittels Quik-Change®-PCR eine Variante der 3α-HSDH mit einem 3xHis-Tag und einem 9xHis-Tag am C-Terminus erzeugt. Nach der Expression (25 °C, 0,5 mM IPTG, 20 Stunden) wurde das Protein aufgereinigt und die Expression sowie die Aufreinigung über ein SDS-Gel überprüft. In der beiliegenden Figur 9 sind die SDS-Gele aufgeführt und in der folgenden Tabelle 12 sind die kinetischen Konstanten der verschiedenen Varianten dargestellt.

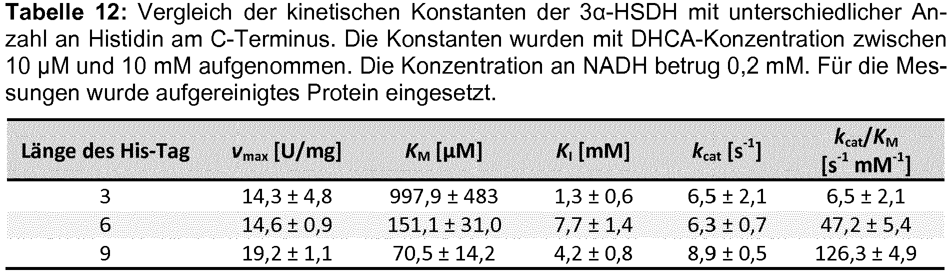

Die kinetischen Konstanten, die in Tabelle 12 dargestellt sind, bestätigen die Vermutung, dass der C-terminale Histidinrest die katalytische Eigenschaft beeinflusst. Aus der Tabelle geht sehr deutlich hervor, dass die Affinität steigt, je länger der His-Tag ist. Die Enzymvariante mit einem Anhang von 9 Histidinen zeigt einen 14-fach besseren *K*_{M}-Wert gegenüber der Variante mit 3 Histidinen am C-Terminus. Weiterhin wird anhand der katalytischen Effizienz (*k*_{cat}/*K*_{M}) deutlich, dass diese mit zunehmender Länge der Histidinkette ansteigt; die Variante mit 9 Histidinen hat eine 19-fache höhere Effizienz als die Variante mit nur 3 Histidinen.

### Beispiel 7: Herstellung und Charakterisierung weiterer Einfach- und Doppelmutanten der 3α-HSDH in den Positionen P185, T188 und ggf. zusätzlich L68

### A) Primer:

Für die positionsgerichtete Mutagenese der 3α-HSDH wurden die in Tabelle 13 angegebenen Mutageneseprimer verwendet. Die Primer wurden auf Grundlage der 3α-HSDH-Gensequenz dahingehend ausgewählt, dass sie den gewünschten Aminosäurenaustausch bewirken.

Zur Erzeugung der Mutanten wurden folgende Primerpaare verwendet:

**Tabelle 13: Primer für die positionsgerichtete Mutagenese der 3α-HSDH an Position 185 und 188**

| Position | | Primer Sequenz sense (forward) | | Primer Sequenz antisense (reverse) | Austausch zu |
|---|---|---|---|---|---|
| P185 | | | | | |
| | P185A-for | AACACCATCGCCGCCGGTGCAACCG | P185A-rev | CGGTTGCACCGGCGGCGATGGTGTT | Alanin |
| | P185G-for | GAACACCATCGCCGGCGGTGCAACCGAG | P185G-rev | CTCGGTTGCACCGCCGGCGATGGTGTTC | Glycin |
| | P185T-for | GAACACCATCGCCACCGGTGCAACCGA | P185T-rev | TCGGTTGCACCGGTGGCGATGGTGTTC | Threonin |
| | P185V-for | | P185V-rev | | Valin |
| | P185S-for | | P185S-rev | | Serin |
| T188 | | | | | |
| | T188A-for | CGCCCCCGGTGCAGCCGAGACTCC | T188A-rev | GGAGTCTCGGCTGCACCGGGGGCG | Alanin |
| | T188S-for | CCCCCGGTGCAAGCGAGACTCCC | T188S-rev | GGGAGTCTCGCTTGCACCGGGGG | Serin |
| | T188V-for | | T188V-rev | | Valin |
| | T188G-for | TCGCCCCCGGTGCAGGCGAGACTCCCTT | T188G-rev | | Gycin |
| | T188W-for | | T188W-rev | | Thryptophan |

Für Mutationen in Position L68 wurden die oben genannten Mutageneseprimer (s. Tabelle 6) verwendet.

### B) QuikChange®-PCR:

Der gezielte Austausch von Aminosäureresten erfolgte in Analogie zu Beispiel 2B. Hierzuwurde das Wildtypgen der 3α-HSDH in dem Plasmid pET28a(+)3α-HSDH(N-His), mit einem N-terminalen Hexahistidin-Tag, als auch mit einem C-terminalen Hexahistidin-Tag im Plasmid pET28a(+) 3α-HSDH (C-His) mittels QuikChange®-PCR so verändert, dass die in folgenden Tabelle 14 und 15 dargestellten Mutanten erzeugt wurden.

Nach Anzucht und Expression wurden die Zellen geerntet und die spezifischen Aktivitäten der zellfreien Rohextrakte bei Substratkonzentrationen von 10 mM DHCA bestimmt. Die Enzymvarianten, bei denen die Aktivitätsmessungen mit Rohextrakt-Enzym am vielversprechendsten waren, wurden mittels Ni-NTA Chromatographie aufgereinigt und ihre kinetischen Konstanten durch Messungen mit unterschiedlichen DHCA Konzentrationen, zwischen 10 µM und 20 mM bestimmt. Außerdem wurde bei sämtlichen Mutanten eine Expressionskontrolle mittels SDS PAGE durchgeführt.

Die, bezogen auf ihre Aktivität bei 10 mM, DHCA vielversprechendsten Kandidaten, im Vergleich zum entsprechenden Wildtyp-Enzym, waren:
T188A, T188S, T188G, P185A, L68A, T188S/L68A, T188A/L68A, P185A/T188A mit N-terminalem und T188A, L68A mit C-terminalem Hexahistidin-Tag

Im Folgenden sind die hierbei erhaltenen Ergebnisse aufgeführt.

### [T188A] N-terminaler Hexahistidin-Tag

Es zeigt sich, dass nach Erreichen der höchsten spez. Aktivität bei 0,5 mM DHCA die Aktivität bereits wieder sinkt. Das Enzym besitzt wie der Wildtyp eine ausgeprägte Substratüberschussinhibierung. Bei ca. 4,5 mM DHCA besitzt das Protein noch etwa 50 % Restaktivität und bei 20 mM etwa 20 %. Es stellt sich ein asymptotischer Verlauf ein.

### [T188S] N-terminaler Hexahistidin-Tag

Es zeigt sich eine Substratüberschussinhibierung nach einer maximalen spezifischen Aktivität bei einer Substratkonzentration von 0,5 mM. Bei einer Substratkonzentration von 20 mM DHCA besitzt das Enzym hier noch eine Restaktivität von über 20 %.

### [T188G] N-terminaler Hexahistidin-Tag

Hier zeigt sich keine ausgeprägte Substratüberschussinhibierung. Allerdings sind die Aktivitäten mit einer maximalen Aktivität von 25 U/mg gegenüber den anderen Enzymvarianten eher gering (etwa 10%). Bei hohen Substratkonzentrationen (>5 mM) ist die Aktivität gegenüber den bisher beschriebenen anderen Varianten in etwa gleichwertig. Anhand der Verlaufsdiagramme wurden die kinetischen Parameter bestimmt.

### [P185A] N-terminaler Hexahistidin-Tag

Es zeigt sich auch hier nach Erreichen der maximalen spezifischen Aktivität bei einer Substratkonzentration DHCA von 0,25 mM eine deutliche Substratüberschussinhibierung. Bei einer Substratkonzentration von 10 mM beträgt die Restaktivität noch 5 %.

### [L68A] N-terminaler Hexahistidin Tag

Neben den oben beschriebenen Positionen T188 und P185, die von [Hwang et al.,2013] identifiziert worden sind, wurde vergleichend die 3α-HSDH L68A erstellt. Die Position 68 befindet sich im Gegensatz zu den beiden anderen im N-terminalen Bereich des Proteins. Alle drei Positionen haben gemeinsam, dass diese Aminosäuren eine Bindung mit dem Coenzym eingehen, deshalb wurde auch diese Mutante mit einem N-terminalen His-Tag erstellt und in die biochemischen Charakterisierung mit einbezogen. Hierfür wurde das Plasmid pET28a(+) 3α-HSDH (N-His) mittels QuickChange®-PCR so verändert, dass die gewünschte Mutante erzeugt wurde. Nach Anzucht und Expression wurden die Proteine mittels Ni-NTA-Chromatografie aufgereinigt, die spezifischen Aktivitäten bestimmt und ihre kinetischen Konstanten ermittelt. Auch hierbei wurde die Substratkonzentration von DHCA zwischen 10 µM und 10 mM variiert, während die Konzentration von NADH konstant 0,2 mM betrug.

Es zeigt sich eine deutliche Substratüberschussinhibierung nach Erreichen der maximalen spezifischen Aktivität bei einer Substratkonzentration von 0,1 mM DHCA. Bei einer Substratkonzentration von 1 mM DHCA beträgt die Restaktivität hier ca. 15 %, bei 10 mM beträgt die Restaktivität ca. 8 %.

Zur Herstellung der folgenden Doppelmutanten wurde jeweils eine schon vorhandene Mutante mittels Quick Change® PCR nochmals, wie oben beschrieben, modifiziert, exprimiert, aufgereinigt und deren Parameter bestimmt.

### [L68A/T188A] N-terminaler Hexahistidin Tag

Es zeigt sich eine geringer ausgeprägte Substratüberschussinhibierung nach Erreichen der maximalen spezifischen Aktivität bei einer Substratkonzentration von 0,5 mM DHCA. Bei einer Substratkonzentration von 1 mM beträgt die Restaktivität noch 85 %, bei 10 mM immer noch über 20 %. Dies ist vergleichsweise mehr als bei den beiden Einzelmutanten.

### [L68A/T188S] N-terminaler Hexahistidin-Tag

Es zeigt sich, dass es nach der maximalen Aktivität bei 0,5 mM zu einem Abfall der Aktivitäten kommt. Dies ist auf eine starke Substratüberschussinhibierung zurückzuführen. Der Verlauf der Kurve gleicht denen der entsprechenden Einzelmutanten. Bei einer Substratkonzentration von 10 mM DHCA zeigt sich immer noch eine Restaktivität von etwa 20%.

### [P185A/T188A] N-terminaler Hexahistidin-Tag

Es zeigt sich eine Substratüberschussinhibierung nach Erreichen der maximalen spezifischen Aktivität bei einer Substratkonzentration DHCA von 0,5 mM. Bei Substratkonzentrationen von 10 mM DHCA beträgt die Restaktivität des Enzyms noch ca. 30 %. Im gesamten sind der Kurvenverlauf und die Substratinhibierung niedriger und geringer ausgeprägt als bei den entsprechenden Einzelmutanten, aber die Aktivitäten sind deutlich geringer.

### C) Zusammenfassung der Aktivitätswerte der in diesem Abschnitt hergestellten Enzymmutanten:

Die photometrischen Aktivitätsmessungen (analog Beispiel 1) der Mutanten, sowie deren kinetischen Parameter sind in den folgenden Tabellen 14 und 15 zusammengefasst.

**Tabelle 14: Gesamtergebnisse der ermittelten kinetischen Parameter der Wildtyp-Enzyme und Enzymvarianten für das Substrat DHCA. Es wurden die apparenten Werte aufgelistet.**

| **Mutante** | **Tag** | ***v*ₘₐₓ [U/mg]** | ***K*_{M} [µM]** | ***k*_{cat} [s-¹]** | ***k*_{cat}/*K*_{M} [s^{-1∗}mM⁻¹]** |
|---|---|---|---|---|---|
| **Wildtyp** | - | 134 ±3,8* | x | 62,5 | x |
| **Wildtyp** | N | 98,7 ± 4,7* | x | 47,1 | x |
| **Wildtyp** | C | 13,86 ± 0,8 | 134,3 ± 25,7 | 6,6 | 49,2 |
| **T188A** | N | 274,0± 11,6* | 94,5 ± 11* | 130,3 | 1371,3 |
| **T188S** | N | 332,5 ± 59,9* | 312,6 ± 112* | 158,2 | 510,6 |
| **T188G** | N | 20,3 ± 0,8* | 1458 ± 182,6* | 9,6 | 6,5 |
| **P185A** | N | 102,8 ± 5,7* | 71,0 ± 11* | 48,5 | 683,1 |
| **L68A** | N | 290,0 ± 45,4* | 75,4 ±22,6* | 138,0 | 1840,5 |
| **T188A** | C | 12,4 ± 12,9* | 2568,3 ± 2,5* | 31,7 | 2,4 |
| **L68A/T188A** | C | 6,2 ± 0,2* | 350,0 ± 25* | 2,9 | 8,4 |
| **L68A** | C | 13,9 ± 6,2* | 218,6 ± 38,8* | 18,1 | 177,4 |
| **P185A/T188A** | N | 36,9 ± 2,6* | 66,9 ± 15* | 17,6 | 262,3 |
| **L68A/T188A** | N | 233,5 ± 12,5* | 164,4 ± 20,4* | 111,1 | 677,4 |
| **L68A/T188S** | N | 193,2 ± 11,4* | 169,6 ± 22,6* | 91,8 | 543,0 |

| | | | | | |
|---|---|---|---|---|---|
| * Apparente Werte X = Wert nicht ermittelbar | | | | | |

**Tabelle 15:: Gesamtergebnisse der ermittelten kinetischen Parameter der Wildtypen und Enzymvarianten für das Substrat NADH. Es wurden die apparenten Werte aufgelistet.**

| **Mutante** | **Tag** | ***v*ₘₐₓ [U/mg]** | ***K*_{M} [µM]** | ***k*_{cat} [s-¹]** | ***k*_{cat}/*K*_{M} [s**^{**-1** ∗} **mM⁻¹]** |
|---|---|---|---|---|---|
| **Wildtyp** | N | 109,0 ± 4,1 | 59,2 ± 6,9 | 47,1 | 795,9 |
| **Wildtyp** | OHNE | 186,0 ± 6,9 | 46,9 ± 5,9 | 88,5 | 1884,7 |
| **Wildtyp** | C | 35,4 ± 8,1 | 427,2 ± 146,3 | 6,6 | 15,4 |
| **L68A** | N | 138,0 ± 4,4 | 17,1 ± 3,7 | 110,3 | 6461,1 |
| **T188A** | N | 236,7 ± 15,9 | 111,5 ± 18,2 | 130,3 | 1168,3 |
| **T188S** | N | 147,2 ± 17,9 | 87,6 ±29,7 | 96,1 | 1097,0 |
| **T188A/P185A** | N | 85,9 ± 19,5 | 333,9 ± 137,5 | 17,6 | 52,6 |
| **L68A/T188A** | N | 228,9 ± 4,7 | 67,5 ± 4,7 | 111,1 | 1645,1 |
| **L68A/T188S** | N | 188,3 ± 2,7 | 47,4 ± 2,6 | 91,8 | 1936,9 |

Zusammenfassend ist festzustellen, dass die erzeugten Mutanten im Vergleich zum korrespondierenden Wildtyp-Enzym eine Verbesserung in ihrer katalytischen Aktivität und/oder Affinität und Effizienz hinsichtlich der Substrate DHCA und /oder NADH aufweisen.

Insbesondere sind zu nennen die N-terminalem His-Tag Mutanten [T188A], [T188S], [L68A], [P185A], [L68A/T188S], [L68A/T188A], sowohl für das Substrat DHCA, als auch für das Substrat NADH.

Insbesondere sind zu nennen die C-terminalen His-Tag Mutanten [T188A] und [L68A].

Hierin Verwendete Sequenzen

| SEQ ID NO: | Beschreibung | Typ |
|---|---|---|
| 1 | 7β-HSDH C. aerofaciens | NS |
| 2 | 7β-HSDH C. aerofaciens | AS |
| 3 | 3α-HSDH C. testosteroni | NS |
| 4 | 3α-HSDH C. testosteroni | AS |
| 5 | 3α-HSDH C. testosteroni Wildtyp + His-Tag | AS |
| 6 | 3α-HSDH [R34N]; | AS |
| 7 | 3α-HSDH [L68A]; | AS |
| 8 | 3α-HSDH [R34N/L68A]; | AS |
| 9 | FDH Wildtyp, M. vaccae | AS |
| 10 | GDH, B. subtilis | NS |
| 11 | GDH B. subtilis | AS |
| 12 | Primer | NS |
| 13 | Primer | NS |
| 14 | Primer | NS |
| 15 | Primer | NS |
| 16 | Primer | NS |
| 17 | Primer | NS |
| 18 | Primer | NS |
| 19 | Primer | NS |
| 20 | Primer | NS |
| 21 | Primer | NS |
| 22 | Primer | NS |
| 23 | Primer | NS |
| 24 | Primer | NS |
| 25 | Primer | NS |
| 26 | Primer | NS |
| 27 | Primer | NS |
| 28 | Primer | NS |
| 29 | Primer | NS |
| 30 | Primer | NS |
| 31 | Primer | NS |
| 32 | Primer | NS |
| 33 | Primer | NS |
| 34 | Primer | NS |
| 35 | Primer | NS |
| 36 | Primer | NS |
| 37 | Primer | NS |
| 38 | Primer | NS |
| 39 | Primer | NS |
| 40 | Primer | NS |
| 41 | Primer | NS |
| 42 | Primer | NS |
| 43 | Primer | NS |
| 44 | Primer | NS |
| 45 | Primer | NS |
| 46 | Primer | NS |
| 47 | Primer | NS |
| 48 | Primer | NS |
| 49 | Primer | NS |

| | | |
|---|---|---|
| AS = Aminosäuresequenz NS = Nukleinsäuresequenz | | |

Auf die Offenbarung der hierin erwähnten Druckschriften wird ausdrücklich Bezug genommen.

## Patentansprüche

1. 3a-Hydroxysteroiddehydrogenase (3a-HSDH), welche wenigstens die stereospezifische enzymatische Reduktion eines 3-Ketosteroids zum korrespondierenden 3-Hydroxysteroid, insbesondere unter stöchiometrischem Verbrauch von NADH, katalysiert, wobei das Enzym wenigstens 80% Sequenzidentität zu SEQ ID NO:4 besitzt; und ausgewählt ist unter:
a) den Einfachmutanten
R34X₁ und
L68X₂ und
b) den Doppelmutanten
R34X₁/L68X₂ worin
X₁ für N, C, S oder L steht und
X₂ für A, C, K, S oder V steht;
c) C-terminal um einen Rest umfassend 9 konsekutive Histidin-Reste verlängerten Additionsmutanten;
und
d) Mutanten gemäß a) und b), jeweils zusätzlich C-terminal um einen Rest umfassend 1 bis 10 Histidin-Reste verlängert und/oder N-terminal um einen Rest umfassend 1 bis 10 Histidin-Reste verlängert;
wobei diese 3α-HSDH im Vergleich zur 3α-HSDH mit SEQ ID NO:4 folgendes Eigenschaftsprofil zeigt:
i) eine erhöhte spezifische Aktivität (vₘₐₓ [U/mg]) für Dehydrocholsäure (DHCA) bei der enzymatischen Reduktion von DHCA mit NADH als Cofaktor,
ii) eine verringerte Substratinhibition durch DHCA,
iii) eine erhöhte spezifische Aktivität (vₘₐₓ [U/mg]) für NADH bei der enzymatischen Reduktion von DHCA mit NADH als Cofaktor,
iv) wobei diese Eigenschaften i) bis iii) einzeln oder in beliebiger Kombination vorliegen können.

2. Nukleotidsequenz, kodierend für eine 3α-HSDH nach Anspruch 1.

3. Expressionskassette, umfassend unter der Kontrolle wenigstens einer regulativer Sequenz wenigstens eine Nukleotidsequenz nach Anspruch 2.

4. Expressionsvektor, umfassend wenigstens eine Expressionskassette nach Anspruch 3.

5. Rekombinanter Mikroorganismus, der wenigstens eine Nukleotidsequenz gemäß Anspruch 2 oder wenigstens eine Expressionskassette nach Anspruch 3 oder wenigstens einen Expressionsvektor nach Anspruch 4 trägt.

6. Rekombinanter Mikroorganismus nach Anspruch 5, der zusätzlich gegebenenfalls die kodierende Sequenz für wenigstens ein weiteres Enzym, ausgewählt unter Hydroxysteroiddehydrogenasen (HSDH) und zur Cofaktorregenerierung geeigneten Dehydrogenasen, trägt.

7. Rekombinanter Mikroorganismus nach Anspruch 6, wobei
die weitere HSDH ausgewählt ist unter 7β-HSDHs (NADPH- und/oder NADH-abhängig); und die Dehydrogenase ausgewählt ist unter NADPH-regenerierenden Enzymen, wie NADPH Dehydrogenasen, NADPH-regenerierenden Alkoholdehydrogenasen (ADH), NADPH-regenerierenden Formiatdehydrogenasen (FDH), NADPH-regenerierenden Glucosedehydrogenasen (GDH), NADPH-regenerierenden Glucose-6-Phosphat-Dehydrogenasen (G-6-PDH) sowie NADPH-regenerierenden Phosphitdehydrogenasen (PtDH), oder NADH-regenerierenden Enzymen, wie NADH Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH).

8. Rekombinanter Mikroorganismus nach einem der Ansprüche 5 bis 7, welcher ein 7α-HSDH knock-out Stamm ist.

9. Verfahren zur enzymatischen oder mikrobiellen Synthese von 3a-Hydroxysteroiden, wobei man das korrespondierende 3-Ketosteroid in Gegenwart einer 3α-HSDH gemäß der Definition in Anspruch 1 oder in Gegenwart eines diese 3α-HSDH exprimierenden rekombinanten Mikroorganismus nach einem der Ansprüche 5 bis 8 reduziert, und gegebenenfalls wenigstens ein gebildetes Reduktionsprodukt aus dem Reaktionsansatz isoliert.

10. Verfahren nach Anspruch 9, wobei das 3-Ketosteroid ausgewählt ist unter Dehydrocholsäure (DHCA),
und den Derivaten davon, wie insbesondere einem Salz, Amid oder Alkylester der Säure.

11. Verfahren nach Anspruch 9 oder 10, wobei die Reduktion in Gegenwart und insbesondere unter Verbrauch von NADPH und/oder NADH erfolgt.

12. Verfahren nach Anspruch 11, wobei verbrauchtes NADPH durch Kopplung mit einem NADPH-regenerierenden Enzym regeneriert wird, wobei dieses insbesondere ausgewählt ist unter NADPH Dehydrogenasen, NADPH-regenerierenden Alkoholdehydrogenasen (ADH), NADPH-regenerierenden Formiatdehydrogenasen (FDH) und NADPH-regenerierenden Glucosedehydrogenasen (GDH), wobei das NADPH-regenerierende Enzym gegebenenfalls von einem rekombinanten Mikroorganismus exprimiert wird; und/oder
wobei verbrauchtes NADH durch Kopplung mit einem NADH-regenerierenden Enzym regeneriert wird, wobei dieses insbesondere ausgewählt ist unter NADH-Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH), wobei das NADH-regenerierende Enzym ggf. in einem rekombinanten Mikroorganismus exprimiert wird.

13. Verfahren nach Anspruch 12, wobei das NADPH-regenerierende Enzym ausgewählt ist unter
a) FDHs, einschließlich Mutanten einer NAD⁺-abhängigen FDH, welche wenigstens die enzymatische Oxidation von Ameisensäure zu CO₂ katalysiert, wobei die Mutante im Vergleich zum nicht-mutierten Enzym zusätzlich NADP⁺ als Cofaktor akzeptiert; und
b) GDHs.

14. Verfahren zur Herstellung von Ursodesoxycholsäure (UDCS) der Formel (1) 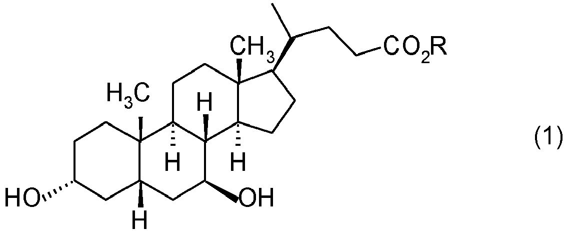 worin
R für Alkyl, H, ein Alkalimetallion oder N(R³)₄⁺steht, worin die Reste R³ gleich oder verschieden sind und für H oder Alkyl stehen, oder die Gruppe -CO₂R durch die Säureamid-Gruppe -CONR¹R², ersetzt ist, worin R¹ und R² unabhängig voneinander für einen Alkylrest stehen;
wobei man
a) gegebenenfalls eine Cholsäure (CS) der Formel (2) 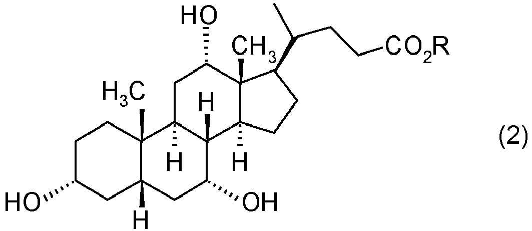 worin R die oben angegebenen Bedeutungen besitzt oder die Gruppe -CO₂R durch die Säureamid-Gruppe -CONR¹R², wie oben definiert ersetzt ist,
zur Dehydrocholsäure (DHCS) der Formel (3) 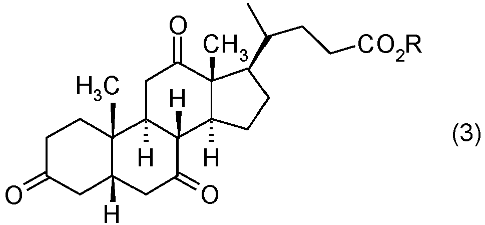 worin R die oben angegebenen Bedeutungen besitzt, oder die Gruppe -CO₂R durch die Säureamid-Gruppe -CONR¹R², wie oben definiert ersetzt ist, chemisch oxidiert;
b) DHCS in Gegenwart wenigstens einer 3a-HSDH-Mutante gemäß der Definition in Anspruch 1 und in Gegenwart wenigstens einer 7β-HSDH zur korrespondierenden 12-Ketoursodesoxychiolsäure (12-Keto UDCS) der Formel (5) 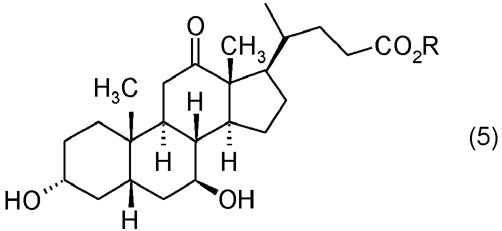 worin R die oben angegebenen Bedeutungen besitzt, oder die Gruppe -CO₂R durch die Säureamid-Gruppe -CONR¹R², wie oben definiert ersetzt ist, insbesondere in Gegenwart und unter Verbrauch von NADH und /oder NADPH reduziert und anschließend
c) 12-Keto-UDCS der Formel (5) chemisch zu UDCS reduziert; und
d) das Reaktionsprodukt gegebenenfalls weiter aufreinigt.

15. Verfahren zur Herstellung von Ursodesoxycholsäure (UDCS) der Formel (1) 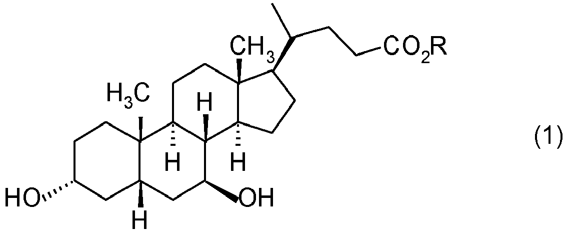 worin
R für Alkyl, H, ein Alkalimetallion oder N(R³)₄⁺steht, worin die Reste R³ gleich oder verschieden sind und für H oder Alkyl stehen, oder die Gruppe -CO₂R durch die Säureamid-Gruppe -CONR¹R², ersetzt ist, worin R¹ und R² unabhängig voneinander für einen Alkylrest stehen;
wobei man
a) gegebenenfalls eine Cholsäure (CS) der Formel (2) 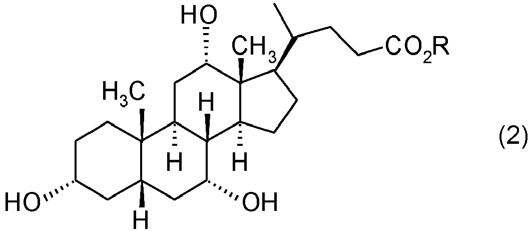 worin R die oben angegebenen Bedeutungen besitzt oder die Gruppe -CO₂R durch die Säureamid-Gruppe -CONR¹R², wie oben definiert, ersetzt ist,
zur Dehydrocholsäure (DHCS) der Formel (3) 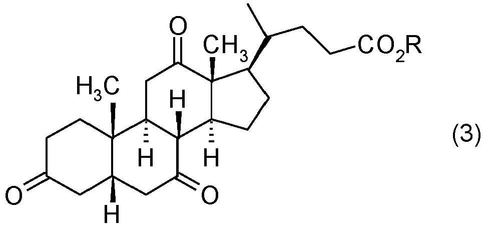 worin R die oben angegebenen Bedeutungen besitzt, oder die Gruppe -CO₂R durch die Säureamid-Gruppe -CONR¹R², wie oben definiert ersetzt ist, chemisch oxidiert;
b) DHCS in Gegenwart wenigstens einer 3a-HSDH-Mutante, ausgewählt unter
(a) den Einfachmutanten
R34X₁ und
L68X₂ und
(b) den Doppelmutanten
R34X₁/L68X₂
worin
X₁ für N, C, S oder L steht und
X₂ für A, C, K, S oder V steht;
(c) C-terminal um einen Rest umfassend 9 konsekutive Histidin-Reste verlängerten Additionsmutanten;
und
(d) Mutanten gemäß a) und b), jeweils zusätzlich C-terminal um einen Rest umfassend 1 bis 10 Histidin-Reste verlängert und/oder N-terminal um einen Rest umfassend 1 bis 10 Histidin-Reste verlängert;
wobei die 3a-HSDH-Mutante wenigstens 80% Sequenzidentität zu SEQ ID NO:4 besitzt;
und in Gegenwart wenigstens einer 7β-HSDH zur korrespondierenden 12-Ketoursodesoxychiolsäure (12-Keto UDCS) der Formel (5), 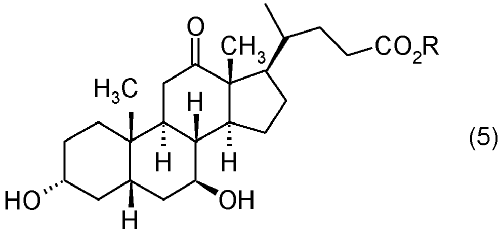 worin R die oben angegebenen Bedeutungen besitzt, oder die Gruppe -CO₂R durch die Säureamid-Gruppe -CONR¹R², wie oben definiert ersetzt ist, insbesondere in Gegenwart und unter Verbrauch von NADH und/oder NADPH reduziert und anschließend
c) 12-Keto-UDCS der Formel (5) chemisch zu UDCS reduziert; und
d) das Reaktionsprodukt gegebenenfalls weiter aufreinigt.

16. Verfahren nach Anspruch 14 oder 15, wobei zumindest Schritt b) in Gegenwart eines rekombinanten Mikroorganismus nach einem der Ansprüche 5 bis 8 durchgeführt wird.

17. Verfahren nach Anspruch 14 bis 16, wobei Schritt b) mit gleichen oder verschiedenen Kofaktorregenerierungssystemen gekoppelt ist.

## Claims

1. 3α-Hydroxysteroid dehydrogenase (3α-HSDH) which catalyses at least the stereospecific enzymatic reduction of a 3-ketosteroid to the corresponding 3-hydroxysteroid, in particular with stoichiometric consumption of NADH, wherein the enzyme possesses at least 80% sequence identity in relation to SEQ ID NO:4; and is selected among:
a) the single mutants
R34X₁ and
L68X₂ and
b) the double mutants
R34X₁/L68X₂
where
X₁ represents N, C, S or L and
X₂ represents A, C, K, S or V;
c) addition mutants which are C-terminally extended by a tail comprising 9 consecutive histidine residues;
and
d) mutants as per a) and b), in each case additionally C-terminally extended by a tail comprising 1 to 10 histidine residues and/or N-terminally extended by a tail comprising 1 to 10 histidine residues;
wherein this 3α-HSDH shows the following property profile in comparison with the 3α-HSDH with SEQ ID NO:4:
i) an increased specific activity (vₘₐₓ [U/mg]) for dehydrocholic acid (DHCA) in the enzymatic reduction of DHCA with NADH as cofactor,
ii) a reduced substrate inhibition by DHCA,
iii) an increased specific activity (vₘₐₓ [U/mg]) for NADH in the enzymatic reduction of DHCA with NADH as cofactor,
iv) it being possible for these properties i) to
iii) to be present individually or in any combination.

2. Nucleotide sequence, encoding a 3α-HSDH according to Claim 1.

3. Expression cassette, comprising at least one nucleotide sequence according to Claim 2 under the control of at least one regulatory sequence.

4. Expression vector, comprising at least one expression cassette according to Claim 3.

5. Recombinant microorganism, which bears at least one nucleotide sequence according to Claim 2 or at least one expression cassette according to Claim 3 or at least one expression vector according to Claim 4.

6. Recombinant microorganism according to Claim 5, which additionally optionally bears the coding sequence for at least one further enzyme, selected among hydroxysteroid dehydrogenases (HSDH) and dehydrogenases suitable for cofactor regeneration.

7. Recombinant microorganism according to Claim 6, wherein
the further HSDH is selected among 7β-HSDHs (NADPH- and/or NADH-dependent); and the dehydrogenase is selected among NADPH-regenerating enzymes, such as NADPH dehydrogenases, NADPH-regenerating alcohol dehydrogenases (ADH), NADPH-regenerating formate dehydrogenases (FDH), NADPH-regenerating glucose dehydrogenases (GDH), NADPH-regenerating glucose-6-phosphate dehydrogenases (G-6-PDH) and NADPH-regenerating phosphite dehydrogenases (PtDH), or NADH-regenerating enzymes, such as NADH dehydrogenases, NADH-regenerating formate dehydrogenases (FDH), NADH-regenerating alcohol dehydrogenases (ADH), NADH-regenerating glucose-6-phosphate dehydrogenases (G6PDH), NADH-regenerating phosphite dehydrogenases (PtDH) and NADH-regenerating glucose dehydrogenases (GDH).

8. Recombinant microorganism according to any of Claims 5 to 7, which is a 7α-HSDH knockout strain.

9. Process for the enzymatic or microbial synthesis of 3α-hydroxysteroids, wherein the corresponding 3-ketosteroid is reduced in the presence of a 3α-HSDH as per the definition in Claim 1 or in the presence of a recombinant microorganism which expresses this 3α-HSDH according to any of Claims 5 to 8, and optionally at least one reduction product formed is isolated from the reaction mixture.

10. Process according to Claim 9, wherein the 3-ketosteroid is selected among dehydrocholic acid (DHCA),
and the derivatives thereof, such as, in particular, a salt, an amide or an alkyl ester of the acid.

11. Process according to Claim 9 or 10, wherein the reduction takes place in the presence of and in particular with the consumption of NADPH and/or NADH.

12. Process according to Claim 11, wherein consumed NADPH is regenerated by coupling with an NADPH-regenerating enzyme, wherein the latter is selected in particular among NADPH dehydrogenases, NADPH-regenerating alcohol dehydrogenases (ADH), NADPH-regenerating formate dehydrogenases (FDH) and NADPH-regenerating glucose dehydrogenases (GDH), wherein the NADPH-regenerating enzyme is optionally expressed by a recombinant microorganism; and/or
wherein consumed NADH is regenerated by coupling with an NADH-regenerating enzyme, wherein the latter is selected in particular among NADH dehydrogenases, NADH-regenerating formate dehydrogenases (FDH), NADH-regenerating alcohol dehydrogenases (ADH), NADH-regenerating glucose-6-phosphate dehydrogenases (G6PDH), NADH-regenerating phosphite dehydrogenases (PtDH) and NADH-regenerating glucose dehydrogenases (GDH), wherein the NADH-regenerating enzyme is optionally expressed in a recombinant microorganism.

13. Process according to Claim 12, wherein the NADPH-regenerating enzyme is selected among
a) FDHs, including mutants of an NAD⁺-dependent FDH, which catalyses at least the enzymatic oxidation of formic acid to CO₂, wherein the mutant in comparison with the unmutated enzyme additionally accepts NADP⁺ as cofactor; and
b) GDHs.

14. Process for the preparation of ursodeoxycholic acid (UDCA) of the formula (1) 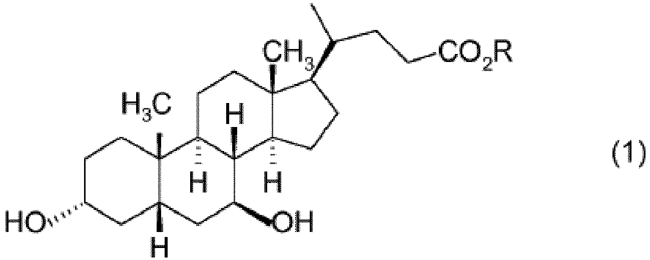 where
R represents alkyl, H, an alkali metal ion or N(R³)₄⁺, where the radicals R³ are identical or different and
represent H or alkyl, or the group -CO₂R is replaced by the acid amide group -CONR¹R², where R¹ and R² independently of one another represent an alkyl radical;
wherein
a) optionally a cholic acid (CA) of the formula (2) 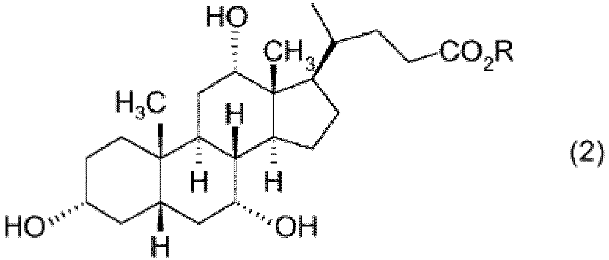
where R has the above-mentioned meanings or the group - CO₂R is replaced by the acid amide group -CONR¹R² as defined hereinabove,
is oxidized chemically to the dehydrocholic acid (DHCA) of the formula (3) 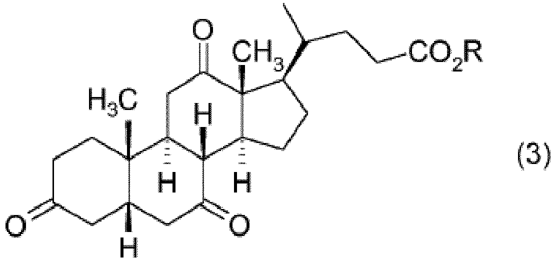
where R has the above-mentioned meanings or the group - CO₂R is replaced by the acid amide group -CONR¹R² as defined hereinabove;
b) DHCA is reduced in the presence of at least one 3α-HSDH mutant as per the definition in Claim 1 and in the presence of at least one 7β-HSDH to the corresponding 12-ketoursodeoxycholic acid (12-keto-UDCA) of the formula (5) 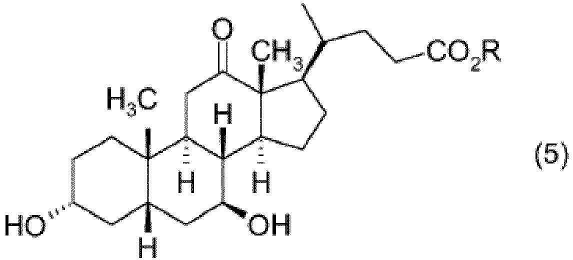 where R has the above-mentioned meanings or the group -CO₂R is replaced by the acid amide group -CONR¹R² as defined hereinabove, in particular in the presence and with the consumption of NADH and/or NADPH, and subsequently
c) 12-keto-UDCA of the formula (5) is reduced chemically to UDCA; and
d) optionally, the reaction product is purified further.

15. Process for the preparation of ursodeoxycholic acid (UDCA) of the formula (1) 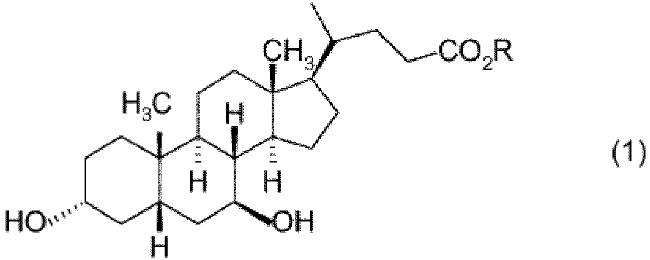 where
R represents alkyl, H, an alkali metal ion or N(R³)₄⁺, where the radicals R³ are identical or different and represent H or alkyl, or the group -CO₂R is replaced by the acid amide group -CONR¹R², where R¹ and R² independently of one another represent an alkyl radical;
wherein
a) optionally a cholic acid (CA) of the formula (2) 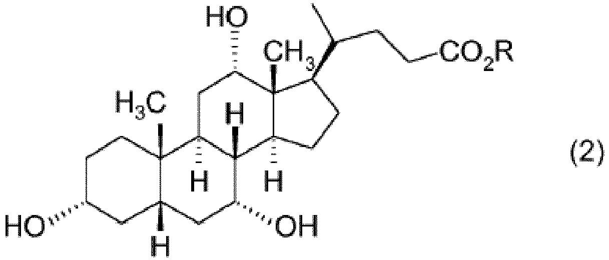
where R has the above-mentioned meanings or the group -CO₂R is replaced by the acid amide group -CONR¹R² as defined hereinabove,
is oxidized chemically to the dehydrocholic acid (DHCA) of the formula (3) 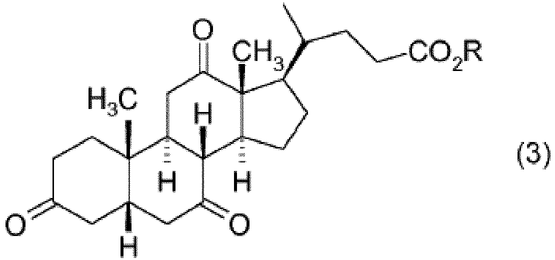
where R has the above-mentioned meanings or the group -CO₂R is replaced by the acid amide group -CONR¹R² as defined hereinabove;
b) DHCA is reduced in the presence of at least one 3α-HSDH mutant, selected among
(a) the single mutants
R34X₁ and
L68X₂ and
(b) the double mutants
R34X₁/L68X₂
where
X₁ represents N, C, S or L and
X₂ represents A, C, K, S or V;
(c) addition mutants which are C-terminally extended by a tail comprising 9 consecutive histidine residues;
and
(d) mutants as per a) and b), in each case additionally C-terminally extended by a tail comprising 1 to 10 histidine residues and/or N-terminally extended by a tail comprising 1 to 10 histidine residues;
wherein the 3α-HSDH mutant possesses at least 80% sequence identity in relation to SEQ ID NO:4;
and in the presence of at least one 7β-HSDH to the corresponding 12-ketoursodeoxycholic acid (12-keto-UDCA) of the formula (5) 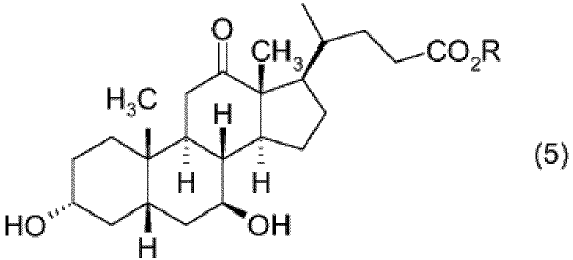 where R has the above-mentioned meanings or the group -CO₂R is replaced by the acid amide group -CONR¹R² as defined hereinabove, in particular in the presence and with the consumption of NADH and/or NADPH,
and subsequently
c) 12-keto-UDCA of the formula (5) is reduced chemically to UDCA; and
d) optionally, the reaction product is purified further.

16. Process according to Claim 14 or 15, wherein at least step b) is carried out in the presence of a recombinant microorganism according to any of Claims 5 to 8.

17. Process according to Claims 14 to 16, wherein step b) is coupled with identical or different cofactor regeneration systems.

## Revendications

1. 3α-Hydroxystéroïde-déshydrogénase (3α-HSDH), qui catalyse au moins la réduction enzymatique stéréospécifique d'un 3-cétostéroïde en le 3-hydroxystéroïde correspondant, notamment avec consommation stœchiométrique de NADH, l'enzyme présentant au moins 80 % d'identité de séquence avec SEQ ID NO : 4 ; et étant choisie parmi :
a) les mutants simples
R34X₁ et
L68X₂ et
b) les mutants doubles
R34X₁/L68X₂
X₁ représentant N, C, S ou L, et
X₂ représentant A, C, K, S ou V ;
c) les mutants d'addition prolongés d'un radical comprenant 9 radicaux histidine consécutifs à la terminaison C ;
et
d) les mutants selon a) et b), chacun en outre prolongés d'un radical comprenant 1 à 10 radicaux histidine à la terminaison C et/ou d'un radical comprenant 1 à 10 radicaux histidine à la terminaison N ;
cette 3α-HSDH présentant le profil de propriétés suivant en comparaison de la 3α-HSDH de SEQ ID NO : 4 :
i) une activité spécifique élevée (vₘₐₓ [U/mg]) pour l'acide déshydrocholique (DHCA) lors de la réduction enzymatique de DHCA avec NADH en tant que cofacteur,
ii) une inhibition du substrat réduite par le DHCA,
iii) une activité spécifique élevée (vₘₐₓ [U/mg]) pour le NADH lors de la réduction enzymatique de DHCA avec NADH en tant que cofacteur,
iv) ces propriétés i) à iii) pouvant être présentes individuellement ou selon une combinaison quelconque.

2. Séquence nucléotidique, codant pour une 3α-HSDH selon la revendication 1.

3. Cassette d'expression, comprenant au moins une séquence nucléotidique selon la revendication 2 sous le contrôle d'au moins une séquence de régulation.

4. Vecteur d'expression, comprenant au moins une cassette d'expression selon la revendication 3.

5. Microorganisme recombinant, qui porte au moins une séquence nucléotidique selon la revendication 2 ou au moins une cassette d'expression selon la revendication 3 ou au moins un vecteur d'expression selon la revendication 4.

6. Microorganisme recombinant selon la revendication 5, qui porte éventuellement en outre la séquence codante pour au moins une enzyme supplémentaire, choisie parmi les hydroxystéroïde-déshydrogénases (HSDH) et les déshydrogénases appropriées pour la régénération du cofacteur.

7. Microorganisme recombinant selon la revendication 6, dans lequel l'HSDH supplémentaire est choisie parmi les 7β-HSDH (dépendantes de NADPH et/ou de NADH) ; et les déshydrogénases sont choisies parmi les enzymes régénérant NADPH, telles que les NADPH déshydrogénases, les alcool-déshydrogénases régénérant NADPH (ADH), les formiate-déshydrogénases régénérant NADPH (FDH), les glucose-déshydrogénases régénérant NADPH (GDH), les glucose-6-phosphate-déshydrogénases régénérant NADPH (G-6-PDH), ainsi que les phosphite-déshydrogénases régénérant NADPH (PtDH), ou les enzymes régénérant NADH, telles que les NADH déshydrogénases, les formiate-déshydrogénases régénérant NADH (FDH), les alcool-déshydrogénases régénérant NADH (ADH), les glucose-6-phosphate-déshydrogénases régénérant NADH (G6PDH), les phosphite-déshydrogénases régénérant NADH (PtDH), ainsi que les glucose-déshydrogénases régénérant NADH (GDH).

8. Microorganisme recombinant selon l'une quelconque des revendications 5 à 7, qui est une souche 7α-HSDH knock-out.

9. Procédé de synthèse enzymatique ou microbienne de 3α-hydroxystéroïdes, selon lequel le 3-cétostéroïde correspondant est réduit en présence d'une 3α-HSDH selon la définition dans la revendication 1 ou en présence d'un microorganisme recombinant exprimant cette 3α-HSDH selon l'une quelconque des revendications 5 à 8, et au moins un produit de réduction formé est éventuellement isolé à partir de la préparation réactionnelle.

10. Procédé selon la revendication 9, dans lequel le 3-cétostéroïde est choisi parmi l'acide déshydrocholique (DHCA) et ses dérivés, tels que notamment un sel, un amide ou un ester alkylique de l'acide.

11. Procédé selon la revendication 9 ou 10, dans lequel la réduction a lieu en présence et notamment avec consommation de NADPH et/ou de NADH.

12. Procédé selon la revendication 11, dans lequel le NADPH consommé est régénéré par couplage avec une enzyme régénérant NADPH, celle-ci étant notamment choisie parmi les NADPH déshydrogénases, les alcool-déshydrogénases régénérant NADPH (ADH), les formiate-déshydrogénases régénérant NADPH (FDH) et les glucose-déshydrogénases régénérant NADPH (GDH), l'enzyme régénérant NADPH étant éventuellement exprimée par un microorganisme recombinant ; et/ou
dans lequel le NADH consommé est régénéré par couplage avec une enzyme régénérant NADH, celle-ci étant notamment choisie parmi les NADH déshydrogénases, les formiate-déshydrogénases régénérant NADH (FDH), les alcool-déshydrogénases régénérant NADH (ADH), les glucose-6-phosphate-déshydrogénases régénérant NADH (G6PDH), les phosphite-déshydrogénases régénérant NADH (PtDH), ainsi que les glucose-déshydrogénases régénérant NADH (GDH), l'enzyme régénérant NADH étant éventuellement exprimée dans un microorganisme recombinant.

13. Procédé selon la revendication 12, dans lequel l'enzyme régénérant NADPH est choisie parmi :
a) les FDH, y compris les mutants d'une FDH dépendante de NAD⁺, qui catalysent au moins l'oxydation enzymatique d'acide formique en CO₂, les mutants acceptant en outre NADP⁺ en tant que cofacteur en comparaison de l'enzyme non mutée ; et
b) les GDH.

14. Procédé de fabrication d'acide ursodésoxycholique (UDCS) de la formule (1) : 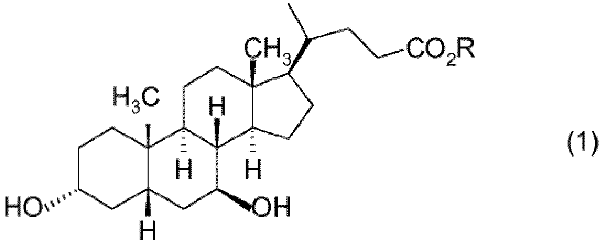 dans laquelle
R représente alkyle, H, un ion de métal alcalin ou N(R³)₄⁺, les radicaux R³ étant identiques ou différents,
et représentant H ou alkyle, ou le groupe -CO₂R est remplacé par le groupe amide d'acide -CONR¹R², R¹ et R² représentant indépendamment l'un de l'autre un radical alkyle ;
selon lequel
a) éventuellement un acide cholique (CS) de la formule (2) : 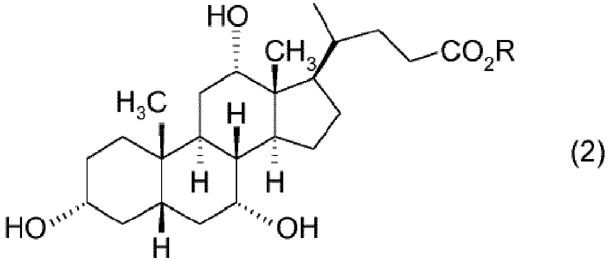 dans laquelle R a les significations indiquées précédemment, ou le groupe -CO₂R est remplacé par le groupe amide d'acide -CONR¹R², tel que défini précédemment,
est oxydé chimiquement en l'acide déshydrocholique (DHCS) de la formule (3) : 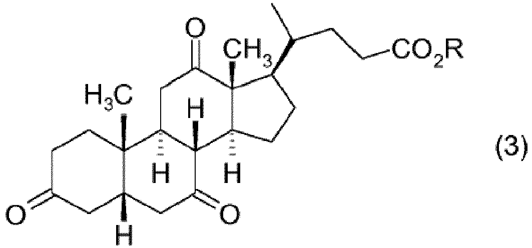 dans laquelle R a les significations indiquées précédemment, ou le groupe -CO₂R est remplacé par le groupe amide d'acide -CONR¹R², tel que défini précédemment ;
b) le DHCS est réduit en présence d'au moins un mutant de 3α-HSDH selon la définition dans la revendication 1 et en présence d'au moins une 7β-HSDH pour former l'acide 12-céto-ursodésoxycholique correspondant (12-céto-UDCS) de la formule (5) : 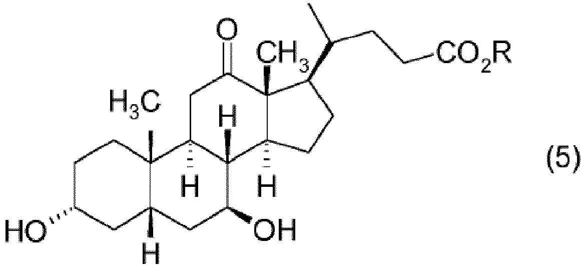 dans laquelle R a les significations indiquées précédemment, ou le groupe -CO₂R est remplacé par le groupe amide d'acide -CONR¹R², tel que défini précédemment, notamment en présence et avec consommation de NADH et/ou de NADPH, puis
c) le 12-céto-UDCS de la formule (5) est réduit chimiquement en UDCS ; et
d) le produit de réaction est éventuellement davantage purifié.

15. Procédé de fabrication d'acide ursodésoxycholique (UDCS) de la formule (1) : 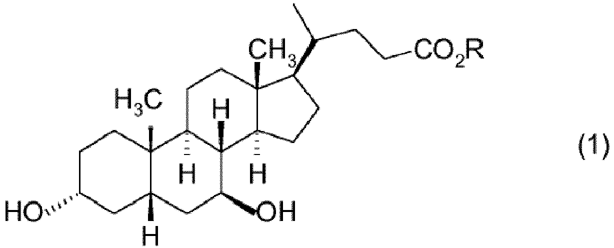 dans laquelle
R représente alkyle, H, un ion de métal alcalin ou N(R³)₄⁺, les radicaux R³ étant identiques ou différents, et représentant H ou alkyle, ou le groupe -CO₂R est remplacé par le groupe amide d'acide -CONR¹R², R¹ et R² représentant indépendamment l'un de l'autre un radical alkyle ;
selon lequel
a) éventuellement un acide cholique (CS) de la formule (2) : 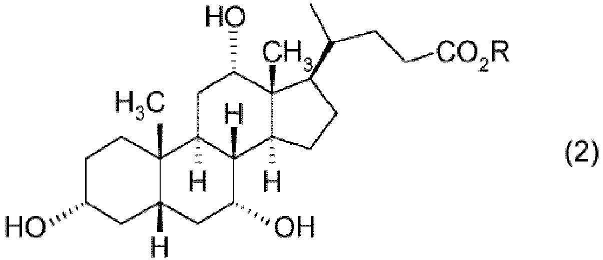 dans laquelle R a les significations indiquées précédemment, ou le groupe -CO₂R est remplacé par le groupe amide d'acide -CONR¹R², tel que défini précédemment, est oxydé chimiquement en acide déshydrocholique (DHCS) de la formule (3) : 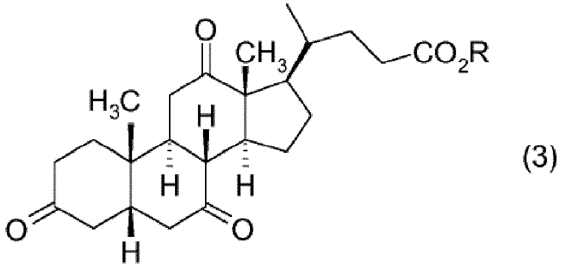 dans laquelle R a les significations indiquées précédemment, ou le groupe -CO₂R est remplacé par le groupe amide d'acide -CONR¹R², tel que défini précédemment ;
b) le DHCS est réduit en présence d'au moins un mutant de 3α-HSDH, choisi parmi :
(a) les mutants simples
R34X₁ et
L68X₂ et
(b) les mutants doubles
R34X₁/L68X₂
X₁ représentant N, C, S ou L, et
X₂ représentant A, C, K, S ou V ;
c) les mutants d'addition prolongés d'un radical comprenant 9 radicaux histidine consécutifs à la terminaison C ;
et
d) les mutants selon a) et b), chacun en outre prolongés d'un radical comprenant 1 à 10 radicaux histidine à la terminaison C et/ou d'un radical comprenant 1 à 10 radicaux histidine à la terminaison N ;
les mutants de 3α-HSDH présentant au moins 80 % d'identité de séquence avec SEQ ID NO : 4 ;
et en présence d'au moins un 7β-HSDH pour former l'acide 12-céto-ursodésoxycholique correspondant (12-céto-UDCS) de la formule (5) : 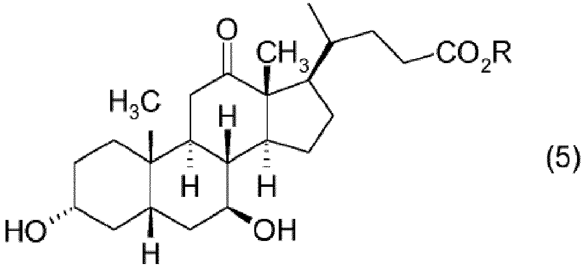 dans laquelle R a les significations indiquées précédemment, ou le groupe -CO₂R est remplacé par le groupe amide d'acide -CONR¹R², tel que défini précédemment, notamment en présence et avec consommation de NADH et/ou de NADPH ; puis
c) le 12-céto-UDCS de la formule (5) est réduit chimiquement en UDCS ; et
d) le produit de réaction est éventuellement davantage purifié.

16. Procédé selon la revendication 14 ou 15, dans lequel au moins l'étape b) est réalisée en présence d'un microorganisme recombinant selon l'une quelconque des revendications 5 à 8.

17. Procédé selon les revendications 14 à 16, dans lequel l'étape b) est couplée avec des systèmes de régénération du cofacteur identiques ou différents.
